# EUROPEAN PATENT SPECIFICATION

(11) **EP 3 177 726 B1**
(45) Date of publication and mention of the grant of the patent: **27.01.2021**
(21) Application number: 15760097.4
(22) Date of filing: 04.08.2015
(51) Int. Cl.: C12N 15/86, C12N 9/52, A61K 48/00, A61P 25/00

(54) **GENOME EDITING FOR THE TREATMENT OF HUNTINGTON'S DISEASE**
GEMONEDITIERUNG ZUR BEHANDLUNG VON MORBUS HUNTINGTON
ÉDITION DE GÉNOME POUR LE TRAITEMENT DE LA MALADIE DE HUNTINGTON

(30) Priority: 04.08.2014 EP 14179767
(43) Date of publication of application: 14.06.2017
(73) Proprietor: Centre Hospitalier Universitaire Vaudois (CHUV), 1011 Lausanne (CH)
(72) Inventor: DEGLON, Nicole, CH-1521 Curtilles (CH); MERIENNE, Nicolas, CH-1081 Montpreveyres (CH)
(74) Representative: KATZAROV S.A.
(86) International application number: PCT/EP2015/067986
(87) International publication number: WO 2016/020399

(56) References cited:
- WO-A1-2013/130824
- WO-A1-2014/093701
- M. GARRIGA-CANUT ET AL: "Synthetic zinc finger repressors reduce mutant huntingtin expression in the brain of R6/2 mice", PROCEEDINGS OF THE NATIONAL ACADEMY OF SCIENCES, vol. 109, no. 45, 10 October 2012 (2012-10-10), pages E3136-E3145, XP055161220, ISSN: 0027-8424, DOI: 10.1073/pnas.1206506109
- CONG LE ET AL: "In Vivo Genome Engineering With AAV Vector Carrying CRISPR-Cas9 System", MOLECULAR THERAPY, vol. 22, no. Suppl. 1, May 2014 (2014-05), page S214, XP002734380, & 17TH ANNUAL MEETING OF THE AMERICAN-SOCIETY-OF-GENE-AND-CELL-THERAPY (ASGCT); WASHINGTON, DC, USA; MAY 21 -24, 2014
- A. FISZER ET AL: "Oligonucleotide-based strategies to combat polyglutamine diseases", NUCLEIC ACIDS RESEARCH, vol. 42, no. 11, 21 May 2014 (2014-05-21), pages 6787-6810, XP055161218, ISSN: 0305-1048, DOI: 10.1093/nar/gku385
- SAMUEL H. STERNBERG ET AL: "DNA interrogation by the CRISPR RNA-guided endonuclease Cas9", NATURE, vol. 507, no. 7490, 29 January 2014 (2014-01-29), pages 62-67, XP055161285, ISSN: 0028-0836, DOI: 10.1038/nature13011
- MICHELE SIMONATO ET AL: "Progress in gene therapy for neurological disorders", NATURE REVIEWS NEUROLOGY, vol. 9, no. 5, 23 April 2013 (2013-04-23), pages 277-291, XP055161287, ISSN: 1759-4758, DOI: 10.1038/nrneurol.2013.56
- C. ZUCCATO ET AL: "Molecular Mechanisms and Potential Therapeutical Targets in Huntington's Disease", PHYSIOLOGICAL REVIEWS, vol. 90, no. 3, 1 July 2010 (2010-07-01), pages 905-981, XP055161189, ISSN: 0031-9333, DOI: 10.1152/physrev.00041.2009
- NICOLAS MERIENNE ET AL: "The Self-Inactivating KamiCas9 System for the Editing of CNS Disease Genes", CELL REPORTS, vol. 20, no. 12, 1 September 2017 (2017-09-01), pages 2980-2991, XP55548036, US ISSN: 2211-1247, DOI: 10.1016/j.celrep.2017.08.075
- ZOGHBI H Y ET AL: "Glutamine repeats and neurodegeneration", ANNUAL REVIEW OF NEUROSCIENCE, ANNUAL REVIEWS INC., PALO ALTO, CA, US, vol. 23, 1 January 2000 (2000-01-01), pages 217-247, XP002443799, ISSN: 0147-006X, DOI: 10.1146/ANNUREV.NEURO.23.1.217

### SEQUENCE LISTING

<110> Centre Hospitalier Universitaire Vaudois CHUV
<120> GENOME EDITING FOR THE TREATMENT OF HUNTINGTON's DISEASE
<130> 19685/EP
<160> 59
<170> PatentIn version 3.5
<210> 1
   <211> 82
   <212> DNA
   <213> Artificial Sequence
<220>
   <223> synthetic sequence
<400> 1
   gttttagagc tagaaatagc aagttaaaat aaggctagtc cgttatcaac ttgaaaaagt 60
ggcaccgagt cggtgctttt tt 82
<210> 2
   <211> 20
   <212> DNA
   <213> Artificial Sequence
<220>
   <223> synthetic sequence
<400> 2
   gaccctggaa aagctgatga 20
<210> 3
   <211> 20
   <212> DNA
   <213> Artificial Sequence
<220>
   <223> synthetic sequence
<400> 3
   gaagttcgag ggcgacaccc 20
<210> 4
   <211> 20
   <212> DNA
   <213> Homo sapiens
<220>
   <221> misc_feature
   <222> (1)..(1)
   <223> n=G or A
<400> 4
   naccctggaa aagctgatga 20
<210> 5
   <211> 20
   <212> DNA
   <213> Homo sapiens
<400> 5
   gtggatgaca taatgctttt 20
<210> 6
   <211> 20
   <212> DNA
   <213> Homo sapiens
<220>
   <221> misc_feature
   <222> (4) .. (4)
   <223> n=G or A
<400> 6
   gganccgctg caccgaccgt 20
<210> 7
   <211> 20
   <212> DNA
   <213> Homo sapiens
<220>
   <221> misc_feature
   <222> (20) .. (20)
   <223> n=G or A
<400> 7
   tgggacgcaa ggcgccgtgn 20
<210> 8
   <211> 4140
   <212> DNA
   <213> Artificial Sequence
<220>
   <223> synthetic sequence
<400> 8
   atggacaaga agtactccat tgggctcgat atcggcacaa acagcgtcgg ctgggccgtc 60
   attacggacg agtacaaggt gccgagcaaa aaattcaaag ttctgggcaa taccgatcgc 120
   cacagcataa agaagaacct cattggcgcc ctcctgttcg actccgggga gacggccgaa 180
   gccacgcggc tcaaaagaac agcacggcgc agatataccc gcagaaagaa tcggatctgc 240
   tacctgcagg agatctttag taatgagatg gctaaggtgg atgactcttt cttccatagg 300
   ctggaggagt cctttttggt ggaggaggat aaaaagcacg agcgccaccc aatctttggc 360
   aatatcgtgg acgaggtggc gtaccatgaa aagtacccaa ccatatatca tctgaggaag 420
   aagcttgtag acagtactga taaggctgac ttgcggttga tctatctcgc gctggcgcat      480
   atgatcaaat ttcggggaca cttcctcatc gagggggacc tgaacccaga caacagcgat      540
   gtcgacaaac tctttatcca actggttcag acttacaatc agcttttcga agagaacccg      600
   atcaacgcat ccggagttga cgccaaagca atcctgagcg ctaggctgtc caaatcccgg      660
   cggctcgaaa acctcatcgc acagctccct ggggagaaga agaacggcct gtttggtaat      720
   cttatcgccc tgtcactcgg gctgaccccc aactttaaat ctaacttcga cctggccgaa      780
   gatgccaagc ttcaactgag caaagacacc tacgatgatg atctcgacaa tctgctggcc      840
   cagatcggcg accagtacgc agaccttttt ttggcggcaa agaacctgtc agacgccatt      900
   ctgctgagtg atattctgcg agtgaacacg gagatcacca aagctccgct gagcgctagt      960
   atgatcaagc gctatgatga gcaccaccaa gacttgactt tgctgaaggc ccttgtcaga     1020
   cagcaactgc ctgagaagta caaggaaatt ttcttcgatc agtctaaaaa tggctacgcc     1080
   ggatacattg acggcggagc aagccaggag gaattttaca aatttattaa gcccatcttg     1140
   gaaaaaatgg acggcaccga ggagctgctg gtaaagctta acagagaaga tctgttgcgc     1200
   aaacagcgca ctttcgacaa tggaagcatc ccccaccaga ttcacctggg cgaactgcac     1260
   gctatcctca ggcggcaaga ggatttctac ccctttttga aagataacag ggaaaagatt     1320
   gagaaaatcc tcacatttcg gataccctac tatgtaggcc ccctcgcccg gggaaattcc     1380
   agattcgcgt ggatgactcg caaatcagaa gagaccatca ctccctggaa cttcgaggaa     1440
   gtcgtggata agggggcctc tgcccagtcc ttcatcgaaa ggatgactaa ctttgataaa     1500
   aatctgccta acgaaaaggt gcttcctaaa cactctctgc tgtacgagta cttcacagtt     1560
   tataacgagc tcaccaaggt caaatacgtc acagaaggga tgagaaagcc agcattcctg     1620
   tctggagagc agaagaaagc tatcgtggac ctcctcttca agacgaaccg gaaagttacc     1680
   gtgaaacagc tcaaagaaga ctatttcaaa aagattgaat gtttcgactc tgttgaaatc     1740
   agcggagtgg aggatcgctt caacgcatcc ctgggaacgt atcacgatct cctgaaaatc     1800
   attaaagaca aggacttcct ggacaatgag gagaacgagg acattcttga ggacattgtc     1860
   ctcaccctta cgttgtttga agatagggag atgattgaag aacgcttgaa aacttacgct     1920
   catctcttcg acgacaaagt catgaaacag ctcaagaggc gccgatatac aggatggggg     1980
   cggctgtcaa gaaaactgat caatgggatc cgagacaagc agagtggaaa gacaatcctg     2040
   gattttctta agtccgatgg atttgccaac cggaacttca tgcagttgat ccatgatgac     2100
   tctctcacct ttaaggagga catccagaaa gcacaagttt ctggccaggg ggacagtctt     2160
   cacgagcaca tcgctaatct tgcaggtagc ccagctatca aaaagggaat actgcagacc     2220
   gttaaggtcg tggatgaact cgtcaaagta atgggaaggc ataagcccga gaatatcgtt     2280
   atcgagatgg cccgagagaa ccaaactacc cagaagggac agaagaacag tagggaaagg 2340
   atgaagagga ttgaagaggg tataaaagaa ctggggtccc aaatccttaa ggaacaccca 2400
   gttgaaaaca cccagcttca gaatgagaag ctctacctgt actacctgca gaacggcagg 2460
   gacatgtacg tggatcagga actggacatc aatcggctct ccgactacga cgtggatcat 2520
   atcgtgcccc agtcttttct caaagatgat tctattgata ataaagtgtt gacaagatcc 2580
   gataaaaata gagggaagag tgataacgtc ccctcagaag aagttgtcaa gaaaatgaaa 2640
   aattattggc ggcagctgct gaacgccaaa ctgatcacac aacggaagtt cgataatctg 2700
   actaaggctg aacgaggtgg cctgtctgag ttggataaag ccggcttcat caaaaggcag 2760
   cttgttgaga cacgccagat caccaagcac gtggcccaaa ttctcgattc acgcatgaac 2820
   accaagtacg atgaaaatga caaactgatt cgagaggtga aagttattac tctgaagtct 2880
   aagctggtct cagatttcag aaaggacttt cagttttata aggtgagaga gatcaacaat 2940
   taccaccatg cgcatgatgc ctacctgaat gcagtggtag gcactgcact tatcaaaaaa 3000
   tatcccaagc ttgaatctga atttgtttac ggagactata aagtgtacga tgttaggaaa 3060
   atgatcgcaa agtctgagca ggaaataggc aaggccaccg ctaagtactt cttttacagc 3120
   aatattatga attttttcaa gaccgagatt acactggcca atggagagat tcggaagcga 3180
   ccacttatcg aaacaaacgg agaaacagga gaaatcgtgt gggacaaggg tagggatttc 3240
   gcgacagtcc ggaaggtcct gtccatgccg caggtgaaca tcgttaaaaa gaccgaagta 3300
   cagaccggag gcttctccaa ggaaagtatc ctcccgaaaa ggaacagcga caagctgatc 3360
   gcacgcaaaa aagattggga ccccaagaaa tacggcggat tcgattctcc tacagtcgct 3420
   tacagtgtac tggttgtggc caaagtggag aaagggaagt ctaaaaaact caaaagcgtc 3480
   aaggaactgc tgggcatcac aatcatggag cgatcaagct tcgaaaaaaa ccccatcgac 3540
   tttctcgagg cgaaaggata taaagaggtc aaaaaagacc tcatcattaa gcttcccaag 3600
   tactctctct ttgagcttga aaacggccgg aaacgaatgc tcgctagtgc gggcgagctg 3660
   cagaaaggta acgagctggc actgccctct aaatacgtta atttcttgta tctggccagc 3720
   cactatgaaa agctcaaagg gtctcccgaa gataatgagc agaagcagct gttcgtggaa 3780
   caacacaaac actaccttga tgagatcatc gagcaaataa gcgaattctc caaaagagtg 3840
   atcctcgccg acgctaacct cgataaggtg ctttctgctt acaataagca cagggataag 3900
   cccatcaggg agcaggcaga aaacattatc cacttgttta ctctgaccaa cttgggcgcg 3960
   cctgcagcct tcaagtactt cgacaccacc atagacagaa agcggtacac ctctacaaag 4020
   gaggtcctgg acgccacact gattcatcag tcaattacgg ggctctatga aacaagaatc 4080
   gacctctctc agctcggtgg agacagcagg gctgacccca agaagaagag gaaggtgtga 4140
<210> 9
   <211> 972
   <212> DNA
   <213> Artificial Sequence
<220>
   <223> synthetic sequence
<400> 9
   ctgcccggcg cctacaacgt caacatcaag ttggacatca cctcccacaa cgaggactac      60
   accatcgtgg aacagtacga acgcgccgag ggccgccact ccaccggcgg catggacgag     120
   ctgtacaagg gtaccagcgc gccgcgtcga gagatggggg aggctaacag aagtgtacgc     180
   gttggctgct gtcaggggta ccatggacgc gtagacaagg tgaggaacct agctcagtca     240
   tgcaccgggc tgcagatgag caagggcgag gagctgttca ccggggtggt gcccatcctg     300
   gtcgagctgg acggcgacgt aaacggccac aagttcagcg tgtccggcga gggcgagggc     360
   gatgccacct acggcaagct gaccctgaag ttcatctgca ccaccggcaa gctgcccgtg     420
   ccctggccca ccctcgtgac caccctgacc tacggcgtgc agtgcttcag ccgctacccc     480
   gaccacatga agcagcacga cttcttcaag tccgccatgc ccgaaggcta cgtccaggag     540
   cgcaccatct tcttcaagga cgacggcaac tacaagaccc gcgccgaggt gaagttcgag     600
   ggcgacaccc tggtgaaccg catcgagctg aagggcatcg acttcaagga ggacggcaac     660
   atcctggggc acaagctgga gtacaactac aacagccaca acgtctatat catggccgac     720
   aagcagaaga acggcatcaa ggccaacttc aagatccgcc acaacatcga ggacggcagc     780
   gtgcagctcg ccgaccacta ccagcagaac acccccatcg gcgacggccc cgtgctgctg     840
   cccgacaacc actacctgag cacccagtcc gccctgagca aagaccccaa cgagaagcgc     900
   gatcacatgg tcctgctgga gttcgtgacc gccgccggga tcactcacgg catggacgag     960
   ctgtacaagt ga                                                         972
<210> 10
   <211> 20
   <212> DNA
   <213> Artificial Sequence
<220>
   <223> synthetic
<220>
   <221> misc_feature
   <222> (26).. (26)
   <223> n=A or G
<400> 10
   gctgaagggc atcgacttca 20
<210> 11
   <211> 20
   <212> DNA
   <213> Artificial Sequence
<220>
   <223> synthetic
<220>
   <221> misc_feature
   <222> (26).. (26)
   <223> n=A or C
<400> 11
   caactacaag acccgcgccg 20
<210> 12
   <211> 10867
   <212> DNA
   <213> Artificial Sequence
<220>
   <223> synthetic
<220>
   <221> misc_feature
   <222> (26) .. (26)
   <223> n=A or G
<400> 12
   tggaagggct aattcactcc caaagaagac aagatatcct tgatctgtgg atctaccaca      60
   cacaaggcta cttccctgat tagcagaact acacaccagg gccaggggtc agatatccac     120
   tgacctttgg atggtgctac aagctagtac cagttgagcc agataaggta gaagaggcca     180
   ataaaggaga gaacaccagc ttgttacacc ctgtgagcct gcatgggatg gatgacccgg     240
   agagagaagt gttagagtgg aggtttgaca gccgcctagc atttcatcac gtggcccgag     300
   agctgcatcc ggagtacttc aagaactgct gatatcgagc ttgctacaag ggactttccg     360
   ctggggactt tccagggagg cgtggcctgg gcgggactgg ggagtggcga gccctcagat     420
   cctgcatata agcagctgct ttttgcctgt actgggtctc tctggttaga ccagatctga     480
   gcctgggagc tctctggcta actagggaac ccactgctta agcctcaata aagcttgcct     540
   tgagtgcttc aagtagtgtg tgcccgtctg ttgtgtgact ctggtaacta gagatccctc     600
   agaccctttt agtcagtgtg gaaaatctct agcagtggcg cccgaacagg gacctgaaag     660
   cgaaagggaa accagagctc tctcgacgca ggactcggct tgctgaagcg cgcgcacggc     720
   aagaggcgag gggcggcgac tggtgagtac gccaaaaatt ttgactagcg gaggctagaa     780
   ggagagagat gggtgcgaga gcgtcagtat taagcggggg agaattagat cgcgatggga     840
   aaaaattcgg ttaaggccag ggggaaagaa aaaatataaa ttaaaacata tagtatgggc     900
   aagcagggag ctagaacgat tcgcagttaa tcctggcctg ttagaaacat cagaaggctg     960
   tagacaaata ctgggacagc tacaaccatc ccttcagaca ggatcagaag aacttagatc 1020
   attatataat acagtagcaa ccctctattg tgtgcatcaa aggatagaga taaaagacac 1080
   caaggaagct ttagacaaga tagaggaaga gcaaaacaaa agtaagacca ccgcacagca 1140
   agcggccgct gatcttcaga cctggaggag gagatatgag ggacaattgg agaagtgaat 1200
   tatataaata taaagtagta aaaattgaac cattaggagt agcacccacc aaggcaaaga 1260
   gaagagtggt gcagagagaa aaaagagcag tgggaatagg agctttgttc cttgggttct 1320
   tgggagcagc aggaagcact atgggcgcag cctcaatgac gctgacggta caggccagac 1380
   aattattgtc tggtatagtg cagcagcaga acaatttgct gagggctatt gaggcgcaac 1440
   agcatctgtt gcaactcaca gtctggggca tcaagcagct ccaggcaaga atcctggctg 1500
   tggaaagata cctaaaggat caacagctcc tggggatttg gggttgctct ggaaaactca 1560
   tttgcaccac tgctgtgcct tggaatgcta gttggagtaa taaatctctg gaacagattg 1620
   gaatcacacg acctggatgg agtgggacag agaaattaac aattacacaa gcttaataca 1680
   ctccttaatt gaagaatcgc aaaaccagca agaaaagaat gaacaagaat tattggaatt 1740
   agataaatgg gcaagtttgt ggaattggtt taacataaca aattggctgt ggtatataaa 1800
   attattcata atgatagtag gaggcttggt aggtttaaga atagtttttg ctgtactttc 1860
   tatagtgaat agagttaggc agggatattc accattatcg tttcagaccc acctcccaac 1920
   cccgagggga cccgacaggc ccgaaggaat agaagaagaa ggtggagaga gagacagaga 1980
   cagatccatt cgattagtga acggatctcg acggtatcgg ttaactttta aaagaaaagg 2040
   ggggattggg gggtacagtg caggggaaag aatagtagac ataatagcaa cagacataca 2100
   aactaaagaa ttacaaaaac aaattacaaa aattcaaaat tttatcgcta gtggatcccc 2160
   cgggctgcag gaattatcac aagtttgtac aaaaaagcag gcttcatcga tcaattggga 2220
   tccaattcga acgctgacgt catcaacccg ctccaaggaa tcgcgggccc agtgtcacta 2280
   ggcgggaaca cccagcgcgc gtgcgccctg gcaggaagat ggctgtgagg gacaggggag 2340
   tggcgccctg caatatttgc atgtcgctat gtgttctggg aaatcaccat aaacgtgaaa 2400
   tgtctttgga tttgggaatc ttataagttc tgtatgagac cacagatctg aagttcgagg 2460
   gcgacacccg ttttagagct agaaatagca agttaaaata aggctagtcc gttatcaact 2520
   tgaaaaagtg gcaccgagtc ggtgcttttt tagatctgga tccaagcttc catggaaggt 2580
   cgggcaggaa gagggcctat ttcccatgat tccttcatat ttgcatatac gatacaaggc 2640
   tgttagagag ataattagaa ttaatttgac tgtaaacaca aagatattag tacaaaatac 2700
   gtgacgtaga aagtaataat ttcttgggta gtttgcagtt ttaaaattat gttttaaaat 2760
   ggactatcat atgcttaccg taacttgaaa gtatttcgat ttcttgggtt tatatatctt 2820
   gtggaaagga cgaaacaccg ctgaagggca tcgacttcag ttttagagct agaaatagca 2880
   agttaaaata aggctagtcc gttatcaact tgaaaaagtg gcaccgagtc ggtgcttttt 2940
   tcatatgcca tgggcggccg ctctagactg cagtatttag catgccccac ccatctgcaa 3000
   ggcattctgg atagtgtcaa aacagccgga aatcaagtcc gtttatctca aactttagca 3060
   ttttgggaat aaatgatatt tgctatgctg gttaaattag attttagtta aatttcctgc 3120
   tgaagctcta gtacgataag taacttgacc taagtgtaaa gttgagattt ccttcaggtt 3180
   tatatagctt gtgcgccgcc tgggtacctc caactacaag acccgcgccg gttttagagc 3240
   tagaaatagc aagttaaaat aaggctagtc cgttatcaac ttgaaaaagt ggcaccgagt 3300
   cggtgctttt ttggtacctc tagactcgag actagtaggc ctgacccagc tttcttgtac 3360
   aaagtggtga taattcgata tcaagcttat cgataccgtc gacctcgacg atggtcgagt 3420
   accgggtagg ggaggcgctt ttcccaaggc agtctggagc atgcgcttta gcagccccgc 3480
   tgggcacttg gcgctacaca agtggcctct ggcctcgcac acattccaca tccaccggta 3540
   ggcgccaacc ggctccgttc tttggtggcc ccttcgcgcc accttctact cctcccctag 3600
   tcaggaagtt cccccccgcc ccgcagctcg cgtcgtgcag gacgtgacaa atggaagtag 3660
   cacgtctcac tagtctcgtg cagatggaca gcaccgctga gcaatggaag cgggtaggcc 3720
   tttggggcag cggccaatag cagctttgct ccttcgcttt ctgggctcag aggctgggaa 3780
   ggggtgggtc cgggggcggg ctcaggggcg ggctcagggg cggggcgggc gcccgaaggt 3840
   cctccggagg cccggcattc tgcacgcttc aaaagcgcac gtctgccgcg ctgttctcct 3900
   cttcctcatc tccgggcctt tcgacctcta ggatcccgcc accatggtga gcaagggcga 3960
   ggaggataac atggccatca tcaaggagtt catgcgcttc aaggtgcaca tggagggctc 4020
   cgtgaacggc cacgagttcg agatcgaggg cgagggcgag ggccgcccct acgagggcac 4080
   ccagaccgcc aagctgaagg tgaccaaggg tggccccctg cccttcgcct gggacatcct 4140
   gtcccctcag ttcatgtacg gctccaaggc ctacgtgaag caccccgccg acatccccga 4200
   ctacttgaag ctgtccttcc ccgagggctt caagtgggag cgcgtgatga acttcgagga 4260
   cggcggcgtg gtgaccgtga cccaggactc ctccctgcag gacggcgagt tcatctacaa 4320
   ggtgaagctg cgcggcacca acttcccctc cgacggcccc gtaatgcaga agaagaccat 4380
   gggctgggag gcctcctccg agcggatgta ccccgaggac ggcgccctga agggcgagat 4440
   caagcagagg ctgaagctga aggacggcgg ccactacgac gctgaggtca agaccaccta 4500
   caaggccaag aagcccgtgc agctgcccgg cgcctacaac gtcaacatca agttggacat 4560
   cacctcccac aacgaggact acaccatcgt ggaacagtac gaacgcgccg agggccgcca 4620
   ctccaccggc ggcatggacg agctgtacaa gtaaagcggc cgcgtcgaga gatgggggag 4680
   gctaactgag ctagcttagt catgcaccgg gctgcaggaa ttcgcggccg cactcgaggg 4740
   aattccgata atcaacctct ggattacaaa atttgtgaaa gattgactgg tattcttaac 4800
   tatgttgctc cttttacgct atgtggatac gctgctttaa tgcctttgta tcatgctatt 4860
   gcttcccgta tggctttcat tttctcctcc ttgtataaat cctggttgct gtctctttat 4920
   gaggagttgt ggcccgttgt caggcaacgt ggcgtggtgt gcactgtgtt tgctgacgca 4980
   acccccactg gttggggcat tgccaccacc tgtcagctcc tttccgggac tttcgctttc 5040
   cccctcccta ttgccacggc ggaactcatc gccgcctgcc ttgcccgctg ctggacaggg 5100
   gctcggctgt tgggcactga caattccgtg gtgttgtcgg ggaagctgac gtcctttcca 5160
   tggctgctcg cctgtgttgc cacctggatt ctgcgcggga cgtccttctg ctacgtccct 5220
   tcggccctca atccagcgga ccttccttcc cgcggcctgc tgccggctct gcggcctctt 5280
   ccgcgtcttc gccttcgccc tcagacgagt cggatctccc tttgggccgc ctccccgcat 5340
   cgggaattcg agctcggtac ctttaaaacc aatgacttac aaggcagctg taaatcttag 5400
   ccacttttta aaagaaaagg ggggactgga agggctaatt cactcccaac gaaaacaaaa 5460
   tctgcttttt gcttgtactg ggtctctctg gttagaccaa atctgagcct gggagctctc 5520
   tggctaacta gggaacccac tgcttaagcc tcaataaagc ttgccttgag tgcttcaagt 5580
   agtgtgtgcc cgtctgttgt gtgactctgg taactagaga tccctcaaac ccttttagtc 5640
   agtgtggaaa atctctagca gcatctagaa ttaattccgt gtattctata gtgtcaccta 5700
   aatcgtatgt gtatgataca taaggttatg tattaattgt agccgcgttc taacgacaat 5760
   atgtacaagc ctaattgtgt agcatctggc ttactgaagc agaccctatc atctctctcg 5820
   taaactgccg tcagagtcgg tttggttgga cgaaccttct gagtttctgg taacgccgtc 5880
   ccgcacccgg aaatggtcag cgaaccaatc agcagggtca tcgctagcca gatcctctac 5940
   gccggacgca tcgtggccgg catcaccggc gccacaggtg cggttgctgg cgcctatatc 6000
   gccgacatca ccgatgggga agatcgggct cgccacttcg ggctcatgag cgcttgtttc 6060
   ggcgtgggta tggtggcagg ccccgtggcc gggggactgt tgggcgccat ctccttgcat 6120
   gcaccattcc ttgcggcggc ggtgctcaac ggcctcaacc tactactggg ctgcttccta 6180
   atgcaggagt cgcataaggg agagcgtcga tatggtgcac tctcagtaca atctgctctg 6240
   atgccgcata gttaagccag ccccgacacc cgccaacacc cgctgacgcg ccctgacggg 6300
   cttgtctgct cccggcatcc gcttacagac aagctgtgac cgtctccggg agctgcatgt 6360
   gtcagaggtt ttcaccgtca tcaccgaaac gcgcgagacg aaagggcctc gtgatacgcc 6420
   tatttttata ggttaatgtc atgataataa tggtttctta gacgtcaggt ggcacttttc 6480
   ggggaaatgt gcgcggaacc cctatttgtt tatttttcta aatacattca aatatgtatc 6540
   cgctcatgag acaataaccc tgataaatgc ttcaataata ttgaaaaagg aagagtatga 6600
   gtattcaaca tttccgtgtc gcccttattc ccttttttgc ggcattttgc cttcctgttt 6660
   ttgctcaccc agaaacgctg gtgaaagtaa aagatgctga agatcagttg ggtgcacgag 6720
   tgggttacat cgaactggat ctcaacagcg gtaagatcct tgagagtttt cgccccgaag 6780
   aacgttttcc aatgatgagc acttttaaag ttctgctatg tggcgcggta ttatcccgta 6840
   ttgacgccgg gcaagagcaa ctcggtcgcc gcatacacta ttctcagaat gacttggttg 6900
   agtactcacc agtcacagaa aagcatctta cggatggcat gacagtaaga gaattatgca 6960
   gtgctgccat aaccatgagt gataacactg cggccaactt acttctgaca acgatcggag 7020
   gaccgaagga gctaaccgct tttttgcaca acatggggga tcatgtaact cgccttgatc 7080
   gttgggaacc ggagctgaat gaagccatac caaacgacga gcgtgacacc acgatgcctg 7140
   tagcaatggc aacaacgttg cgcaaactat taactggcga actacttact ctagcttccc 7200
   ggcaacaatt aatagactgg atggaggcgg ataaagttgc aggaccactt ctgcgctcgg 7260
   cccttccggc tggctggttt attgctgata aatctggagc cggtgagcgt gggtctcgcg 7320
   gtatcattgc agcactgggg ccagatggta agccctcccg tatcgtagtt atctacacga 7380
   cggggagtca ggcaactatg gatgaacgaa atagacagat cgctgagata ggtgcctcac 7440
   tgattaagca ttggtaactg tcagaccaag tttactcata tatactttag attgatttaa 7500
   aacttcattt ttaatttaaa aggatctagg tgaagatcct ttttgataat ctcatgacca 7560
   aaatccctta acgtgagttt tcgttccact gagcgtcaga ccccgtagaa aagatcaaag 7620
   gatcttcttg agatcctttt tttctgcgcg taatctgctg cttgcaaaca aaaaaaccac 7680
   cgctaccagc ggtggtttgt ttgccggatc aagagctacc aactcttttt ccgaaggtaa 7740
   ctggcttcag cagagcgcag ataccaaata ctgtccttct agtgtagccg tagttaggcc 7800
   accacttcaa gaactctgta gcaccgccta catacctcgc tctgctaatc ctgttaccag 7860
   tggctgctgc cagtggcgat aagtcgtgtc ttaccgggtt ggactcaaga cgatagttac 7920
   cggataaggc gcagcggtcg ggctgaacgg ggggttcgtg cacacagccc agcttggagc 7980
   gaacgaccta caccgaactg agatacctac agcgtgagct atgagaaagc gccacgcttc 8040
   ccgaagggag aaaggcggac aggtatccgg taagcggcag ggtcggaaca ggagagcgca 8100
   cgagggagct tccaggggga aacgcctggt atctttatag tcctgtcggg tttcgccacc 8160
   tctgacttga gcgtcgattt ttgtgatgct cgtcaggggg gcggagccta tggaaaaacg 8220
   ccagcaacgc ggccttttta cggttcctgg ccttttgctg gccttttgct cacatgttct 8280
   ttcctgcgtt atcccctgat tctgtggata accgtattac cgcctttgag tgagctgata 8340
   ccgctcgccg cagccgaacg accgagcgca gcgagtcagt gagcgaggaa gcggaagagc 8400
   gcccaatacg caaaccgcct ctccccgcgc gttggccgat tcattaatgc agctgtggaa 8460
   tgtgtgtcag ttagggtgtg gaaagtcccc aggctcccca gcaggcagaa gtatgcaaag 8520
   catgcatctc aattagtcag caaccaggtg tggaaagtcc ccaggctccc cagcaggcag 8580
   aagtatgcaa agcatgcatc tcaattagtc agcaaccata gtcccgcccc taactccgcc 8640
   catcccgccc ctaactccgc ccagttccgc ccattctccg ccccatggct gactaatttt 8700
   ttttatttat gcagaggccg aggccgcctc ggcctctgag ctattccaga agtagtgagg 8760
   aggctttttt ggaggcctag gcttttgcaa aaagcttgga cacaagacag gcttgcgaga 8820
   tatgtttgag aataccactt tatcccgcgt cagggagagg cagtgcgtaa aaagacgcgg 8880
   actcatgtga aatactggtt tttagtgcgc cagatctcta taatctcgcg caacctattt 8940
   tcccctcgaa cactttttaa gccgtagata aacaggctgg gacacttcac atgagcgaaa 9000
   aatacatcgt cacctgggac atgttgcaga tccatgcacg taaactcgca agccgactga 9060
   tgccttctga acaatggaaa ggcattattg ccgtaagccg tggcggtctg taccgggtgc 9120
   gttactggcg cgtgaactgg gtattcgtca tgtcgatacc gtttgtattt ccagctacga 9180
   tcacgacaac cagcgcgagc ttaaagtgct gaaacgcgca gaaggcgatg gcgaaggctt 9240
   catcgttatt gatgacctgg tggataccgg tggtactgcg gttgcgattc gtgaaatgta 9300
   tccaaaagcg cactttgtca ccatcttcgc aaaaccggct ggtcgtccgc tggttgatga 9360
   ctatgttgtt gatatcccgc aagatacctg gattgaacag ccgtgggata tgggcgtcgt 9420
   attcgtcccg ccaatctccg gtcgctaatc ttttcaacgc ctggcactgc cgggcgttgt 9480
   tctttttaac ttcaggcggg ttacaatagt ttccagtaag tattctggag gctgcatcca 9540
   tgacacaggc aaacctgagc gaaaccctgt tcaaaccccg ctttaaacat cctgaaacct 9600
   cgacgctagt ccgccgcttt aatcacggcg cacaaccgcc tgtgcagtcg gcccttgatg 9660
   gtaaaaccat ccctcactgg tatcgcatga ttaaccgtct gatgtggatc tggcgcggca 9720
   ttgacccacg cgaaatcctc gacgtccagg cacgtattgt gatgagcgat gccgaacgta 9780
   ccgacgatga tttatacgat acggtgattg gctaccgtgg cggcaactgg atttatgagt 9840
   gggccccgga tctttgtgaa ggaaccttac ttctgtggtg tgacataatt ggacaaacta 9900
   cctacagaga tttaaagctc taaggtaaat ataaaatttt taagtgtata atgtgttaaa 9960
   ctactgattc taattgtttg tgtattttag attccaacct atggaactga tgaatgggag 10020
   cagtggtgga atgcctttaa tgaggaaaac ctgttttgct cagaagaaat gccatctagt 10080
   gatgatgagg ctactgctga ctctcaacat tctactcctc caaaaaagaa gagaaaggta 10140
   gaagacccca aggactttcc ttcagaattg ctaagttttt tgagtcatgc tgtgtttagt 10200
   aatagaactc ttgcttgctt tgctatttac accacaaagg aaaaagctgc actgctatac 10260
   aagaaaatta tggaaaaata ttctgtaacc tttataagta ggcataacag ttataatcat 10320
   aacatactgt tttttcttac tccacacagg catagagtgt ctgctattaa taactatgct 10380
   caaaaattgt gtacctttag ctttttaatt tgtaaagggg ttaataagga atatttgatg 10440
   tatagtgcct tgactagaga tcataatcag ccataccaca tttgtagagc ttttacttgc 10500
   tttaaaaaac ctcccacacc tccccctgaa cctgaaacat aaaatgaatg caattgttgt 10560
   tgttaacttg tttattgcag cttataatgg ttacaaataa agcaatagca tcacaaattt 10620
   cacaaataaa gcattttttt cactgcattc tagttgtggt ttgtccaaac tcatcaatgt 10680
   atcttatcat gtgtggatca actggataac tcaagctaac caaaatcatc ccaaacttcc 10740
   caccccatac cctattacca ctgccaaatt acctgtggtt tcatttactc taaacctgtg 10800
   attcctctga attattttca ttttaaagaa attgtatttg ttaaatatgt actacaaact 10860
   tagtagt 10867
<210> 13
   <211> 20
   <212> DNA
   <213> Artificial Sequence
<220>
   <223> synthetic
<220>
   <221> misc_feature
   <222> (19) .. (19)
   <223> n= G or A
<400> 13
   ggtaatttgt gtttgtgtnc 20
<210> 14
   <211> 20
   <212> DNA
   <213> Artificial Sequence
<220>
   <223> synthetic
<220>
   <221> misc_feature
   <222> (18)..(18)
   <223> n= T or C
<400> 14
   gacagcagag aaacagcngt 20
<210> 15
   <211> 20
   <212> DNA
   <213> Artificial Sequence
<220>
   <223> synthetic
<220>
   <221> misc_feature
   <222> (11)..(11)
   <223> n= T or C
<400> 15
   gagaaacagc ngttagttcc 20
<210> 16
   <211> 20
   <212> DNA
   <213> Artificial Sequence
<220>
   <223> synthetic
<220>
   <221> misc_feature
   <222> (19) .. (19)
   <223> n= C or G
<400> 16
   tggctaaagt aggctttant 20
<210> 17
   <211> 20
   <212> DNA
   <213> Artificial Sequence
<220>
   <223> synthetic
<220>
   <221> misc_feature
   <222> (19) .. (19)
   <223> n= T or C
<400> 17
   cagggctgtc cgggtgagna 20
<210> 18
   <211> 20
   <212> DNA
   <213> Artificial Sequence
<220>
   <223> synthetic
<220>
   <221> misc_feature
   <222> (13)..(13)
   <223> n=C or T
<400> 18
   tgtccgggtg agnatggctc 20
<210> 19
   <211> 20
   <212> DNA
   <213> Artificial Sequence
<220>
   <223> synthetic
<220>
   <221> misc_feature
   <222> (20) .. (20)
   <223> n= C or G or A
<400> 19
   aggcccatgc ggaaaggatn 20
<210> 20
   <211> 20
   <212> DNA
   <213> Artificial Sequence
<220>
   <223> synthetic
<220>
   <221> misc_feature
   <222> (8)..(8)
   <223> n= G or C or T
<400> 20
   ggcggggnat cctttccgca 20
<210> 21
   <211> 20
   <212> DNA
   <213> Artificial Sequence
<220>
   <223> synthetic
<220>
   <221> misc_feature
   <222> (17)..(17)
   <223> n= C or G
<400> 21
   ggggctcaac ggagagngga 20
<210> 22
   <211> 20
   <212> DNA
   <213> Artificial Sequence
<220>
   <223> synthetic
<220>
   <221> misc_feature
   <222> (19) .. (19)
   <223> n= A or G
<400> 22
   ggcagaacct gcgggggcng 20
<210> 23
   <211> 20
   <212> DNA
   <213> Artificial Sequence
<220>
   <223> synthetic
<220>
   <221> misc_feature
   <222> (15)..(15)
   <223> n= A or G
<400> 23
   gaacctgcgg gggcnggggc 20
<210> 24
   <211> 20
   <212> DNA
   <213> Artificial Sequence
<220>
   <223> synthetic
<220>
   <221> misc_feature
   <222> (17)..(17)
   <223> n= A or T
<400> 24
   attttaccag tattccngtc 20
<210> 25
   <211> 20
   <212> DNA
   <213> Artificial Sequence
<220>
   <223> synthetic
<220>
   <221> misc_feature
   <222> (20) .. (20)
   <223> n= C or T
<400> 25
   ctgtgtgtcg agtgtacagt 20
<210> 26
   <211> 20
   <212> DNA
   <213> Artificial Sequence
<220>
   <223> synthetic
<220>
   <221> misc_feature
   <222> (20) .. (20)
   <223> n= C or T
<400> 26
   ataaaaataa gttaacactn 20
<210> 27
   <211> 20
   <212> DNA
   <213> Artificial Sequence
<220>
   <223> synthetic
<220>
   <221> misc_feature
   <222> (14)..(14)
   <223> n= G or A
<400> 27
   gcaggagaat ggcntgaact 20
<210> 28
   <211> 20
   <212> DNA
   <213> Artificial Sequence
<220>
   <223> synthetic
<220>
   <221> misc_feature
   <222> (12)..(12)
   <223> n= C or T
<400> 28
   ataatatagt anggccactc 20
<210> 29
   <211> 20
   <212> DNA
   <213> Artificial Sequence
<220>
   <223> synthetic
<220>
   <221> misc_feature
   <222> (19) .. (19)
   <223> n= G or A
<400> 29
   cataaaactg aacataccnt 20
<210> 30
   <211> 20
   <212> DNA
   <213> Artificial Sequence
<220>
   <223> synthetic
<220>
   <221> misc_feature
   <222> (15)..(15)
   <223> n=A or G
<400> 30
   agcaattggg caacntcgtg 20
<210> 31
   <211> 20
   <212> DNA
   <213> Artificial Sequence
<220>
   <223> syntetic
<220>
   <221> misc_feature
   <222> (17)..(17)
   <223> n= T or C
<400> 31
   agacggggcc tcacgangtt 20
<210> 32
   <211> 20
   <212> DNA
   <213> Artificial Sequence
<220>
   <223> synthetic
<220>
   <221> misc_feature
   <222> (20) .. (20)
   <223> n= T or C
<400> 32
   agaaggtgct gctagttcan 20
<210> 33
   <211> 20
   <212> DNA
   <213> Artificial Sequence
<220>
   <223> synthetic
<220>
   <221> misc_feature
   <222> (12)..(12)
   <223> n= G or A
<220>
   <221> misc_feature
   <222> (14)..(14)
   <223> n= G or A
<400> 33
   tgtgtgtgtg tntntatatt 20
<210> 34
   <211> 20
   <212> DNA
   <213> Artificial Sequence
<220>
   <223> synthetic
<220>
   <221> misc_feature
   <222> (19) .. (19)
   <223> n= A or - deletion
<400> 34
   gttgtggttg ccaagtaang 20
<210> 35
   <211> 20
   <212> DNA
   <213> Artificial Sequence
<220>
   <223> synthetic
<220>
   <221> misc_feature
   <222> (14)..(14)
   <223> n= A or - deletion
<400> 35
   ggagtggcgg ggcngggggg 20
<210> 36
   <211> 20
   <212> DNA
   <213> Artificial Sequence
<220>
   <223> synthetic
<400> 36
   cgggacgggt ccaagatgga 20
<210> 37
   <211> 20
   <212> DNA
   <213> Artificial Sequence
<220>
   <223> synthetic
<400> 37
   gggctgtcaa tcatgctggc 20
<210> 38
   <211> 20
   <212> DNA
   <213> Artificial Sequence
<220>
   <223> synthetic
<400> 38
   cttttccagg gtcgccatgg 20
<210> 39
   <211> 20
   <212> DNA
   <213> Artificial Sequence
<220>
   <223> synthetic
<400> 39
   gacaatatgt gaaaacatag 20
<210> 40
   <211> 20
   <212> DNA
   <213> Artificial Sequence
<220>
   <223> synthetic
<400> 40
   aatggagtac ttcttgtcca 20
<210> 41
   <211> 4221
   <212> DNA
   <213> Artificial Sequence
<220>
   <223> synthetic
<400> 41
   atgccaactt tgtacaaaaa agcaggctcc accatggaca agaagtactc cattgggctc      60
   gatatcggca caaacagcgt cggctgggcc gtcattacgg acgagtacaa ggtgccgagc     120
   aaaaaattca aagttctggg caataccgat cgccacagca taaagaagaa cctcattggc     180
   gccctcctgt tcgactccgg ggagacggcc gaagccacgc ggctcaaaag aacagcacgg     240
   cgcagatata cccgcagaaa gaatcggatc tgctacctgc aggagatctt tagtaatgag     300
   atggctaagg tggatgactc tttcttccat aggctggagg agtccttttt ggtggaggag     360
   gataaaaagc acgagcgcca cccaatcttt ggcaatatcg tggacgaggt ggcgtaccat     420
   gaaaagtacc caaccatata tcatctgagg aagaagcttg tagacagtac tgataaggct     480
   gacttgcggt tgatctatct cgcgctggcg catatgatca aatttcgggg acacttcctc     540
   atcgaggggg acctgaaccc agacaacagc gatgtcgaca aactctttat ccaactggtt     600
   cagacttaca atcagctttt cgaagagaac ccgatcaacg catccggagt tgacgccaaa     660
   gcaatcctga gcgctaggct gtccaaatcc cggcggctcg aaaacctcat cgcacagctc     720
   cctggggaga agaagaacgg cctgtttggt aatcttatcg ccctgtcact cgggctgacc     780
   cccaacttta aatctaactt cgacctggcc gaagatgcca agcttcaact gagcaaagac     840
   acctacgatg atgatctcga caatctgctg gcccagatcg gcgaccagta cgcagacctt     900
   tttttggcgg caaagaacct gtcagacgcc attctgctga gtgatattct gcgagtgaac     960
   acggagatca ccaaagctcc gctgagcgct agtatgatca agcgctatga tgagcaccac    1020
   caagacttga ctttgctgaa ggcccttgtc agacagcaac tgcctgagaa gtacaaggaa    1080
   attttcttcg atcagtctaa aaatggctac gccggataca ttgacggcgg agcaagccag    1140
   gaggaatttt acaaatttat taagcccatc ttggaaaaaa tggacggcac cgaggagctg    1200
   ctggtaaagc ttaacagaga agatctgttg cgcaaacagc gcactttcga caatggaagc    1260
   atcccccacc agattcacct gggcgaactg cacgctatcc tcaggcggca agaggatttc    1320
   tacccctttt tgaaagataa cagggaaaag attgagaaaa tcctcacatt tcggataccc    1380
   tactatgtag gccccctcgc ccggggaaat tccagattcg cgtggatgac tcgcaaatca    1440
   gaagagacca tcactccctg gaacttcgag gaagtcgtgg ataagggggc ctctgcccag    1500
   tccttcatcg aaaggatgac taactttgat aaaaatctgc ctaacgaaaa ggtgcttcct    1560
   aaacactctc tgctgtacga gtacttcaca gtttataacg agctcaccaa ggtcaaatac    1620
   gtcacagaag ggatgagaaa gccagcattc ctgtctggag agcagaagaa agctatcgtg    1680
   gacctcctct tcaagacgaa ccggaaagtt accgtgaaac agctcaaaga agactatttc    1740
   aaaaagattg aatgtttcga ctctgttgaa atcagcggag tggaggatcg cttcaacgca    1800
   tccctgggaa cgtatcacga tctcctgaaa atcattaaag acaaggactt cctggacaat 1860
   gaggagaacg aggacattct tgaggacatt gtcctcaccc ttacgttgtt tgaagatagg 1920
   gagatgattg aagaacgctt gaaaacttac gctcatctct tcgacgacaa agtcatgaaa 1980
   cagctcaaga ggcgccgata tacaggatgg gggcggctgt caagaaaact gatcaatggg 2040
   atccgagaca agcagagtgg aaagacaatc ctggattttc ttaagtccga tggatttgcc 2100
   aaccggaact tcatgcagtt gatccatgat gactctctca cctttaagga ggacatccag 2160
   aaagcacaag tttctggcca gggggacagt cttcacgagc acatcgctaa tcttgcaggt 2220
   agcccagcta tcaaaaaggg aatactgcag accgttaagg tcgtggatga actcgtcaaa 2280
   gtaatgggaa ggcataagcc cgagaatatc gttatcgaga tggcccgaga gaaccaaact 2340
   acccagaagg gacagaagaa cagtagggaa aggatgaaga ggattgaaga gggtataaaa 2400
   gaactggggt cccaaatcct taaggaacac ccagttgaaa acacccagct tcagaatgag 2460
   aagctctacc tgtactacct gcagaacggc agggacatgt acgtggatca ggaactggac 2520
   atcaatcggc tctccgacta cgacgtggat catatcgtgc cccagtcttt tctcaaagat 2580
   gattctattg ataataaagt gttgacaaga tccgataaaa atagagggaa gagtgataac 2640
   gtcccctcag aagaagttgt caagaaaatg aaaaattatt ggcggcagct gctgaacgcc 2700
   aaactgatca cacaacggaa gttcgataat ctgactaagg ctgaacgagg tggcctgtct 2760
   gagttggata aagccggctt catcaaaagg cagcttgttg agacacgcca gatcaccaag 2820
   cacgtggccc aaattctcga ttcacgcatg aacaccaagt acgatgaaaa tgacaaactg 2880
   attcgagagg tgaaagttat tactctgaag tctaagctgg tctcagattt cagaaaggac 2940
   tttcagtttt ataaggtgag agagatcaac aattaccacc atgcgcatga tgcctacctg 3000
   aatgcagtgg taggcactgc acttatcaaa aaatatccca agcttgaatc tgaatttgtt 3060
   tacggagact ataaagtgta cgatgttagg aaaatgatcg caaagtctga gcaggaaata 3120
   ggcaaggcca ccgctaagta cttcttttac agcaatatta tgaatttttt caagaccgag 3180
   attacactgg ccaatggaga gattcggaag cgaccactta tcgaaacaaa cggagaaaca 3240
   ggagaaatcg tgtgggacaa gggtagggat ttcgcgacag tccggaaggt cctgtccatg 3300
   ccgcaggtga acatcgttaa aaagaccgaa gtacagaccg gaggcttctc caaggaaagt 3360
   atcctcccga aaaggaacag cgacaagctg atcgcacgca aaaaagattg ggaccccaag 3420
   aaatacggcg gattcgattc tcctacagtc gcttacagtg tactggttgt ggccaaagtg 3480
   gagaaaggga agtctaaaaa actcaaaagc gtcaaggaac tgctgggcat cacaatcatg 3540
   gagcgatcaa gcttcgaaaa aaaccccatc gactttctcg aggcgaaagg atataaagag 3600
   gtcaaaaaag acctcatcat taagcttccc aagtactctc tctttgagct tgaaaacggc 3660
   cggaaacgaa tgctcgctag tgcgggcgag ctgcagaaag gtaacgagct ggcactgccc 3720
   tctaaatacg ttaatttctt gtatctggcc agccactatg aaaagctcaa agggtctccc    3780
   gaagataatg agcagaagca gctgttcgtg gaacaacaca aacactacct tgatgagatc    3840
   atcgagcaaa taagcgaatt ctccaaaaga gtgatcctcg ccgacgctaa cctcgataag    3900
   gtgctttctg cttacaataa gcacagggat aagcccatca gggagcaggc agaaaacatt    3960
   atccacttgt ttactctgac caacttgggc gcgcctgcag ccttcaagta cttcgacacc    4020
   accatagaca gaaagcggta cacctctaca aaggaggtcc tggacgccac actgattcat    4080
   cagtcaatta cggggctcta tgaaacaaga atcgacctct ctcagctcgg tggagacagc    4140
   agggctgacc ccaagaagaa gaggaaggtg ggtaccggta agcctatccc taaccctctc    4200
   ctcggtctcg attctacgtg a                                                4221
<210> 42
   <211> 4885
   <212> DNA
   <213> Homo sapiens
<400> 42
   cttactccct gctgggcagg ctggacagct gccctctccg tttgcccctc taccacccaa      60
   aaggcaggag gctctggaga ccaggaccct gcccgccacg gcctgtgtcc caggcgtgag     120
   ggggtgcccc acagacctct gctgagctgc tgctgaatga cgccccttgg gggtcctgcc     180
   ggaaggtcag agcaggggtg cactcccata aagaaacgcc cccaggtcgg gactcattcc     240
   tgtgggcggc atcttgtggc catagctgct tctcgctgca ctaatcacag tgcctctgtg     300
   ggcagcaggc gctgaccacc caggcctgcc ccagaccctc tcctcccttc cggggcgctg     360
   cgctgggacc gatggggggc gccaggcctg tggacaccgc cctgcagggg cctctccagc     420
   tcactggggg tggggtgggg gtcacacttg gggtcctcag gtcgtgccga ccacgcgcat     480
   tctctgcgct ctgcgcagga gctcgcccac cctctccccg tgcagagagc cccgcagctg     540
   gctccccgca gggctgtccg ggtgagtatg gctctggcca cgggccagtg tggcgggagg     600
   gcaaacccca aggccacctc ggctcagagt ccacggccgg ctgtcgcccc gctccaggcg     660
   tcggcggggg atcctttccg catgggcctg cgcccgcgct cggcgccccc tccacggccc     720
   cgccccgtcc atggccccgt ccttcatggg cgagcccctc catggccctg cccctccgcg     780
   ccccacccct ccctcgcccc acctctcacc ttcctgcccc gcccccagcc tccccacccc     840
   tcaccggcca gtcccctccc ctatcccgct ccgcccctca gccgccccgc ccctcagccg     900
   gcctgcctaa tgtccccgtc cccagcatcg ccccgccccg cccccgtctc gccccgcccc     960
   tcaggcggcc tccctgctgt gccccgcccc ggcctcgcca cgcccctacc tcaccacgcc    1020
   ccccgcatcg ccacgccccc cgcatcgcca cgcctccctt accatgcagt cccgccccgt    1080
   cccttcctcg tcccgcctcg ccgcgacact tcacacacag cttcgcctca ccccattaca    1140
   gtctcaccac gccccgtccc ctctccgttg agccccgcgc cttcgcccgg gtggggcgct 1200
   gcgctgtcag cggccttgct gtgtgaggca gaacctgcgg gggcaggggc gggctggttc 1260
   cctggccagc cattggcaga gtccgcaggc tagggctgtc aatcatgctg gccggcgtgg 1320
   ccccgcctcc gccggcgcgg ccccgcctcc gccggcgcag cgtctgggac gcaaggcgcc 1380
   gtgggggctg ccgggacggg tccaagatgg acggccgctc aggttctgct tttacctgcg 1440
   gcccagagcc ccattcattg ccccggtgct gagcggcgcc gcgagtcggc ccgaggcctc 1500
   cggggactgc cgtgccgggc gggagaccgc catggcgacc ctggaaaagc tgatgaaggc 1560
   cttcgagtcc ctcaagtcct tccagcagca acagcagcaa cagcagcaac agcagcaaca 1620
   gcagcaacag cagcaacagc aacagccgcc accgccgccg ccgccgccgc cgcctcctca 1680
   gcttcctcag ccgccgccgc aggcacagcc gctgctgcct cagccgcagc cgcccccgcc 1740
   gccgcccccg ccgccacccg gcccggctgt ggctgaggag ccgctgcacc gaccgtgagt 1800
   ttgggcccgc tgcagctccc tgtcccggcg ggtcccaggc tacggcgggg atggcggtaa 1860
   ccctgcagcc tgcgggccgg cgacacgaac ccccggcccc gcagagacag agtgacccag 1920
   caacccagag cccatgaggg acacccgccc cctcctgggg cgaggccttc ccccacttca 1980
   gccccgctcc ctcacttggg tcttcccttg tcctctcgcg aggggaggca gagccttgtt 2040
   ggggcctgtc ctgaattcac cgaggggagt cacggcctca gccctctcgc ccttcgcagg 2100
   atgcgaagag ttggggcgag aacttgtttc tttttatttg cgagaaacca gggcgggggt 2160
   tcttttaact gcgttgtgaa gagaacttgg aggagccgag atttgctcag tgccacttcc 2220
   ctcttctagt ctgagaggga agagggctgg gggcgcggga cacttcgaga ggaggcgggg 2280
   tttggagctg gagagatgtg ggggcagtgg atgacataat gcttttagga cgcctcggcg 2340
   ggagtggcgg ggcagggggg gggcggggag tgagggcgcg tccaatggga gatttctttt 2400
   cctagtggca cttaaaacag cctgagattt gaggctcttc ctacattgtc aggacatttc 2460
   atttagttca tgatcacggt ggtagtaaca cgattttaag caccacctaa gagatctgct 2520
   catctaagcc taagttggtc tgcaggcgtt tgaatgagtt gtggttgcca agtaaagtgg 2580
   tgaacttacg tggtgattaa tgaaattatc ttaaatatta ggaagagttg attgaagttt 2640
   tttgcctatg tgtgttggga ataaaaccaa cacgttgctg atggggaggt taattgccga 2700
   gggatgaatg aggtgtacat tttaccagta ttccagtcag gcttgccaga atacgggggg 2760
   tccgcagact ccgtgggcat ctcagatgtg ccagtgaaag ggtttctgtt tgcttcattg 2820
   ctgacagctt gttacttttt ggaagctagg ggtttctgtt gcttgttctt ggggagaatt 2880
   tttgaaacag gaaaagagag accattaaaa catctagcgg aaccccagga ctttccctgg 2940
   aagtctgtgt gtcgagtgta cagtaggagt taggaagtac tctggtgcag ttcaggcctt 3000
   tctcttacct ctcagtattc tatttccgat ctggatgtgt cccagatggc atttggtaag 3060
   aatatctctg ttaagactga ttaattttta gtaatatttc ttgttctttg tttctgttat 3120
   gatccttgtc tcgtcttcaa agtttaatta gaaaatgatt cggagagcag tgttagctta 3180
   tttgttggaa taaaatttag gaataaatta ttctaaagga tggaaaaact ttttggatat 3240
   ttggagaaat tttaaaacaa tttggcttat ctcttcagta agtaatttct catccagaaa 3300
   tttactgtag tgcttttcta ggaggtaggt gtcataaaag ttcacacatt gcatgtatct 3360
   tgtgtaaaca ctaaacaggg ctcctgatgg gaaggaagac ctttctgctg ggctgcttca 3420
   gacacttgat cattctaaaa atatgccttc tctttcttat gctgatttga cagaacctgc 3480
   atttgcttat cttcaaaata tgggtatcaa gaaatttcct ttgctgcctt gacaaaggag 3540
   atagattttg tttcattact ttaaggtaat atatgattac cttatttaaa aaatttaatc 3600
   aggactggca aggtggctta cacctttaat ccgagcactt tgggaggcct aggtggacga 3660
   atcacctgag gtcaggagtt tgagaccagc ctggctaaca tggtgaaacc ctgtctctac 3720
   taaaaataca aaaattagct ggtcatggtg gcacgtgcct gtaatccaag ctacctggga 3780
   ggctgaggca ggaaaatcgc ttgaacccgg gaggcagagt ctgcagtgag ttgagatcac 3840
   gccactgcac tccagcctgg gtgacagagc gagactctat ctcaaaaaaa atttttttta 3900
   atgtattatt tttgcataag taatacattg acatgataca aattctgtaa ttacaaaagg 3960
   gcaataatta aaatatcttc cttccacccc tttcctctga gtacctaact ttgtccccaa 4020
   gaacaagcac tatttcagtt cctcatgtat cctgccagat ataacctgtt catattgtaa 4080
   gatagattta aaatgctcta aaaacaaaag tagtttagaa taatatatat ctatatattt 4140
   tttgagatgt agtctcacat tgtcacccag gctggagtgc agtgatacaa tctcggctca 4200
   ctgcagtctc tgcctcccag gttcaaatgc ttctcctgcc tcagccttct gagtagctgg 4260
   gattacaggc gcccaccacc atgtccagct aatttttgta tttttagtag agatggggtt 4320
   tcaccatgtt ggccaggctg gtcttgaact cctgaccttg tgatctgtcc acctcggcct 4380
   cccaaagtgc tgggattaca ggtgtgagcc accatgcctg gctagaataa taacttttaa 4440
   aggttcttag catgctctga aatcaactgc attaggttta tttatagttt tatagttatt 4500
   ttaaataaaa tgcatatttg tcatatttct ctgtattttg ctgttgagaa aggaggtatt 4560
   cactaatttt gagtaacaaa cactgctcac aaagtttgga ttttggcagt tctgttcacg 4620
   tgcttcagcc aaaaaatcct cttctcaaag taagattgat gaaagcaatt tagaaagtat 4680
   ctgttctgtt tttatggctc ttgctctttg gtgtggaact gtggtgtcac gccatgcatg 4740
   ggcctcagtt tatgagtgtt tgtgctctgc tcagcataca ggatgcagga gttccttatg 4800
   gggctggctg caggctcagc aaatctagca tgcttgggag ggtcctcaca gtaattagga 4860
   ggcaattaat acttgcttct ggcag 4885
<210> 43
   <211> 4840
   <212> DNA
   <213> Homo sapiens
<400> 43
   cttttacctg cggcccagag ccccattcat tgccccggtg ctgagcggcg ccgcgagtcg      60
   gcccgaggcc tccggggact gccgtgccgg gcgggagacc gccatggcga ccctggaaaa     120
   gctgatgaag gccttcgagt ccctcaagtc cttccagcag caacagcagc aacagcagca     180
   acagcagcaa cagcagcaac agcagcaaca gcaacagccg ccaccgccgc cgccgccgcc     240
   gccgcctcct cagcttcctc agccgccgcc gcaggcacag ccgctgctgc ctcagccgca     300
   gccgcccccg ccgccgcccc cgccgccacc cggcccggct gtggctgagg agccgctgca     360
   ccgaccgtga gtttgggccc gctgcagctc cctgtcccgg cgggtcccag gctacggcgg     420
   ggatggcggt aaccctgcag cctgcgggcc ggcgacacga acccccggcc ccgcagagac     480
   agagtgaccc agcaacccag agcccatgag ggacacccgc cccctcctgg ggcgaggcct     540
   tcccccactt cagccccgct ccctcacttg ggtcttccct tgtcctctcg cgaggggagg     600
   cagagccttg ttggggcctg tcctgaattc accgagggga gtcacggcct cagccctctc     660
   gcccttcgca ggatgcgaag agttggggcg agaacttgtt tctttttatt tgcgagaaac     720
   cagggcgggg gttcttttaa ctgcgttgtg aagagaactt ggaggagccg agatttgctc     780
   agtgccactt ccctcttcta gtctgagagg gaagagggct gggggcgcgg gacacttcga     840
   gaggaggcgg ggtttggagc tggagagatg tgggggcagt ggatgacata atgcttttag     900
   gacgcctcgg cgggagtggc ggggcagggg gggggcgggg agtgagggcg cgtccaatgg     960
   gagatttctt ttcctagtgg cacttaaaac agcctgagat ttgaggctct tcctacattg    1020
   tcaggacatt tcatttagtt catgatcacg gtggtagtaa cacgatttta agcaccacct    1080
   aagagatctg ctcatctaag cctaagttgg tctgcaggcg tttgaatgag ttgtggttgc    1140
   caagtaaagt ggtgaactta cgtggtgatt aatgaaatta tcttaaatat taggaagagt    1200
   tgattgaagt tttttgccta tgtgtgttgg gaataaaacc aacacgttgc tgatggggag    1260
   gttaattgcc gagggatgaa tgaggtgtac attttaccag tattccagtc aggcttgcca    1320
   gaatacgggg ggtccgcaga ctccgtgggc atctcagatg tgccagtgaa agggtttctg    1380
   tttgcttcat tgctgacagc ttgttacttt ttggaagcta ggggtttctg ttgcttgttc    1440
   ttggggagaa tttttgaaac aggaaaagag agaccattaa aacatctagc ggaaccccag    1500
   gactttccct ggaagtctgt gtgtcgagtg tacagtagga gttaggaagt actctggtgc    1560
   agttcaggcc tttctcttac ctctcagtat tctatttccg atctggatgt gtcccagatg    1620
   gcatttggta agaatatctc tgttaagact gattaatttt tagtaatatt tcttgttctt    1680
   tgtttctgtt atgatccttg tctcgtcttc aaagtttaat tagaaaatga ttcggagagc 1740
   agtgttagct tatttgttgg aataaaattt aggaataaat tattctaaag gatggaaaaa 1800
   ctttttggat atttggagaa attttaaaac aatttggctt atctcttcag taagtaattt 1860
   ctcatccaga aatttactgt agtgcttttc taggaggtag gtgtcataaa agttcacaca 1920
   ttgcatgtat cttgtgtaaa cactaaacag ggctcctgat gggaaggaag acctttctgc 1980
   tgggctgctt cagacacttg atcattctaa aaatatgcct tctctttctt atgctgattt 2040
   gacagaacct gcatttgctt atcttcaaaa tatgggtatc aagaaatttc ctttgctgcc 2100
   ttgacaaagg agatagattt tgtttcatta ctttaaggta atatatgatt accttattta 2160
   aaaaatttaa tcaggactgg caaggtggct tacaccttta atccgagcac tttgggaggc 2220
   ctaggtggac gaatcacctg aggtcaggag tttgagacca gcctggctaa catggtgaaa 2280
   ccctgtctct actaaaaata caaaaattag ctggtcatgg tggcacgtgc ctgtaatcca 2340
   agctacctgg gaggctgagg caggaaaatc gcttgaaccc gggaggcaga gtctgcagtg 2400
   agttgagatc acgccactgc actccagcct gggtgacaga gcgagactct atctcaaaaa 2460
   aaattttttt taatgtatta tttttgcata agtaatacat tgacatgata caaattctgt 2520
   aattacaaaa gggcaataat taaaatatct tccttccacc cctttcctct gagtacctaa 2580
   ctttgtcccc aagaacaagc actatttcag ttcctcatgt atcctgccag atataacctg 2640
   ttcatattgt aagatagatt taaaatgctc taaaaacaaa agtagtttag aataatatat 2700
   atctatatat tttttgagat gtagtctcac attgtcaccc aggctggagt gcagtgatac 2760
   aatctcggct cactgcagtc tctgcctccc aggttcaaat gcttctcctg cctcagcctt 2820
   ctgagtagct gggattacag gcgcccacca ccatgtccag ctaatttttg tatttttagt 2880
   agagatgggg tttcaccatg ttggccaggc tggtcttgaa ctcctgacct tgtgatctgt 2940
   ccacctcggc ctcccaaagt gctgggatta caggtgtgag ccaccatgcc tggctagaat 3000
   aataactttt aaaggttctt agcatgctct gaaatcaact gcattaggtt tatttatagt 3060
   tttatagtta ttttaaataa aatgcatatt tgtcatattt ctctgtattt tgctgttgag 3120
   aaaggaggta ttcactaatt ttgagtaaca aacactgctc acaaagtttg gattttggca 3180
   gttctgttca cgtgcttcag ccaaaaaatc ctcttctcaa agtaagattg atgaaagcaa 3240
   tttagaaagt atctgttctg tttttatggc tcttgctctt tggtgtggaa ctgtggtgtc 3300
   acgccatgca tgggcctcag tttatgagtg tttgtgctct gctcagcata caggatgcag 3360
   gagttcctta tggggctggc tgcaggctca gcaaatctag catgcttggg agggtcctca 3420
   cagtaattag gaggcaatta atacttgctt ctggcagttt cttattctcc ttcagattcc 3480
   tatctggtgt ttccctgact ttattcattc atcagtaaat atttactaaa catgtactat 3540
   gtgcctggca ctgttatagg tgcagggctc agcagtgagc agacaaagct ctgccctcgt 3600
   gaagctttca ttctaatgaa ggacatagac agtaagcaag atagataagt aaaatataca 3660
   gtacgttaat acgtggagga acttcaaagc agggaagggg atagggaaat gtcagggtta 3720
   atcgagtgtt aacttatttt tatttttaaa aaaattgtta agggctttcc agcaaaaccc 3780
   agaaagcctg ctagacaaat tccaaaagag ctgtagcact aagtgttgac atttttattt 3840
   tattttgttt tgttttgttt tttttgagac agttcttgct ctatcagcca ggctggagtg 3900
   cactagtgtg atcttggctc actgcaacct ctgcctcttg ggttcaagtg attctcatgc 3960
   ctcagcctcc tgtttagctg ggattataga catgcactgc catgcctggg taattttttt 4020
   tttttccccc gagacggagt cttgctctgt cgcccaggct ggagtgcagt ggcgcgatct 4080
   cagctcactg caagctccgc ttcccgagtt cacgccattc tcctgcctca gtctcccaag 4140
   tagctgggac tacaggcgcc tgccaccacg tccagctaat ttttttgtat ttttaataga 4200
   gacggggttt caccgtgtta gccaggatga tcttgatctc ctgacctcgt catccgccga 4260
   ccttgtgatc cgcccacctc ggcctcccaa agtgctggga ttacaggcat gagccactgt 4320
   gcccggccac gcctgggtaa tttttgtatt tttagtagag atggggtttt gccatgatga 4380
   gcaggctggt ctcgaactcc cggcctcatg tgatctgcct gccttggcct cccaaagtgc 4440
   taggattaca ggcatgagcc accatacctg gccagtgttg atattttaaa tacggtgttc 4500
   agggaaggtc cactgagaag acagcttttt tttttttttt ttttggggtt ggggggcaag 4560
   gtcttgctct ttaacccagg ctggaatgca gtatcactat cgtagctcac ttcagccttg 4620
   aactcctggg ctcaagtgat cctcccacct caacctcaca atgtgttggg actataggtg 4680
   tgagccatca cacctggcca gatgatggct tttgagtaaa gacctcaagc gagttaagag 4740
   tctagtgtaa gggtgtatga agtagtggta ttccagatgg ggggaacagg tccaaaatct 4800
   tcctgtttca ggaatagcaa ggatgtcatt ttagttgggt 4840
<210> 44
   <211> 21
   <212> DNA
   <213> Artificial Sequence
<220>
   <223> synthetic
<400> 44
   tgtgtgcccg tctgttgttg t 21
<210> 45
   <211> 20
   <212> DNA
   <213> Artificial Sequence
<220>
   <223> synthetic
<400> 45
   gagtcctgcg tcgagagagc 20
<210> 46
   <211> 21
   <212> DNA
   <213> Artificial Sequence
<220>
   <223> synthetic
<400> 46
   atggacggcc gctcaggttc t 21
<210> 47
   <211> 21
   <212> DNA
   <213> Artificial Sequence
<220>
   <223> synthetic
<400> 47
   gctcagcacc ggggcaatga a 21
<210> 48
   <211> 30
   <212> DNA
   <213> Artificial Sequence
<220>
   <223> synthetic
<400> 48
   acggcgagtt catctacaag gtgaagctgc 30
<210> 49
   <211> 20
   <212> DNA
   <213> Artificial Sequence
<220>
   <223> synthetic
<400> 49
   tgacaaatgg aagtagcacg 20
<210> 50
   <211> 30
   <212> DNA
   <213> Artificial Sequence
<220>
   <223> synthetic
<400> 50
   ttgtactcca gcttgtgccc caggatgttg 30
<210> 51
   <211> 23
   <212> DNA
   <213> Artificial Sequence
<220>
   <223> synthetic
<400> 51
   gagatgagtt tttgttcgaa ggg 23
<210> 52
   <211> 25
   <212> DNA
   <213> Artificial Sequence
<220>
   <223> synthetic
<400> 52
   ttgctgtgtg aggcagaacc tgcgg 25
<210> 53
   <211> 20
   <212> DNA
   <213> Artificial Sequence
<220>
   <223> synthetic
<400> 53
   tgcagcggct cctcagccac 20
<210> 54
   <211> 20
   <212> DNA
   <213> Artificial Sequence
<220>
   <223> synthetic
<400> 54
   cacttcacac acagcttcgc 20
<210> 55
   <211> 20
   <212> DNA
   <213> Artificial Sequence
<220>
   <223> synthetic
<400> 55
   tgctgctgga aggacttgag 20
<210> 56
   <211> 20
   <212> DNA
   <213> Artificial Sequence
<220>
   <223> synthetic
<400> 56
   tgacaaatgg aagtagcacg 20
<210> 57
   <211> 23
   <212> DNA
   <213> Artificial Sequence
<220>
   <223> synthetic
<400> 57
   gagatgagtt tttgttcgaa ggg 23
<210> 58
   <211> 22
   <212> DNA
   <213> Artificial Sequence
<220>
   <223> synthetic
<400> 58
   cctcctcact tcttttctat cg 22
<210> 59
   <211> 21
   <212> DNA
   <213> Artificial Sequence
<220>
   <223> synthetic
<400> 59
   agcattatgt catccactac c 21

## Description

### FIELD OF THE INVENTION

The invention relates to the treatment of Huntington's disease (HD) using the Clustered-Regularly Interspaced Short Palindromic Repeats (CRISPR) system. This technology offers the possibility to design a single guide RNA (sgRNA), which is incorporated into a CRISPR-associated protein (Cas9) to recognize and induce DNA double-strand breaks at a specific target location. DNA double-strand breaks will be repaired by cellular machinery either by non-homologous end joining (NHEJ) or homologous recombination (HR) in the presence of a donor sequence for HD gene repair. In the context of HD, this allows to block the expression of the mutant huntingtin (mHTT) or repair the CAG expansion causing the disease.

### BACKGROUND OF THE INVENTION

Huntington's disease (HD) is a monogenic neurodegenerative disease characterized by a global impairment leading to death in 15-20 years. Although precise mechanisms leading to neuronal death are not yet understood, a pathologic expansion of CAG repeats (more than 40 CAG) at the end of the exon 1 of the huntingtin gene (*HTT*) has been identified as the cause of HD. The huntingtin locus located in chromosome 4 is large, spanning 180 kb and consisting of 67 exons. In HD, the huntingtin protein (HTT) forms aggregates in cells, which interfere with normal cellular functions (1). In addition, normal function of HTT is altered and new pathologic interactions of mutant HTT (mHTT) with partners also participate to the neuronal death observed in HD (2). Currently, there are no curative treatments for HD but experimental approaches based on drug, cell and gene therapy are under investigation (3). One of the most promising is the silencing of *mHTT* with small-hairpin RNA (shRNA), which will either degrade wild-type (WT) and *mHTT* mRNA (4, 5) or induce an allele-specific silencing based on the presence of single nucleotide polymorphism (SNP) in the HTT genome and selective silencing of *mHTT mRNA* (6). Most of the patients are heterozygous for the disease-causing mutation. SNP with a high frequency in the human population have been used to selectively reduce the expression of the mutant HTT allele containing the matching SNP while leaving the expression of the WT allele (mismatched SNP) unaltered.

Recently, approaches modulating *HTT* gene expression have also been described, in particular with zinc finger and TALE repressors. Allele and non-allele specific targeting of mutant *HTT* using ZFP- or TALE-repressors were used to block the expression of the mutant *HTT* (7). ZFP targeting the expanded CAG were shown to preferentially repress the expression of the human *HTT* containing longer CAG repeats *in vitro.* In the R6/2 mice, the injection of an AAV2/1 vector expressing ZF11xHunt-Kox-1 reduces the level of mutant *HTT* mRNA and the accumulation of misfolded HTT in the striatum by 40% on average. Clasping behavior and motor coordination were also improved (7).

Based on the same principle, WO 2013/130824 (Sangamo Biosciences, Inc.) and Garriga-canut et al 2012 (7) disclose methods and compositions for treating or preventing Huntington's disease. In particular, these documents provide methods and compositions for modulating expression of a mutant HTT allele so as to treat Huntington's disease. Also provided are methods and compositions for generating animal models of Huntington's disease. The invention is based on ZFN and TALE with engineered DNA binding domain targeting the HTT gene. These proteins are in operative linkage with regulatory (or functional domain) as part of a fusion protein. The functional domain can be a transcriptional activation domain or a transcriptional repression domain. These strategies will therefore either repress or activate the expression of the human HTT gene (global or allele-specific). However, such methods require a permanent expression of the therapeutic gene and are not definitely modifying the *HTT*DNA sequence.

WO 2014/093701 (Feng Zhang, MIT, USA) and Cong et al 2014 (8) relate to compositions, methods, applications and screens used in functional genomics that focus on gene function in a cell and that may use vector systems and other aspects related to Clustered Regularly Interspaced Short Palindromic Repeats (CRISPR)-Cas systems and components thereof. Provided are vectors and vector systems, some of which encode one or more components of a CRISPR complex, as well as methods for the design and use of such vectors. Also provided are methods of directing CRISPR complex formation in eukaryotic cells and methods for utilizing the CRISPR-Cas system.

Huda Y. Zoghbi et al. (2000) "Glutamine Repeats and Neurodegeneration", Annual Review of Neuroscience, Vol. 23:217-247, disclose that a growing number of neurodegenerative diseases have been found to result from the expansion of an unstable trinucleotide repeat. Over the past 6 years, researchers have focused on identifying the mechanism by which the expanded polyglutamine tract renders a protein toxic to a subset of vulnerable neurons. In this review, authors summarize the clinicopathologic features of these disorders (spinobulbar muscular atrophy, Huntington disease, and the spinocerebellar ataxias, including dentatorubropallidoluysian atrophy), describe the genes involved and what is known about their products, and discuss the model systems that have lent insight into pathogenesis. The review concludes with a model for pathogenesis that illuminates the unifying features of these polyglutamine disorders. This model may prove relevant to other neurodegenerative disorders as well.

The present invention is based on entirely new and innovative strategies for allele or non-allele-specific *HTT* editing and is also describing gene repair strategies targeting the promoter, transcription and/or translation start sites and/or SNP in the *HTT* gene. HTT gene disruption based on sgRNA targeting the CAG expansion or double-strand break with overhang (2-4 sgRNA and Cas nickase) are not considered in the present invention.

The recently described method based on the bacterial clustered regularly interspaced short palindromic repeats (CRISPR) system is a promising tool for gene editing in mammals and offers a unique opportunity to irreversibly modify the *HTT* gene itself and develop therapeutic strategies based on DNA editing/repair (9). The CRISPR system contains two components: (i) a dual crRNA:tracrRNA which was engineered as an artificial single guide RNA (sgRNA) of approximately 20 nucleotides recognizing the target sequence by Watson-Crick base-pairing fused with the tracrRNA at the 3' side of the guide sequence that bind to Cas9 (tracrRNA), (ii) the second component of the system is the CRISPR-associated protein Cas9 nuclease cleaving at the target site. Any sequence of approximately 23 nucleotides including the protospacer adjacent motif at the 3' end of the target sequence (PAM = NGG or NAG for Cas9) and located on both strands of DNA could be a CRISPR target sequence, which provides a large number of potential targets for each gene and greatly facilitates the development of DNA repair strategies compared to the other systems. Sternberg and collaborators demonstrated that both binding and cleavage of DNA by Cas9 require recognition of the short trinucleotide PAM (10). The DNA endonuclease binds targeted nucleotides to perform double-strand breaks (DSB) and non-homologous end joining (NHEJ) with insertions or deletions (indels) in the sequence created by the cellular repair machinery. When an exogenous DNA sequence with homology with the target sequence is added, HR and integration into the desired locus is occurring after the DSB.

One prerequisite for the development *of in vivo* gene repair strategy in the CNS is an efficient delivery system. The design, production, and efficiency of gene transfer vectors, has improved remarkably over time, leading to safer transduction and long-term and robust transgene expression in the brain (11). This has led to the initiation of several phase I/II clinical trials with adeno-associated vectors (AAV) and lentiviral vectors (LV) in patients suffering from Parkinson's, Alzheimer's, Batten, adrenoleukodystrophy or Canavan's disease (12-14). The identification of numerous natural or chimeric AAV serotypes with variable capsid proteins has been exploited to modulate the entry into the host and enhance transduction efficiencies in the CNS (15, 16). Most studies with LV have been performed with VSV-G (vesicular stomatitis virus glycoprotein G) pseudotyped vectors, but the tropism of the vector could be altered with the use of heterologous envelopes. Their relatively large cloning capacity (8-9 kb compared with 4-5kb for AAV) is particularly advantageous for the cloning of complex expression cassettes with large cDNAs.

Current approaches based on *HTT* silencing (RNAi, ASO) or modulation of HTT expression (ZFN/TALEN-repressors) required a continuous expression of the compounds to maintain the therapeutic benefits and partial *HTT* silencing or repression are obtained. In contrast, there is a need to engineer an effective CRISPR/Cas9 system inducing indels or DNA repair, with the goal to provide permanent and irreversible reactions, leading to a complete *HTT* gene disruption or repair and ensuring a long-lasting therapeutic benefit.

### BRIEF DESCRIPTION OF THE INVENTION

One of the objects of the present invention is to provide a kit suitable for the treatment of Huntington's disease (HD) for allele or non-allele-specific huntingtin (*HTT*) gene editing or repair comprising a gene delivery vector consisting of:
- one or more nucleic acid sequences of at least one viral vector selected from the group consisting of adeno-associated vector serotypes (AA from the group consisting of V) and lentiviral vectors (LV);
- at least one nucleic acid sequence that encodes a Cas9 being human codon-optimized or fused to an epitope tag selected among the group of FLAG, His, myc, Tap, HA, V5. Preferably Cas9 is fused to fused to an epitope tag (e.g V5 tag).
- at least one nucleic acid sequence that encodes at least one artificial single guide RNA (sgRNA) having a total size from 63-115 nucleotides comprising a tracrRNA sequence of 48-85 nucleotides long (preferably 82bp) and a crRNA sequence encoded by a nucleic acid sequence selected from the group consisting of SEQ ID NOS: 4-7 and 13-39 and recognizing the sequence of the *HTT* gene within the region defined by position 3066800 to position 3086939 excluding the expanded CAG repeat mutation (ENSG00000197386, position of the first CAG repeat in exon 1: 3'074'877-3'074'879), wherein said crRNA sequence binds directly upstream of the required 5'-NGG/NAG-3' protospacer adjacent motif (PAM) and whereas said crRNA sequence base-pairs with the target *HTT* sequence and Cas9 mediates a double-stranded break (DSB) 3-4 bp upstream of said PAM.

Another object of the present invention is to provide a kit, for use in a method of treatment of Huntington's disease in patients by *in vivo* non-allele-specific *HTT* inactivation of the human *HTT* wild-type (WT) and mutant alleles. For this, a region around the expanded CAG repeat mutation and comprising the region defined by position 3066800 to position 3086939 (excluding the expanded CAG repeat mutation) are targeted for the selection of the sgRNA.

A further object of the invention is to provide a kit, for use in a method of treatment of Huntington's disease in patients by *in vivo* non-allele-specific *HTT* gene repair based on HR with a DNA template containing a WT *HTT* sequence.

Also provided is a kit, for use in a method of treatment of Huntington's disease in patients by *in vivo* method for allele-specific *HTT* inactivation of the human mutant *HTT* gene wherein said at least one sgRNA is capable of recognizing SNP sequences located upstream a PAM along the *HTT* gene with a high frequency of heterozygosity in the human population at least > 5% (according to dbSNP or 1000 Genome Project database).

The present invention also provides a kit for use in a method of treatment of Huntington's disease in patients by *in vivo* mutant *HTT* gene repair based on HR with a DNA template containing a wild-type *HTT* sequence, wherein said at least one sgRNA is capable of recognizing SNP sequences located upstream a PAM along the *HTT* gene with a high frequency of heterozygosity in the human population at least > 5% (according to dbSNP or 1000 Genome Project database).

Other objects and advantages of the invention will become apparent to those skilled in the art from a review of the ensuing detailed description, which proceeds with reference to the following illustrative drawings, and the attendant claims.

Applicants use the CRISPR system to definitely dirsupt or repair the HD mutation. Applicants target sites with the CRISPRin a region comprising the region upstream of the *HTT* gene (position 3066800) up to the 5' end of intron 1 (position 3086939), with an emphasis around the transcription start site and the translation initiation codon ATG with the CRISPR to block the expression of the mutant *HTT* at the DNA level. Both Cas9 and sgRNAs are delivered in CNS structures affected in HD (neurons and/or astrocytes) of rodent expressing the human mutant *HTT* with virals vectors, for example VSV-G pseudotyped LV or AAV2/5 vector, expressing a human codon optimized Cas9 under the PGK, CMV or CBA promoters followed by the woodchuck post-transcriptional regulatory element (WPRE). The second one expresses one copy or multiple sgRNA under the HI promoter or other polymerase II or III promoters and stuffer DNA to ensure independent expression of each sgRNA. Blocking the expression *of mHTT in* adult neurons and /or glial cells helps to rescue the HD phenotype and therefore provides therapeutic benefit.

DNA disruption/repair of Huntington's disease mutation using the CRISPR/Cas9 system represents a new and original therapeutic approach. The present invention offers the possibility to act at the DNA level with engineered nucleases to inactivate or repair a disease-causing mutation. Previous works using RNA silencing obtained a significant decrease of *mHTT* expression, which lead to an improved phenotype. Recently, an approach based on zinc-finger repressors was evaluated in HD mouse model brain and reached a low but significant reduction *of mHTT* expression. However, all these approaches need a continuous expression of the therapeutic gene. In addition, only a partial silencing of the mutant HTT is obtained and this has encouraged the development of complete/irreversible reverse genetic approaches. In the case of the genome editing strategy of the present invention, only a transient expression of the nuclease is required to induce the DSB. HTT gene disruption or repair mediated by this DSB will be permanent.

### BRIEF DESCRIPTION OF THE FIGURES

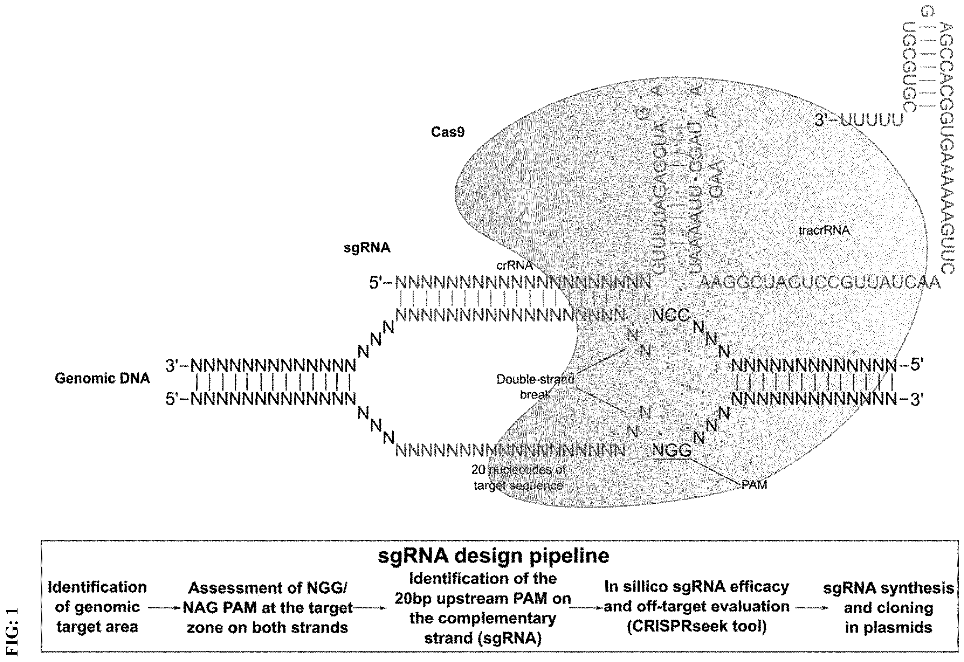

Figure 1: Schematic representation of the CRISPR system showing the Cas9 protein, sgRNA and *HTT* target sequence with the adjacent PAM. The sgRNA is composed of a tracrRNA sequence of 48-85 nucleotides (scaffold sequence) and a crRNA sequence of 15-30 nucleotides base-pairing with the target sequence. The design and specific features of the resulting chimeric sgRNA are shown. Applicants analyze sequences of interest for all possible targetable sequences (preferably 20 nt) immediately 5' to a NGG/NAG PAM motif. Unique genomic sequences were selected to minimize off-target effects (based on dedicated softwares, such as the CRISPRseek Bioconductor package to identify Target-Specific Guide RNAs for CRISPR-Cas9 Genome-Editing Systems). The sgRNA was cloned into a delivery system containing preferably, polymerase III promoters (HI, U6 and 7SK), which are leading to high expression levels.

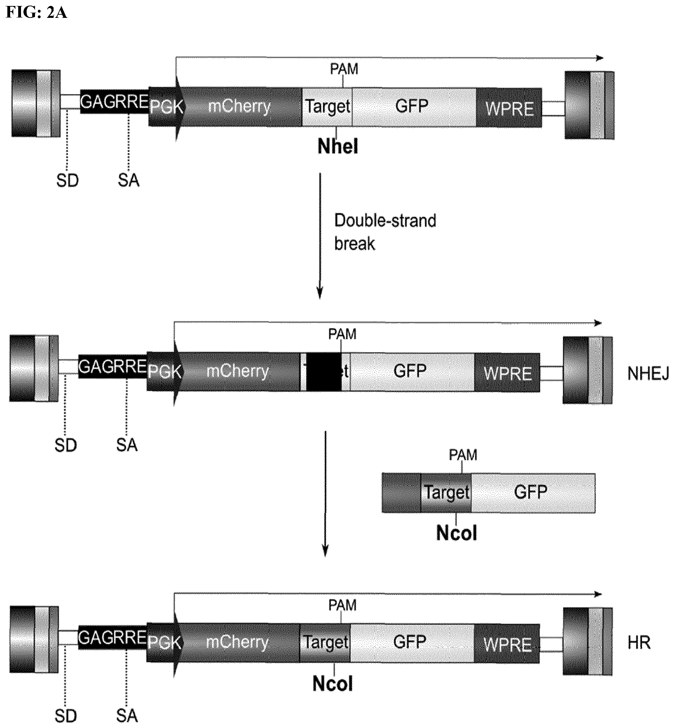

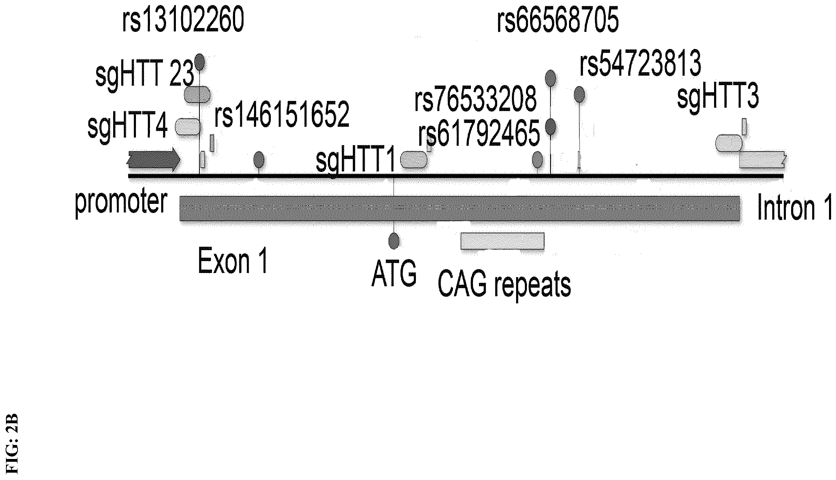

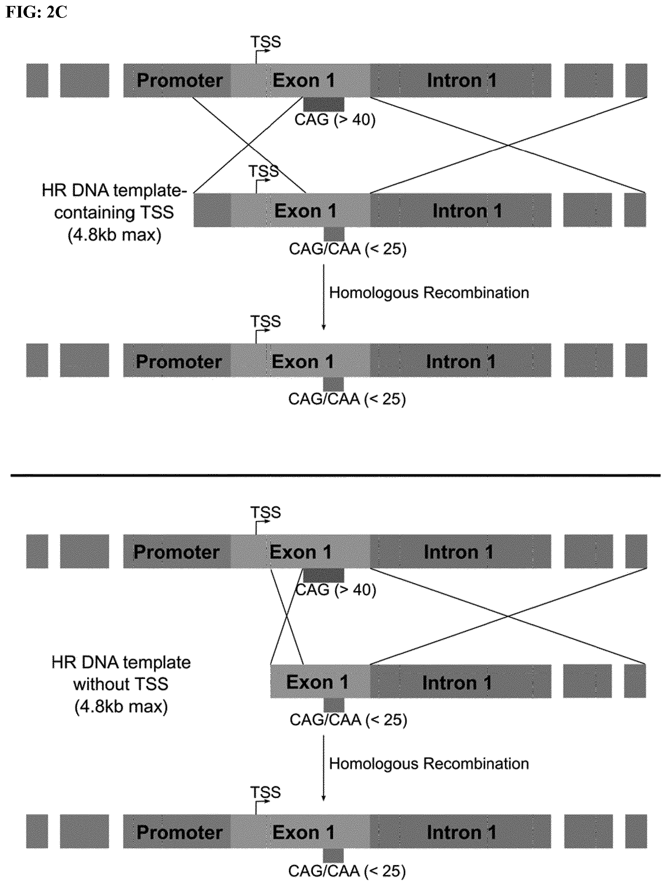

Figure 2: Description of the reporter gene constructs. (A) An artificial target sequence was used and cloned in viral vectors. An artificial target sequence was introduced between the mCherry-GFP sequence expressed under the PGK promoter. This target consists of 20 nucleotides carrying a STOP codon two nucleotides 5' of the NGG PAM. Cells expressing this construct will display only mCherry fluorescence. If DSB occurs after target sequence recognition by the CRISPR system, the STOP codon will be removed and a fusion protein: mCherry-GFP will be expressed. For HR experiments, the presence of the homologous donor DNA without the STOP codon will be integrated at the desire locus. In addition, HR leads to the insertion of an NcoI restriction site at the end of the target sequence. (**B**) Schematic representation of the 3' end of the human *HTT* (*hHTT*) promoter, exon 1 and 5' end of intron 1 showing examples of sgHTT. Circles represent positions of known SNP in the *hHTT gene* with their corresponding name (starting with rs; dbSNP http://www.ncbi.nlm.nih.gov/SNP/). The selected sgHTT with their associated PAM are represented by squares (see SEQ ID NO: 4-7, 13-39). CAG expansion and initiation of the translation (ATG) are also represented. (C) Schematic representation of strategies used for *mHTT* repair by HR. Donor templates with (upper panel) or without TSS (lower panel) are indicated. In both cases, the maximum size of the homologous sequence is 4.8kb to be encapsidated in a promoter-less AAV.

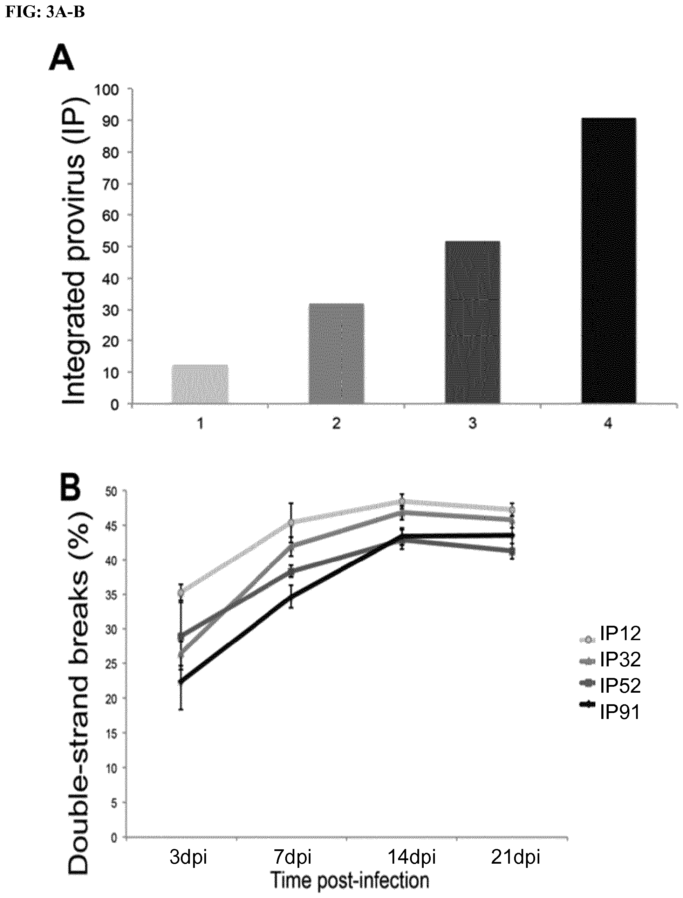

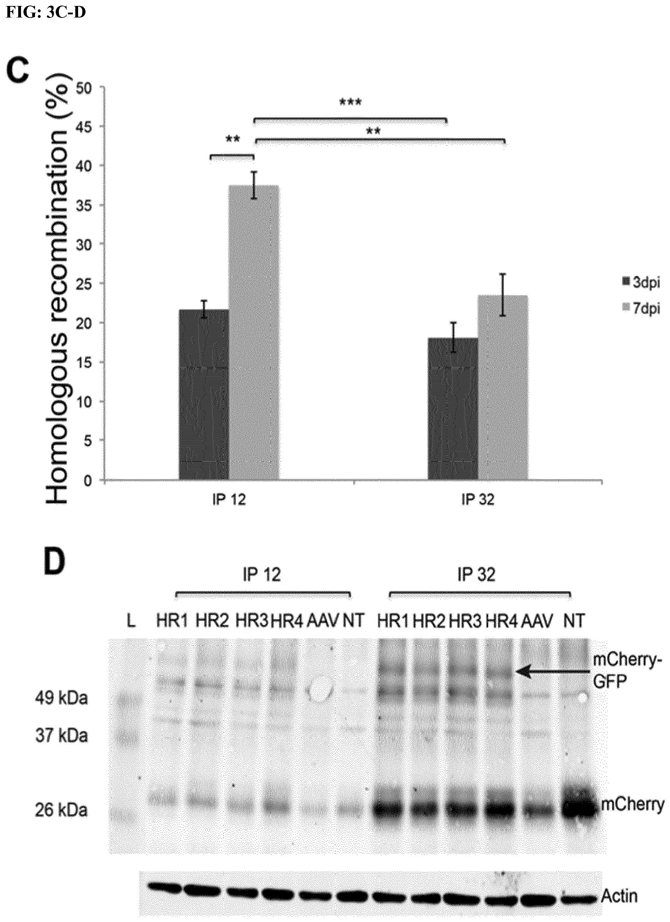

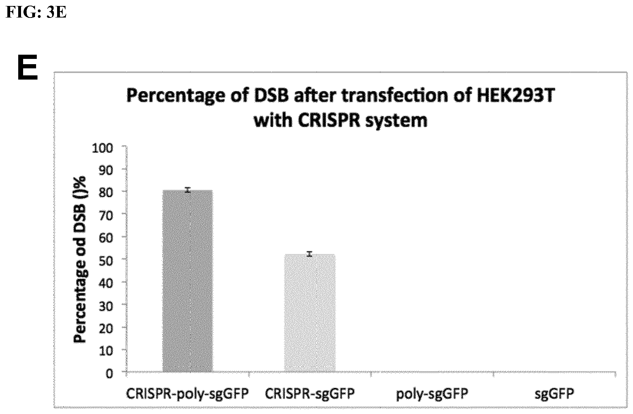

Figure 3: Optimization of Cas9 and sgRNA delivery *in vitro.* (**A**) HEK-293T cells were infected with increasing amounts of LV-TARGET (MOI 1, 10, 50 and 100) to produce HEK-TARGET cells. FACS analysis of mCherry fluorescence was used to select the corresponding cell populations. The number of integrated provirus was determined by qPCR on genomic DNA. The four cell populations contain on average 12, 32, 52 and 91 integrated target sequences, respectively. (**B**) Equal amounts of LV-hCas9 and LV-sgTARGET were incubated on 300'000 cells of the 4 populations. Genomic DNA was extracted 3, 7, 14 and 21 days post-infection to perform surveyor mutation detection assay. Results show that the amount of DSB increases with time in all the populations (Time effect, factorial ANOVA, p < 0.01). Cells containing low numbers of target sequences reach a maximal DSB earlier than cells containing higher copies of target sequence, (MOI effect, factorial ANOVA, p < 0.01). Results are presented as mean ± SEM. (**C**) HR was evaluated in HEK-TARGET cells (MOI 12 and 32) by transient transfection of plasmids encoding hCas9, the sgTARGET and the homologous donor DNA. Genomic DNA was extracted 3 and 7 days post-transfection to perform surveyor mutation detection assay and NcoI RFLP to evaluate the percent of HR Surveyor analysis reveal up to 45% of DSB at 7 days post-transfection (data not shown). NcoI digestion displays an efficient HR at the expected locus 3 days post-transfection. Efficiency of HR increased with time to reach up to 37.5% at 7 days post-transfection. Results are presented as mean ± SEM. ** p < 0.01, *** p < 0.001. (**D**) Results obtained for HR experiments were confirmed by western blots using anti-mCherry antibody. The presence of a band around 54 kDa corresponding to the molecular weight of mCherry-GFP fusion protein is observed in treated samples. Actin was used as a control for normalization. HR1-4: four replicates transfected with plasmids expressing hCas9, sgTARGET and the donor DNA. AAV: cells transfected with the donor DNA and an empty plasmid. NT: untransfected cells. (**E**) Additive effects of multiple sgRNA targeting a reporter gene. Plasmids encoding GFP, hCas9, H1-sgGFP1 (CRISPR-sgGFP) or H1-sgGFP1-U6-sgGFP2-7SK-sgGFP3 (CRISPR-poly-sgGFP) were co-transfected in HEK-293T cells. Cells co-transfected without the hCas9 plasmid (sgGFP and poly-sgGFP) were used as negative controls. Genomic DNA was extracted 3 days post-transfection, PCR-amplified and used for Surveyor analysis. Quantifications show an increase in the rate of NHEJ formation at the target locus with the combination of the three sgGFP targeting the same region (respectively 81% and 52% with only the sgGFP1).

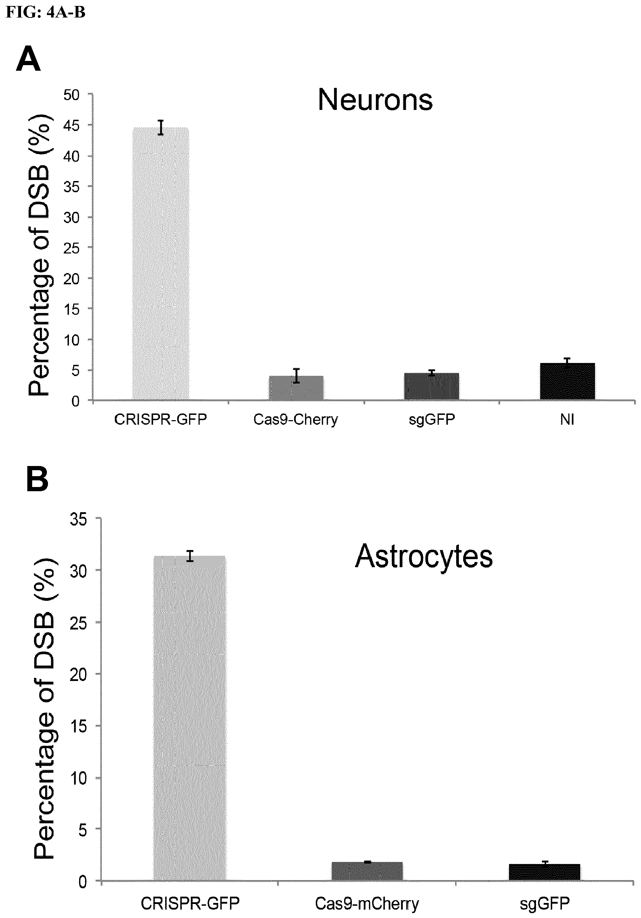

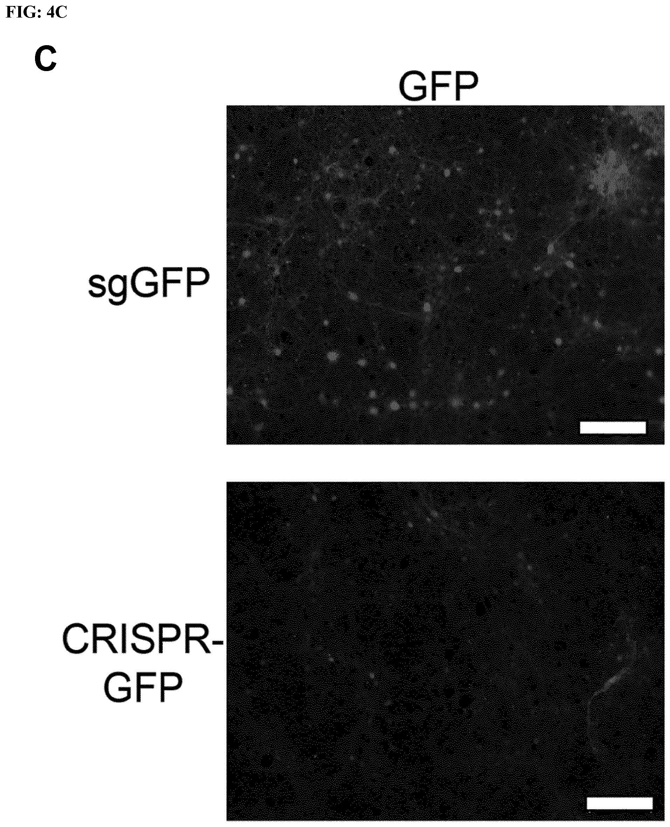

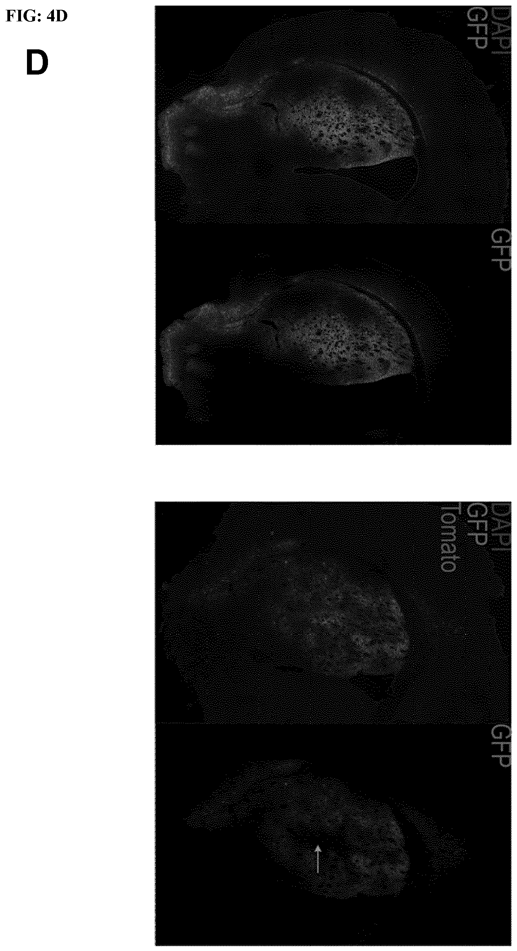

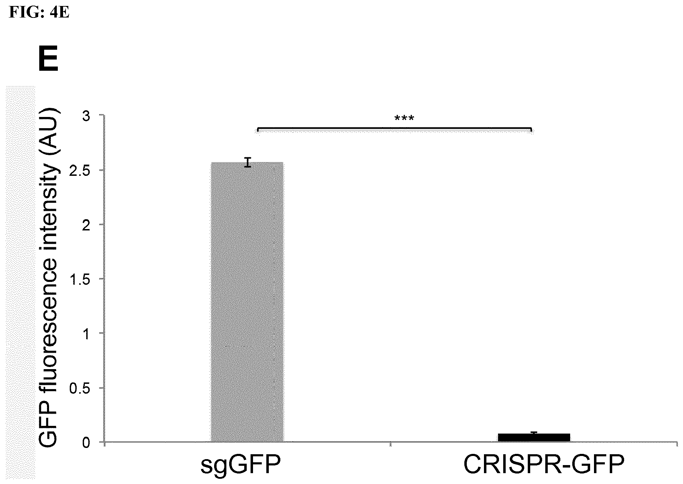

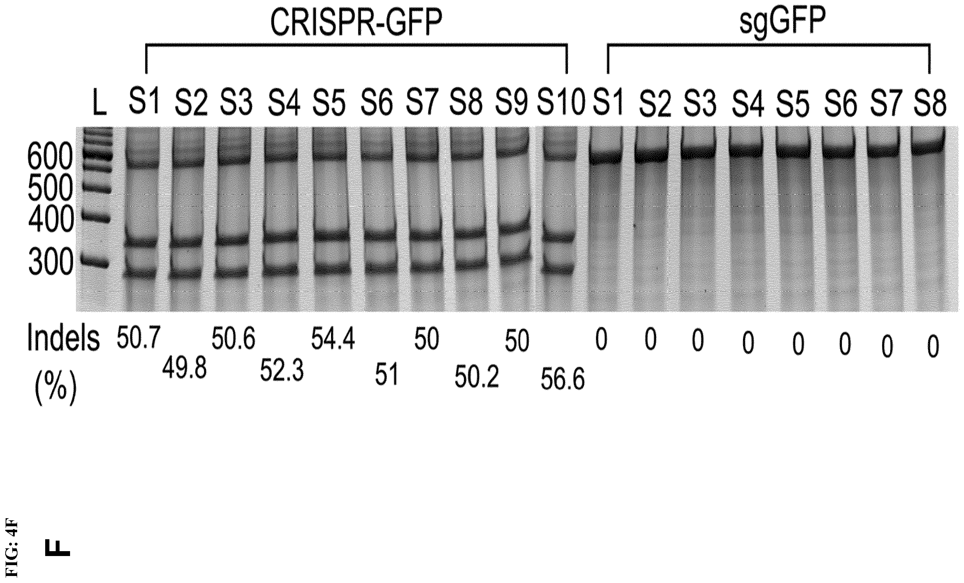

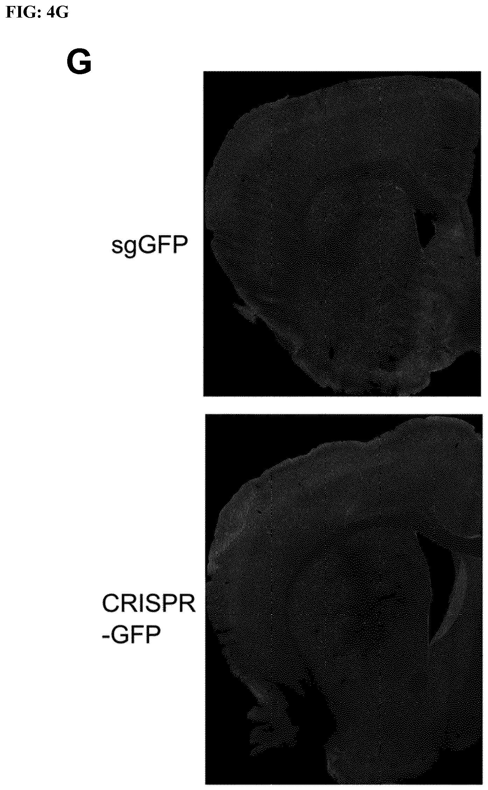

Figure 4: Reporter gene editing in neurons and astrocytes *in vitro* and *in vivo.* (**A**) Mouse E17 cortical primary neurons were infected by an LV expressing GFP at day in vitro 1 (DIV1). A second infection was performed at DIV4 with LV expressing hCas9 and LV expressing Tomato-sgGFPl (CRISPR-GFP), LV-hCas9 and LV expressing mCherry reporter protein (Cas9-Cherry) or LV-Tomato-sgGFP1 alone (sgGFP). Genomic DNA was extracted 3 weeks post-infection with the CRISPR system (DIV25) and subjected to Surveyor assay. Results show around 45% of NHEJ formation at the target site in the CRISPR-GFP group whereas less than 5% of NHEJ was observed in control samples. NI: non-infected neurons. (**B**) Mouse PI cortical primary astrocytes were infected by an LV expressing GFP at DIV7. A second infection was performed at DIV10 with LV expressing hCas9 and LV expressing Tomato-sgGFP1 (CRISPR-GFP), LV-hCas9 and LV expressing mCherry reporter protein (Cas9-Cherry) or LV-Tomato-sgGFP1 alone (sgGFP). Genomic DNA was extracted 3 weeks post-infection with the CRISPR system (DIV25) and subjected to Surveyor assay. Results show around 30% of NHEJ formation at the target site in the CRISPR-GFP group whereas less than 5% of NHEJ was observed in control samples. (C) Mouse E17 cortical primary neurons were infected by an LV expressing GFP at DIV1. A second infection was performed at DIV4 with LV expressing hCas9 and LV expressing Tomato-sgGFPl (CRISPR-GFP) or LV-Tomato-sgGFP1 alone (sgGFP). Neurons were fixed with 4% PFA 3 weeks post-infection with the CRISPR system (DIV25). Microscope acquisitions show numerous GFP-positive neurons in the sgGFP group whereas few GFP-positive cells were observed in the CRISPR-GFP group, showing that GFP disruption leads to the loss of GFP fluorescence. (**D**) Episomal DNA editing was evaluated in the mouse brain with AAV vectors. AAV expressing GFP and LV expressing hCas9 and Tomato-sgGFPl (CRISPR-GFP, right panel) or AAV-GFP alone (left panel) were co-injected in the mouse striatum. Brains were fixed 3 weeks post-infection. Direct fluorescence reveals a strong GFP loss at the Tomato-positive area in the CRISPR-GFP group. (**E**) Genomic DNA editing was evaluated *in vivo* with LV. LV-GFP, LV-hCas9 and LV-Tomato-sgGFP1 (CRISPR-GFP) or LV-GFP and LV-Tomato-sgGFP1 (sgGFP) were co-injected in the mouse striatum. Brains were fixed with 4% PFA 3 weeks post-infection. Mosaic pictures show a high GFP loss in the Tomato-positive area in the CRISPR-GFP group whereas Tomato-positive-GFP-positive neurons were observed in the sgGFP group. Cell-by-cell GFP fluorescence intensity quantification reveals around 95% of GFP loss in the CRISPR-GFP samples compared to the controls. (**F**) Genomic DNA of the Tomato-positive area was extracted from mouse injected with the same experimental design as in Figure 5E. The target sequence was PCR-amplified and subjected to Surveyor assay. Quantifications show around 50% of NHEJ formation at the target sequence in CRISPR-GFP group whereas no GFP disruption was observed in sgGFP samples. (**G**) LV expressing hCas9 under the gfaABC1D(B)₃ promoter (astrocytic) and LV-Tomato-sgGFP1 (CRISPR-GFP) were injected into the striatum of BAC GLT1-eGFP mice expressing eGFP in astrocytes. Mice injected only with the LV-Tomato-sgGFP1 were used as negative controls (sgGFP). Brains were processed 3 weeks post-infection. Mosaic acquisitions reveal a strong loss of eGFP in astrocytes on the CRISPR-GFP group compared to the sgGFP samples.

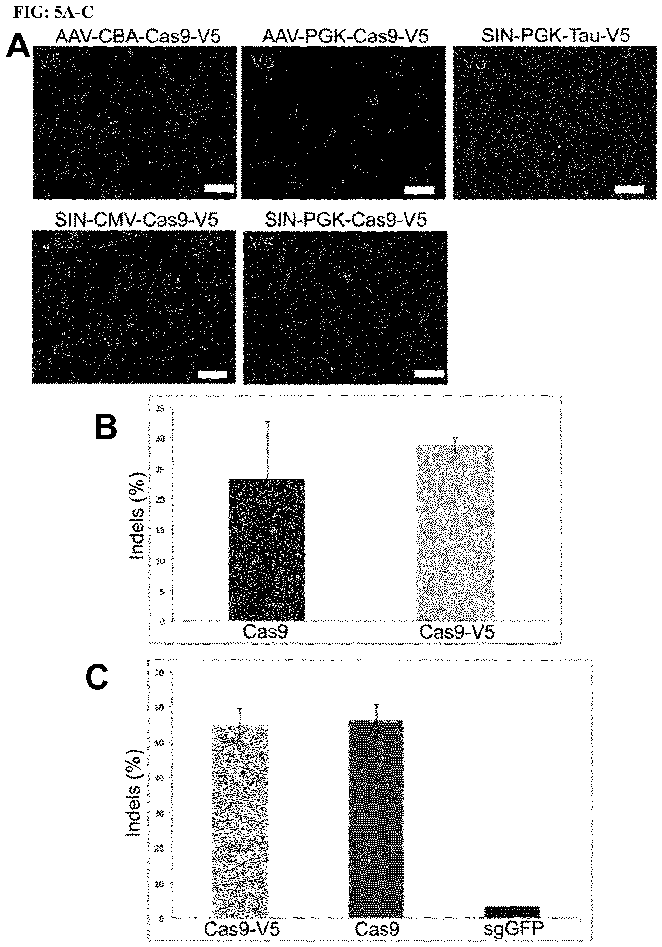

Figure 5: Impact of V5 tag on hCas9 expression and functionality. (**A**) HEK-293T cells were co-transfected with a plasmid expressing nuclear GFP (GFPnuc) and an AAV plasmid expressing hCas9-V5 under the CBA promoter (AAV-CBA-Cas9-V5) or an AAV plasmid expressing hCas9-V5 under the PGK promoter (AAV-PGK-Cas9-V5) or an LV plasmid expressing hCas9-V5 under the CMV promoter (SIN-CMV-Cas9-V5) or an LV plasmid expressing hCas9-V5 under the PGK promoter (SIN-PGK-Cas9-V5). As positive control for the V5 immunostaining, we used a plasmid expressing a Tau-V5 protein (SIN-PGK-Tau-V5). Cells were fixed 3 days post-transfection and an immunostaining against V5-tag was performed. Pictures reveal that all plasmids encoding hCas9 are expressed. In addition, the majority of the staining was observed in the cytoplasm with few staining in the nucleus, meaning that the addition of the V5-tag reduced the incorporation of hCas9 into the nucleus. (**B**) Efficiency of hCas9-V5 was determined in HEK-293T cells. Cells were co-transfected with plasmids coding for sgTARGET, TARGET sequence and hCas9 (Cas9 group) or hCas9-V5 (hCas9-V5) group. Genomic DNA was extracted 3 days post-transfection and indels formation was analyzed with the Surveyor assay. Results show that both hCas9 and hCas9-V5 efficiently disrupt target sequence. (**C**) Efficiency of hCas9-V5 was evaluated by infection in mouse E17 cortical primary neurons. Neurons were infected by an LV expressing GFP at DIV1. A second infection was performed at DIV4 with LV expressing hCas9 (Cas9 group) or LV-hCas9-V5 (Cas9-V5 group) and LV expressing Tomato-sgGFPl. Neurons infected only with LV-sgGFP1 (sgGFP) were used as negative controls. Genomic DNA was extracted 4 weeks post-infection with the CRISPR system (DIV31) and subjected to Surveyor assay. Results show equal efficiency for both hCas9 and hCas9-V5 in primary neurons.

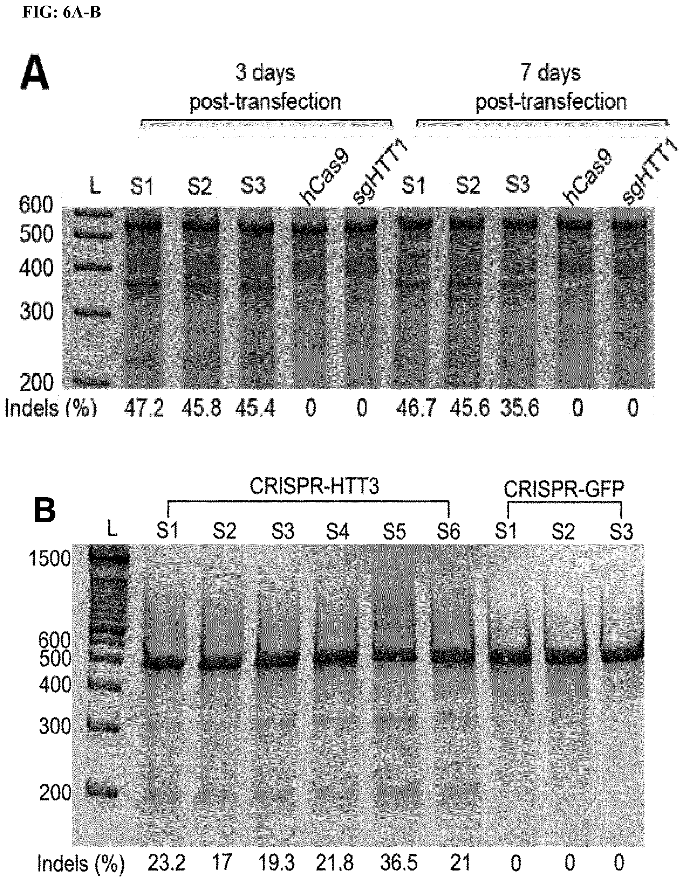

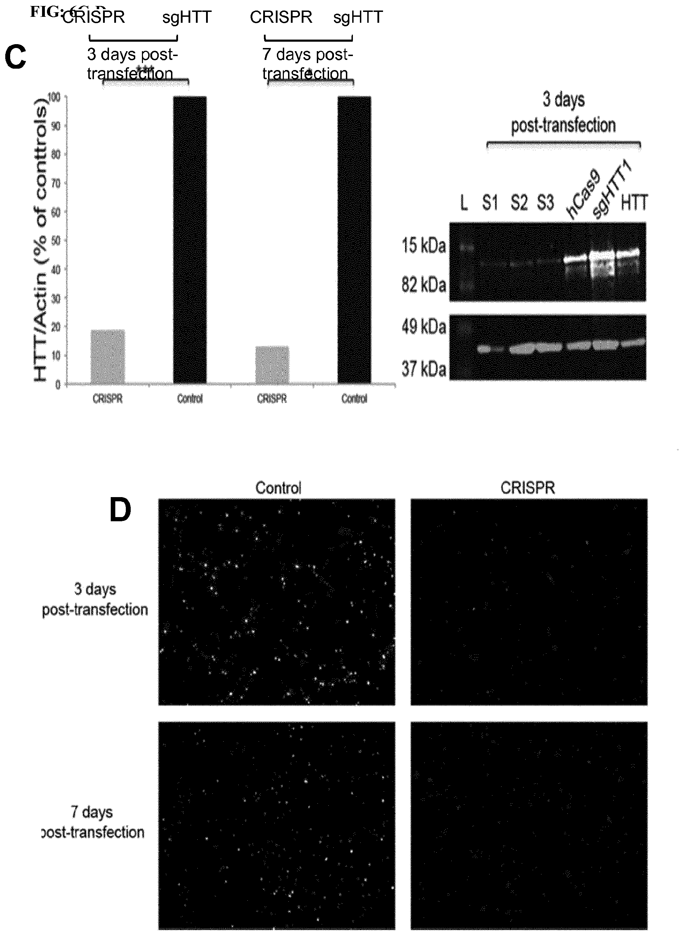

Figure 6: Editing of WT *HTT* gene in HEK-293T cells. (**A-B**) Plasmids encoding hCas9 and sgHTT1 (downstream of the ATG, conserved in mouse and human, panel **A**) or sgHTT3 (panel **B**) were transfected in HEK-293T cells for a period of 3 and 7 days (only for sgHTT1). Genomic DNA was extracted for surveyor mutation detection analysis. Transfections with sgHTT1 or hCas9 only were used as negative controls for sgHTT1 experiment whereas co-transfection of plasmids encoding hCas9 and sgGFP 1 (CRISPR-GFP) was used as control for sgHTT3 experiment. Results show a mean of 45% of DSB formation at the *HTT* locus at both time points for sgHTT1, whereas a lower efficiency (mean of 21%) was observed at the sgHTT3 target site. (C) Plasmids encoding hCas9, sgHTT1 and the first 171 amino-acids of the human HTT with 82 CAG fused to the GFP (Htt171-82Q-GFP) were transfected in HEK-293T cells for 3 and 7 days. Total proteins were extracted and the amount of mutant HTT was evaluated by western blot with an anti-HTT antibody. Results shown a strong loss of mutant HTT in samples treated with hCas9 and the sgHTT1 when compared with control samples. Actin was used as control protein for normalization. Results are presented as mean ± SEM. * p < 0.05, *** p < 0.001. (**D**) Analysis 3 and 7 days post-transfection indicates that control cells (Control panels) transfected with hCas9, Htt171-82Q-GFP and an sgRNA (no homology for either the target plasmid) have a high number of GFP-positive aggregates whereas HEK-293T cells transfected with hCas9, sgHTT1 and Htt171-82Q-GFP (CRISPR panels) have a reduced number GFP-positive aggregates of less intense GFP-signal. Scale bar = 200µm.

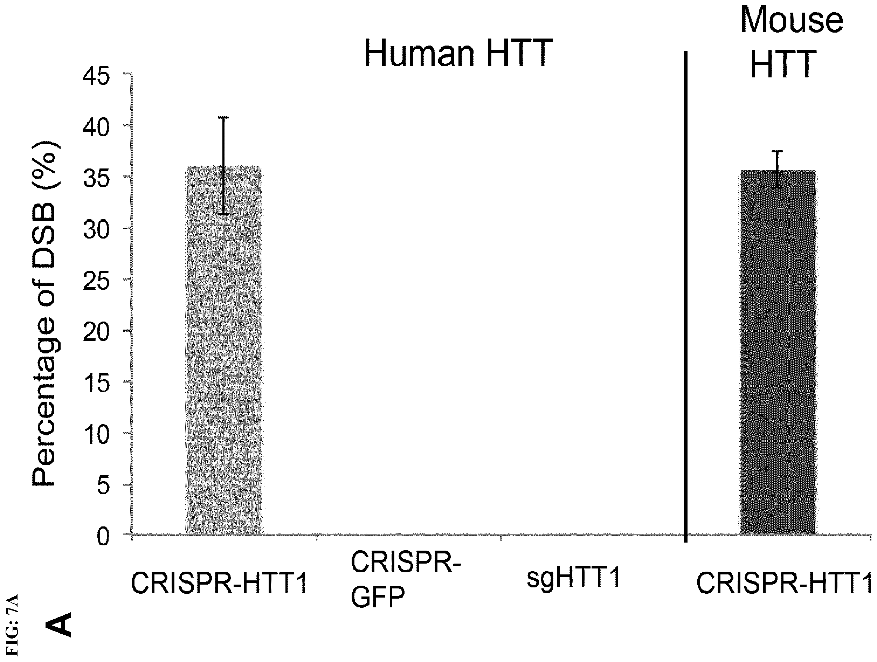

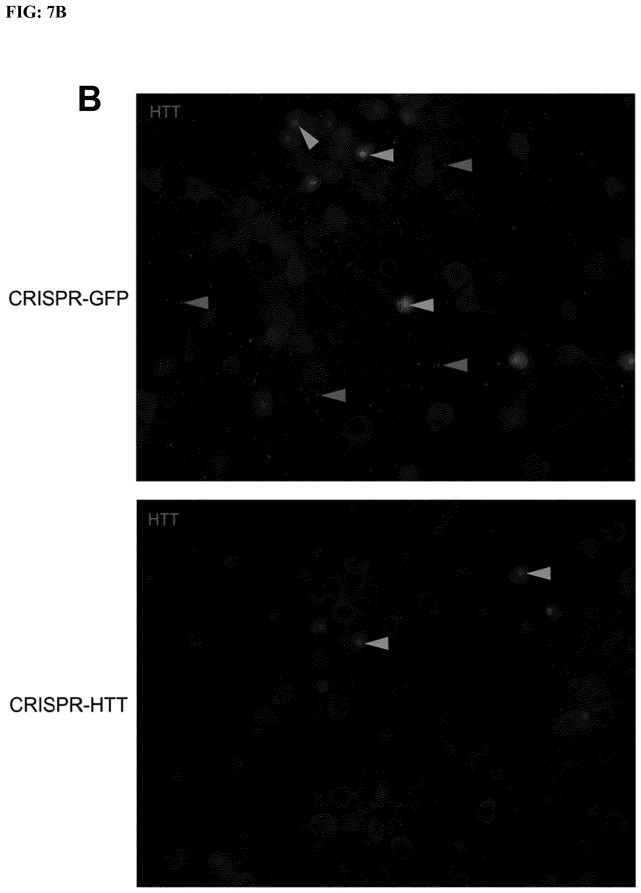

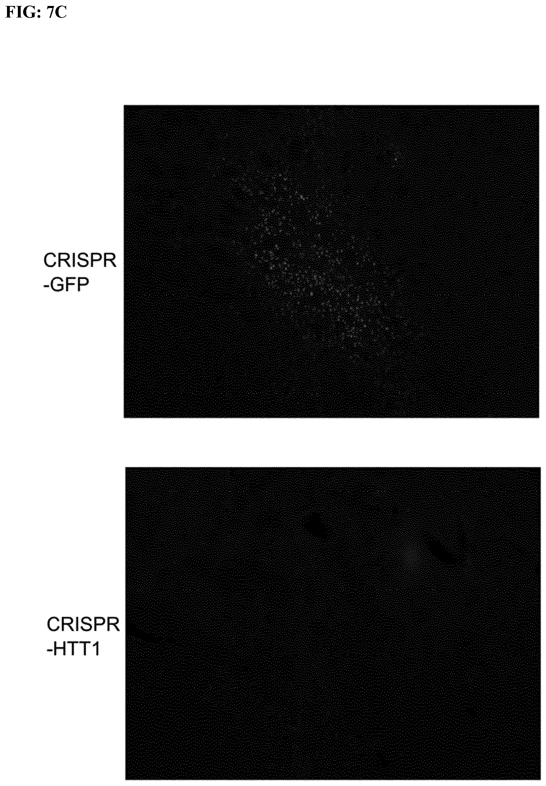

Figure 7: Mutant *HTT* disruption in primary neurons and in the striatum. (A) Mouse E17 cortical primary neurons were infected by LV-hCas9 and LV-sgHTT1 (CRISPR-HTT1), LV-hCas9 and LV-sgGFP1 (CRISPR-GFP) or LV-sgHTT1 (sgHTT1) at DIV1. A second infection was performed at DIV4 with LV expressing the first 171 amino acid of the human HTT with 82 CAG (LV-171HTT-82Q). Genomic DNA was extracted 3 weeks post-infection with the CRISPR system (DIV25). Primers specific for the human *HTT* were used to amplify the *mHTT.* Surveyor analysis shows around 35% of human *HTT* disruption in the CRISPR-HTT1 group (left part of the graph). Because the sgHTT1 has full homology for the mouse *HTT* except for the first nucleotide (5' end of the crRNA), mouse-specific primers were used to amplify the endogenous *HTT* for surveyor assay. Results show that the sgHTT1 is able to induce mouse *HTT* disruption at the same efficiency than human *HTT.* (**B**) Mouse E17 cortical primary neurons were infected by LV-hCas9 and LV-sgHTT1 (CRISPR-HTT1) or LV-hCas9 and LV-sgGFP1 (CRISPR-GFP) at DIV1. A second infection was performed at DIV4 with LV expressing the first 171 amino acid of the human HTT with 82 CAG (LV-171HTT-82Q). Neurons were fixed at DIV25 with 4% PFA and an immunostaining against the mutant HTT was performed. Microscope acquisitions in the CRISPR-GFP group reveal numerous large mHTT aggregates (light gray arrow heads), which are mainly localized in the nucleus and cytoplasm of neurons, and small mHTT aggregates (dark gray arrow heads), which are mainly localized in neuritis. A strong loss of large and small mHTT aggregates is observed in the CRISPR-HTT group as a result of *mHTT* disruption. (**C**) LV-171HTT-82Q, LV-hCas9 and LV-sgHTT1-mCherry (CRISPR-HTT) were co-injected into the striatum of C57Bl/6 mice. As negative controls, LV-171HTT-82Q, LV-hCas9 and LV-Tomato-sgGFP1 (CRISPR-GFP) were used. Brains were processed 4 weeks post-injection and stained with an anti-mHTT antibody. Pictures reveal a strong loss of mHTT-positive aggregates in the CRISPR-HTT group compared with the CRISPR-GFP group.

### BRIEF DESCRIPTION OF THE SEQUENCES

SEQ ID NO: 1 corresponds to the 82 nucleotides of the artificial chimeric tracrRNA containing the poly-T signal: 5'-GTTTTAGAGCTAGAAATAGCAAGTTAAAATAAGGCTAGTCCGTTATCAACTTGAA AAAGTGGCACCGAGTCGGTGCTTTTTT -3'
SEQ ID NO: 2 corresponds to the 20 nucleotides of the artificial sgTARGET: 5'- GGCTGCTGTCAGGGCTAGCA -3'
SEQ ID NO: 3 corresponds to the 20 nucleotides of the artificial sgGFP: 5'-GAAGTTCGAGGGCGACACCC-3'
SEQ ID NO: 4 corresponds to the 20 nucleotides of the sgHTT1 in exon 1, which is conserved between human, *Macaca Fascicularis* and *Mus Musculus HTT* gene with one mismatch at position 1 (G/A): 5'- GACCCTGGAAAAGCTGATGA -3'
SEQ ID NO: 5 corresponds to the 20 nucleotides of the sgHTT2, which is fully conserved between humans, *Macaca Fascicularis, Rattus Norvegicus* and *Mus Musculus HTT* gene: 5'- GTGGATGACATAATGCTTTT -3'
SEQ ID NO: 6 corresponds to the 20 nucleotides of the sgHTT3 between exon 1 and between intron 1, which is conserved between humans, *Macaca Fascicularis, Rattus Norvegicus* and *Mus Musculus HTT* gene with one mismatch at position 4 (G/A): 5'- GGAGCCGCTGCACCGACCGT -3'
SEQ ID NO: 7 corresponds to the 20 nucleotides of the sgHTT4, which is human specific with SNP rs13102260 at position 20 (G/A underline) for allele-specific approaches: 5'-TGGGACGCAAGGCGCCGTGG -3'
SEQ ID NO: 8 corresponds to the human codon-optimized Cas9 fused to one nuclear localization signal (NLS coding for: PKKKRKV) at the C-terminus (sequence: https://www.addgene.org/41815, (17)).
SEQ ID NO: 9 corresponds the artificial HR sequence used in the example 2 containing the end of the mCherry, the corrected target site and the GFP sequence.
SEQ ID NO: 10 corresponds to the 20 nucleotides of the artificial sgGFP2: 5'-GCTGAAGGGCATCGACTTCA -3'
SEQ ID NO: 11 corresponds to the 20 nucleotides of the artificial sgGFP3: 5'-CAACTACAAGACCCGCGCCG -3'
SEQ ID NO: 12 corresponds to the lentiviral vector SIN-cPPT-H1-sgGFP1-U6-sgGFP2-7SK-sgGFP3-PGK-mCherry-WPRE for the expression of multiple sgRNA under the control polymerase III promoters (HI, U6, 7SK) and containing a mCherry reporter gene to visualize infected cells.
SEQ ID NO: 13 corresponds to the 20 nucleotides of the artificial sgHTT24, which is human-specific and containing the human SNP rs9993542 at position 19 (C/T) for allele-specific approaches:
   5'- GGTAATTTGTGTTTGTGTGC -3'
   The sgHTT24 was designed on the complementary strand of the HTT gene, as a consequence the polymorphism of the human SNP rs9993542 at position 19 corresponds to the complementary sequence (G/A underline).
SEQ ID NO: 14 corresponds to the 20 nucleotides of the artificial sgHTT25, which is human specific with SNP rs762855 at position 18 (A/G) for allele-specific approaches 5'-GACAGCAGAGAAACAGCTGT- 3'
   The sgHTT25 was designed on the complementary strand of the HTT gene, as a consequence the polymorphism of the human SNP rs762855 at position 18 corresponds to the complementary sequence (T/C underline).
SEQ ID NO: 15 corresponds to the 20 nucleotides of the artificial sgHTT5, which is conserved between human and *Macaca Fascicularis* with one mismatch at the 11^{th} position from 5' (A/G) and containing the human SNP rs762855 at position 11 (A/G) for allele-specific approaches
   5'- GAGAAACAGCTGTTAGTTCC- 3'
   The sgHTT5 was designed on the complementary strand of the HTT gene, as a consequence the polymorphism of the human SNP rs762855 at position 11 corresponds to the complementary sequence (T/C underline).
SEQ ID NO: 16 corresponds to the 20 nucleotides of the artificial sgHTT26, which is human specific with SNP rs9996199 at position 19 (C/G) for allele-specific approaches 5'- TGGCTAAAGTAGGCTTTACT -3'
SEQ ID NO: 17 corresponds to the 20 nucleotides of the artificial sgHTT6, which is human specific with SNP rs28431418 at position 19 (T/C underline) for allele-specific approaches 5'-CAGGGCTGTCCGGGTGAGTA -3'
SEQ ID NO: 18 corresponds to the 20 nucleotides of the artificial sgHTT7, which is human specific with SNP rs28431418 at position 13 (T/C underline) for allele-specific approaches 5'-TGTCCGGGTGAGTATGGCTC-3'
SEQ ID NO: 19 corresponds to the 20 nucleotides of the artificial sgHTT27, which is human specific with SNP rs2857935 at position 20 (G/C/T) for allele-specific approaches 5'-AGGCCCATGCGGAAAGGATC-3'
   The sgHTT27 was designed on the complementary strand of the HTT gene, as a consequence the polymorphism of the human SNP rs2857935 at position 20 corresponds to the complementary sequence (C/G/A underline).
SEQ ID NO: 20 corresponds to the 20 nucleotides of the artificial sgHTT8, which is human specific with SNP rs2857935 at position 8 (G/C/T underline) for allele-specific approaches 5'-GGCGGGGGATCCTTTCCGCA-3'
SEQ ID NO: 21 corresponds to the 20 nucleotides of the artificial sgHTT10, which is human specific with SNP rs13122415 at position 17 (C/G) for allele-specific approaches 5'-GGGGCTCAACGGAGAGGGGA -5'
   The sgHTT10 was designed on the complementary strand of the HTT gene, as a consequence the polymorphism of the human SNP rs13122415 at position 17 corresponds to the complementary sequence (G/C underline)
SEQ ID NO: 22 corresponds to the 20 nucleotides of the artificial sgHTT28, which is human specific with SNP rs13132932 at position 19 (A/G underline) for allele-specific approaches 5'-GGCAGAACCTGCGGGGGCAG-3'
SEQ ID NO: 23 corresponds to the 20 nucleotides of the artificial sgHTT9, which is human specific with SNP rs13132932 at position 15 (A/G underline) for allele-specific approaches 5'-GAACCTGCGGGGGCAGGGGC-3'
SEQ ID NO: 24 corresponds to the 20 nucleotides of the artificial sgHTT14, which is human specific with SNP rs34045730 at position 17 (A/T underline) for allele-specific approaches 5'- ATTTTACCAGTATTCCAGTC -3'
SEQ ID NO: 25 corresponds to the 20 nucleotides of the artificial sgHTT15, which is human specific with SNP rs28656215 at position 20 (C/T underline) for allele-specific approaches 5'- CTGTGTGTCGAGTGTACAGT -3'
SEQ ID NO: 26 corresponds to the 20 nucleotides of the artificial sgHTT16, which is human specific with SNP rs3856973 at position 20 (G/A) for allele-specific approaches 5'- ATAAAAATAAGTTAACACTC -3'
   The sgHTT16 was designed on the complementary strand of the HTT gene, as a consequence the polymorphism of the human SNP rs3856973 at position 20 corresponds to the complementary sequence (C/T underline).
SEQ ID NO: 27 corresponds to the 20 nucleotides of the artificial sgHTT17, which is human specific with SNP rs57666989 at position 14 (C/T) for allele-specific approaches 5'-GCAGGAGAATGGCGTGAACT -3'
   The sgHTT17 was designed on the complementary strand of the HTT gene, as a consequence the polymorphism of the human SNP rs57666989 at position 14 corresponds to the complementary sequence (G/A underline).
SEQ ID NO: 28 corresponds to the 20 nucleotides of the artificial sgHTT19, which is fully conserved between human and *Macaca Fascicularis* with human SNP rs61792477 at position 12 (C/T underline) for allele-specific approaches 5'- ATAATATAGTACGGCCACTC -3'
SEQ ID NO: 29 corresponds to the 20 nucleotides of the artificial sgHTT20, which is human specific with SNP rs28867436 at position 19 (A/G underline) for allele-specific approaches 5'- CATAAAACTGAACATACCGT -3'
SEQ ID NO: 30 corresponds to the 20 nucleotides of the artificial sgHTT21, which is human specific with SNP rs2285087 at position 15 (A/G underline) for allele-specific approaches 5'- AGCAATTGGGCAACATCGTG -3'
SEQ ID NO: 31 corresponds to the 20 nucleotides of the artificial sgHTT30, which is human specific with SNP rs2285087 at position 17 (A/G) for allele-specific approaches 5'- AGACGGGGCCTCACGATGTT-3'
   The sgHTT30 was designed on the complementary strand of the HTT gene, as a consequence the polymorphism of the human SNP rs2285087 at position 17corresponds to the complementary sequence (T/C underline).
SEQ ID NO: 32 corresponds to the 20 nucleotides of the artificial sgHTT22, which is human specific with SNP rs2285086 at position 20 (A/G) for allele-specific approaches 5'- AGAAGGTGCTGCTAGTTCAT -3'
   The sgHTT22 was designed on the complementary strand of the HTT gene, as a consequence the polymorphism of the human SNP rs2285086 at position 20 corresponds to the complementary sequence (T/C underline).
SEQ ID NO: 33 corresponds to the 20 nucleotides of the artificial sgHTT18, which is human specific with SNP rs61792473 at position 12 (C/T) and rs61792474 at position 14 (C/T) for allele-specific approaches 5'- TGTGTGTGTGTGTGTATATT-3'
   The sgHTT18 was designed on the complementary strand of the HTT gene, as a consequence the polymorphism of the human SNP SNP rs61792473 at position 12 and rs61792474 at position 14 corresponds to the complementary sequence (G/A underline).
SEQ ID NO: 34 corresponds to the 20 nucleotides of the artificial sgHTT13, which is human specific with SNP rs58870770 at position 19 (-/A) (- = deletion of one nucleotide) for allele-specific approaches 5'-GTTGTGGTTGCCAAGTAAAG-3'
SEQ ID NO: 35 corresponds to the 20 nucleotides of the artificial sgHTT12, which is human specific with SNP rs150577220 at position 14 (-/A) (- = deletion of one nucleotide) for allele-specific approaches 5'- GGAGTGGCGGGGCAGGGGGG -3'
SED ID NO: 36: corresponds to the 20 nucleotides of the human-specific artificial sgHTT31: 5'-CGGGACGGGTCCAAGATGGA -3'
SEQ ID NO 37: corresponds to the 20 nucleotides of the human-specific artificial sgHTT35, which is fully conserved human and *Macaca Fascicularis* 5'- GGGCTGTCAATCATGCTGGC -3'
SED ID NO: 38: corresponds to the 20 nucleotides of the artificial sgHTT33, which is fully conserved between human and *Macaca Fascicularis:* 5'-CTTTTCCAGGGTCGCCATGG -3'
SED ID NO: 39: corresponds to the 20 nucleotides of the artificial sgHTT34, which is fully conserved between human and *Macaca Fascicularis:* 5'- GACAATATGTGAAAACATAG -3'
SEQ ID NO: 40 corresponds to the 20 nucleotides of the artificial sgCas9: 5'- AATGGAGTACTTCTTGTCCA -3'
SEQ ID NO: 41 Cas9-V5 corresponds to the human codon-optimized Cas9 fused to one nuclear localization signal and V5 epitope at the C-terminus of Cas9 to visualize the protein.
SEQ ID NO: 42 human HTT with the TSS corresponds to one exogenous DNA template used for HR
SEQ ID NO: 43 human HTT HR without the TSS corresponds to one exogenous DNA template used for HR

### DETAILED DESCRIPTION OF THE INVENTION

Although methods and materials similar or equivalent to those described herein can be used in the practice or testing of the present invention, suitable methods and materials are described below. The publications and applications discussed herein are provided solely for their disclosure prior to the filing date of the present application. In addition, the materials, methods, and examples are illustrated below.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise" is generally used in the sense of include, that is to say permitting the presence of one or more features or components.

As used in the specification and claims, the singular forms "a", "an" and "the" include plural references unless the context clearly dictates otherwise.

As used herein the terms "subject" or "patient" are well-recognized in the art, and, are used interchangeably herein to refer to a mammal, including dog, cat, rat, mouse, monkey, cow, horse, goat, sheep, pig, camel, and, most preferably, a human. In some embodiments, the subject is a subject in need of treatment or a subject with a disease or disorder. However, in other embodiments, the subject can be a normal subject. The term does not denote a particular age or sex. Thus, adult and newborn subjects, whether male or female, are intended to be covered.

The term "an effective amount" refers to an amount necessary to obtain a physiological effect. The physiological effect may be achieved by one application dose or by repeated applications. The dosage administered may, of course, vary depending upon known factors, such as the physiological characteristics of the particular composition; the age, health and weight of the subject; the nature and extent of the symptoms; the kind of concurrent treatment; the frequency of treatment; and the effect desired and can be adjusted by a person skilled in the art.
The terms "nucleic acid," "polynucleotide," and "oligonucleotide" are used interchangeably and refer to a deoxyribonucleotide or ribonucleotide polymer, in linear or circular conformation, and in either single- or double-stranded form. For the purposes of the present disclosure, these terms are not to be construed as limiting with respect to the length of a polymer. The terms can encompass known analogues of natural nucleotides, as well as nucleotides that are modified in the base, sugar and/or phosphate moieties (e.g. phosphorothioate backbones). In general, an analogue of a particular nucleotide has the same base-pairing specificity; i.e., an analogue of A will base-pair with T.

The terms "polypeptide," "peptide" and "protein" are used interchangeably to refer to a polymer of amino acid residues. The term also applies to amino acid polymers in which one or more amino acids are chemical analogues or modified derivatives of a corresponding naturally-occurring amino acids.

"Recombination" refers to a process of exchange of genetic information between two polynucleotides. For the purposes of this disclosure, "homologous recombination (HR)" refers to the specialized form of such exchange that takes place, for example, during repair of double-strand breaks in cells via homology-directed repair mechanisms. This process requires nucleotide sequence homology, uses a "donor" molecule to template repair of a "target" molecule (i.e. the one that experienced the double-strand break), and is variously known as "non- crossover gene conversion" or "short tract gene conversion," because it leads to the transfer of genetic information from the donor to the target. Without wishing to be bound by any particular theory, such transfer can involve mismatch correction of heteroduplex DNA that forms between the broken target and the donor, and/or "synthesis-dependent strand annealing," in which the donor is used to resynthesize genetic information that will become part of the target, and/or related processes. Such specialized HR often results in an alteration of the sequence of the target molecule such that part or all of the sequence of the donor polynucleotide is incorporated into the target polynucleotide.

A "reporter gene" or "reporter sequence" refers to any sequence that produces a protein product that is easily measured, preferably although not necessarily in a routine assay. Suitable reporter genes include, but are not limited to, sequences encoding proteins that mediate antibiotic resistance (e.g., ampicillin resistance, neomycin resistance, G418 resistance, puromycin resistance), sequences encoding colored or fluorescent or luminescent proteins (e.g., green fluorescent protein, enhanced green fluorescent protein, red fluorescent protein, luciferase), and proteins which mediate enhanced cell growth and/or gene amplification (e.g., dihydrofolate reductase).

The term "Tag" refers to short amino acid sequences constituting an epitope recognized by an antibody. Epitope tags are added at the N- or C-terminus of a protein to facilitate visualization by immunohistochemistry. Epitope tags include, for example, one or more copies of FLAG, His, myc, Tap, HA, V5 or any detectable amino acid sequence. Cas9 has been previously fused to FLAG tags however; Applicants preferably use the V5 tag to localize Cas9 protein in the present experiments. "Expression tags" include sequences that encode reporters that may be operably linked to a desired gene sequence in order to monitor expression of the gene of interest.

"The CRISPR" (Clustered Regularly Interspaced Short Palindromic Repeats)/Cas9 (CRISPR Associated) nuclease system is a recently engineered nuclease system based on a bacterial system that can be used for genome engineering. It is based on part of the adaptive immune response of many bacteria and archea. When a virus or plasmid invades a bacterium, segments of the invader's DNA are converted into CRISPR RNAs (crRNA) by the 'immune' response. This crRNA then associates, through a region of partial complementarity, with another type of RNA called tracrRNA to guide the Cas9 nuclease to a region homologous to the crRNA in the target DNA called a "protospacer." Cas9 cleaves the DNA to generate blunt ends at the DSB at sites specified by a 20-nucleotide guide sequence contained within the crRNA transcript. Cas9 requires both the crRNA and the tracrRNA for site-specific DNA recognition and cleavage. This system has now been engineered such that the crRNA and tracrRNA can be combined into one molecule (the "single guide RNA"), and the crRNA equivalent portion of the single guide RNA can be engineered to guide the Cas9 nuclease to target any desired sequence (9, 18). Thus, the CRISPR/Cas9 system can be engineered to create a DSB at a desired target in a genome, and repair of the DSB can be influenced by the use of repair inhibitors to cause an increase in error prone repair.

As used herein the term "selectivity of an sgRNA" relates to the ability for an sgRNA sequence and Cas9 complex, to recognize a particular allele of a target gene (i.e. mutant vs WT allele of *HTT).*

The term "specificity of an sgRNA" relates to the ability for a given sgRNA sequence and Cas9 complex to recognize a given target i.e., the *HTT* gene.

The term "haplotype" relates to the ordered, linear combination of polymorphisms (e.g., SNPs) in the sequence of each form of a gene (on individual chromosomes) that exists in the population.

The term "small insertions-deletions (indels)" relates to small insertion or deletions in genomic DNA due to cellular DNA repair following DSB by non-homologous end joining (NHEJ).

The term "allele" relates to a particular form of a genetic locus, distinguished from other forms by its specific nucleotide sequence.

A "Single Nucleotide Polymorphism" (SNP) refers to a single base (nucleotide) difference in a specific location in the DNA sequence among individuals in a population. A subset of SNPs gives rise to changes in the encoded amino acid sequence; these are referred to as coding SNPs, or cSNPs.

The term "allele-specific" when used in reference to nucleic acid sequences, such as oligonucleotides, primers, sgRNA, means that a particular position of the nucleic acid sequence is complementary with an allele of a target polynucleotide sequence. Allele-specific sgRNA are capable of discriminating between different alleles of a target polynucleotide.

The term "vector", as used herein, refers to a DNA or RNA molecule such as a plasmid, virus or other vehicle, which contains one or more heterologous or recombinant DNA sequences and is designed for transfer between different host cells. The terms "expression vector" and "gene therapy vector" refer to any vector that is effective to incorporate and express heterologous DNA fragments in a cell. A cloning or expression vector may comprise additional elements, for example, post-transcriptional regulatory elements. Any suitable vector can be employed that is effective for introduction of nucleic acids into cells, e.g. a viral vector. The terms "heterologous DNA" refer to a "heterologous coding or non-coding sequence" or a "transgene".

The term "homology" between two sequences is determined by sequence identity. If two sequences, which are to be compared with each other differ in length, sequence identity preferably relates to the percentage of the nucleotide residues of the shorter sequence which are identical with the nucleotide residues of the longer sequence. Sequence identity can be determined conventionally with the use of computer programs such as the Bestfit program (Wisconsin Sequence Analysis Package, Version 8 for Unix, Genetics Computer Group, University Research Park, 575 Science Drive Madison, WI 53711). Bestfit utilizes the local homology algorithm of Smith and Waterman, Advances in Applied Mathematics 2 (1981), 482-489, in order to find the segment having the highest sequence identity between two sequences. When using Bestfit or another sequence alignment program to determine whether a particular sequence has for instance 95% identity with a reference sequence of the present invention, the parameters are preferably so adjusted that the percentage of identity is calculated over the entire length of the reference sequence and that homology gaps of up to 5% of the total number of the nucleotides in the reference sequence are permitted. When using Bestfit, the so-called optional parameters are preferably left at their preset ("default") values. The deviations appearing in the comparison between a given sequence and the above-described sequences of the invention may be caused for instance by addition, deletion, substitution, insertion or recombination. Such a sequence comparison can preferably also be carried out with the program "fasta20u66" (version 2.0u66, September 1998 by William R. Pearson and the University of Virginia; see also W.R. Pearson (1990), Methods in Enzymology 183, 63-98, appended examples and http://workbench.sdsc.edu/). For this purpose, the "default" parameter settings may be used.

It is one object of the invention to provide a kit suitable for the treatment of Huntington's disease (HD) for allele or non-allele-specific huntingtin (*HTT*) gene editing or repair comprising a gene delivery vector consisting of:
- one or more nucleic acid sequences of at least one viral vector selected from the group consisting of adeno-associated vector serotypes (AA from the group consisting of V) and lentiviral vectors (LV);
- at least one nucleic acid sequence that encodes a Cas9 being human codon-optimized or fused to an epitope tag selected among the group of FLAG, His, myc, Tap, HA, V5. Preferably Cas9 is fused to fused to an epitope tag (e.g V5 tag).
- at least one nucleic acid sequence that encodes at least one artificial single guide RNA (sgRNA) having a total size from 63-115 nucleotides comprising a tracrRNA sequence of 48-85 nucleotides long (preferably 82bp) and a crRNA sequence encoded by a nucleic acid sequence selected from the group consisting of SEQ ID NOS: 4-7 and 13-39 and recognizing the sequence of the *HTT* gene within the region defined by position 3066800 to position 3086939 excluding the expanded CAG repeat mutation (ENSG00000197386, position of the first CAG repeat in exon 1: 3'074'877-3'074'879), wherein said crRNA sequence binds directly upstream of the required 5'-NGG/NAG-3' protospacer adjacent motif (PAM) and whereas said crRNA sequence base-pairs with the target *HTT* sequence and Cas9 mediates a double-stranded break (DSB) 3-4 bp upstream of said PAM

In particular, the region around the expanded CAG repeat mutation in the *HTT* gene includes: the promoter (Ensembl accession number: ENSG00000197386; positions 3066800 to 3074509),
exon 1 of the *HTT* gene including the 5'UTR the transcription and translation start sites (TSS and ATG; Ensembl accession number: ENSG00000197386: positions 3074510 to 3075088), intron 1 of the *HTT* (Ensembl accession number: ENSG00000197386: positions 3075089 to 3086939)

Preferably, the gene delivery vector contains various expression cassettes comprising promoters and/or miRNA-regulated system so as to modulate transgene expression.

In a preferred mode of the invention, the AAV is a mutant vector and preferably the AAV-DJ for HR

In another embodiment, of the invention, the Cas9 is mutated to improve the efficiency and safety. Preferably said mutated Cas9 is a Cas9 nickase or a Cas9-V5.

In another embodiment of the invention the gene delivery vector consists in a single vector or several gene delivery vectors.

According to a preferred mode of the invention, the kit comprises multiple transcriptionally independent sgRNA as defined in claim 1 targeting the *HTT* gene.

In another embodiment of the invention, the kit further comprises an exogenous DNA template sharing homology around the *DSB* target site for homologous recombination (HR). Said exogenous DNA template having at least 95% of homology (preferably 98%) with the homology arms located 10-20bp away from the double strand break site and with a minimum length of 100bp and up to 2.5 kb on both side of the double-stranded break (DSB).

In such embodiment, the exogenous DNA template for *HTT* HR contains or does not contain the transcriptional start sites (TSS) and said exogenous DNA template may be selected among the group comprising SEQ ID NO: 42-43. Preferably said exogenous DNA template for HR does not contain the TSS such as SEQ ID NO: 43.

It is another object of the invention to provide a kit, for use in a method of treatment of Huntington's disease in patients by *in vivo* method non-allele-specific HTT inactivation of the human (or other species having a conserved sequences) *HTT* WT (wild type) and mutant alleles by preferentially targeting the region upstream of the *HTT* gene (position 3066800) up to the 5' end of intron 1 (position 3086939). As exemplified in SEQ ID NO: 4-6 and SEQ ID NO: 15, 28, 37-39 or SEQ ID NO 7, 13-14, 16-27, 29-35, if the SNP is homozygous (i.e. WT and mutant HTT alleles have the same sequence at the SNP considered) in the treated HD patients.

Another object of the invention is to provide a kit, for use in a method of treatment of Huntington's disease in patients by *in vivo* non-allele-specific *HTT* gene repair based on HR with a DNA template containing a WT *HTT* sequence as exemplified in SEQ ID NO: 42-43.

A further object of the invention is to provide a kit, for use in a method of treatment of Huntington's disease in patients by *in vivo* method for allele-specific *HTT* inactivation of the human mutant *HTT* gene wherein the sgRNA of said kit is capable of recognizing SNP sequences located upstream a PAM along the *HTT* gene with a high frequency of heterozygosity in the human population at least > 5% (according to dbSNP or 1000 Genome Project database), preferably more than >15%, even more than >20% and most preferably > 30% according to dbSNP; http://www.ncbi.nlm.nih.gov/projects/SNP/snp_ref.cgi?geneId=3064 or 1000 Genome Project database). This is exemplified for example by SEQ ID NO: 7, SEQ ID NO: 13-38.

The invention also provides a kit, for use in a method of treatment of Huntington's disease in patients by *in vivo* mutant *HTT* gene repair based on HR with a DNA template containing a WT *HTT* sequence wherein the sgRNA of said kit is capable of recognizing SNP sequences located upstream a PAM along the *HTT* gene with a high frequency of heterozygosity in the human population at least > 5% (according to dbSNP or 1000 Genome Project database), preferably more than >15%, even more than >20% and most preferably > 30% according to dbSNP; http://www.ncbi.nlm.nih.gov/projects/SNP/snp_ref.cgi?geneId=3064 or 1000 Genome Project database). This is exemplified for example by SEQ ID NO: 7, SEQ ID NO: 13-38.

A frequency of heterozygosity in the human population at least > 5%, means that, on average, >5% of the human population will have a different sequence (heterozygous SNP) on the WT and mutant HTT allele at the SNP considered and >5% of the population would be eligible for an allele-specific strategy. As a consequence, the other <95% of the human population will have the same sequence (homozygous SNP) on the WT and mutant HTT alleles and would be eligible for a non-allele specific HTT gene editing. SNP with high frequency of heterozygosity would greatly facilitate allele-specific therapeutic strategies, because a limited number of products need to be developed to treat a great majority of the HD patients.

Permanent expression of Cas9 after target sequence editing could raise potential adverse effects through off-target mutagenesis.

In a preferred aspect, the kit suitable for the treatment of Huntington's disease further comprises an additional transcriptionally independent artificial sgRNA capable of self-inactivating Cas9 expression after human *HTT* editing, wherein said additional transcriptionally independent artificial sgRNA comprises a crRNA sequence which recognizes key regions driving Cas9 expression selected among the ATG and/or the promoter driving the expression of Cas9.

In particular said additional transcriptionally independent artificial single guide RNA (sgRNA) has a total size from 63-115 nucleotides, preferably 102 nucleotides, comprising a tracrRNA sequence of 48-85 nucleotides long (preferably 82bp) and a crRNA sequence of 20 nt, recognizing key regions driving Cas9 expression (ATG and/or vector promoter) in order to self-inactivating Cas9 expression after human *HTT* editing (SEQ ID NO: 40). Weak polymerase III promoters, with for example the 7SK promoter are used to express sgCas9, while strong polymerase III promoters (HI and U6) are used to express the sgHTT. This ensures a rapid cleavage of the human *HTT* gene and then a progressive induction of DSB in Cas9 (self-cleavage by the sgCas9). Because of Cas9 gene disruption, the expression of the protein is blocked thus, preventing potential adverse effects associated with long-term and uncontrolled Cas9 expression.

In a yet further embodiment, the kit is for use in a method of treating Huntington's disease patient in need thereof, wherein the gene delivery vector used to deliver said (CRISP system) Cas9 and said at least one artificial single guide RNA (sgRNA) as defined in claim 1 to neuronal and glial cells and is suitable for a local administration in the human striatum or is engineered to allow diffusion and/or transport from the striatum to the entire of the basal ganglia network of said patient. This is achieved through the combined use of cell-type specific promoters, microRNA regulatory mechanisms, LV pseudotypes with various envelopes or AAV serotypes.

Besides, diffusion and/or transport may be carried out through the use of specific envelopes including the VSV-G-rabies chimeric envelops for the pseudotyping of LV or use of specific AAV serotypes (19, 20).

Preferably the gene delivery vector is capable of targeting neurons and/or glial cells. Huntington's disease is a neurodegenerative disorder characterized by a selective vulnerability of the striatum in the first stages of the pathology, followed by a more global degeneration throughout the brain, in particular in the basal ganglia network. Described is the use of a kit containing viral vectors expressing the CRISPR system after injection into the striatum. Applicants also provide engineered viral vectors with the capacity to diffuse and/or being transported trans-synaptically for infection of cells along the basal ganglia network.

Mutant HTT exerts numerous molecular adverse effects leading to the degeneration of neurons of the striatum (medium-sized spiny neurons or MSN), but also other brain regions at later stages. If neurons are dying, glial cells, in particular astrocytes, are also vulnerable to mHTT and their dysfunctions exacerbate neuronal deficits. Whether treating neurons in HD patients will be sufficient to solve the pathology has not been fully addressed. In the present invention, Applicants provide a kit containing viral vectors expressing the CRISPR system being engineered to target neurons or glial cells or both neuronal and glial cells in HD patients.

Also provided is a pharmaceutical composition comprising the gene delivery vector of the invention in combination with a pharmaceutically acceptable carrier. The above detailed description disclosing the kit of the present invention is incorporated herein by reference in its entirety for a full disclosure of the pharmaceutical composition comprising the gene delivery vector of the present invention. Namely, the pharmaceutical composition comprises the gene delivery vector consisting of:
- one or more nucleic acid sequences of at least one viral vector selected from the group consisting of adeno-associated vector serotypes (AA from the group consisting of V) and lentiviral vectors (LV);
- at least one nucleic acid sequence that encodes a Cas9 being human codon-optimized or fused to an epitope tag selected among the group of FLAG, His, myc, Tap, HA, V5. Preferably Cas9 is fused to fused to an epitope tag (e.g V5 tag).
- at least one nucleic acid sequence that encodes at least one artificial single guide RNA (sgRNA) having a total size from 63-115 nucleotides comprising a tracrRNA sequence of 48-85 nucleotides long (preferably 82bp) and a crRNA sequence encoded by a nucleic acid sequence selected from the group consisting of SEQ ID NOS: 4-7 and 13-39 and recognizing the sequence of the *HTT* gene within the region defined by position 3066800 to position 3086939 excluding the expanded CAG repeat mutation (ENSG00000197386, position of the first CAG repeat in exon 1: 3'074'877-3'074'879), wherein said crRNA sequence binds directly upstream of the required 5'-NGG/NAG-3' protospacer adjacent motif (PAM) and whereas said crRNA sequence base-pairs with the target *HTT* sequence and Cas9 mediates a double-stranded break (DSB) 3-4 bp upstream of said PAM

Preferably, the pharmaceutical composition of the invention further comprises pharmaceutically acceptable diluents, or excipients that are well known to the skilled in the art.

Applicants propose to use gene editing with the CRISPR system approach for HD. It concerns a new therapy for a fatal neurodegenerative disorder with no available treatment. This represents the first attempt *of in vivo* gene editing and repair for a CNS disorder.

Contrary to WO 2014/093701 (Feng Zhang, MIT, USA), if a gene editing strategy has been proposed for HD, the target sequence is limited to:
1) a mutant HTT allele specific guide RNA: "nucleotide sequences unique to the mutant HTT allele (CAG repeats) and its use to design guide RNA. Authors ensure that the mutant base is located within the last 9 bp of the guide RNA (which Authors have ascertained it has the ability to discriminate between single DNA base mismatches between the target size and the guide RNA)" or alternatively
2) "Authors use Cas9 nickases in combination with pairs of guide RNAs to generate DNA double strand breaks with defined overhangs. It is stated that when two pairs of guide RNAs are used, it is possible to excise an intervening DNA fragment".

The present invention is based on entirely new and innovative strategies for allele or non-allele-specific *HTT* editing and which is also capable of gene repair strategies targeting the promoter, transcription and/or translation start sites and/or SNP in the *HTT* gene. HTT gene disruption based on sgRNA targeting the CAG expansion or double-strand break with overhang (2-4 sgRNA and Cas nickase) are not considered in the present invention.

Applicants strategy is not based on repressors to inhibit mHTT expression (WO 2013/130824 & Garriga-Canut et al. 2012 (7)) but will adress the possibility to perform gene editing to inactivate mutant HTT and DNA repair to restore WT HTT expression using DNA-targeted nucleases. In particular, one will use the recently described CRISPR system to induce mHTT double-strand break for permanent mHTT knock-out or repair.

One important prerequisite for the development *of in vivo* gene repair strategy in the CNS is an efficient delivery system. Applicants have integrated the CRISPR system in lentiviral vectors (LV expressing Cas9 and the sgRNA) and adeno-associated virus (AAV containing the donor DNA for HR or expressing Cas9 and the sgRNA), which have been widely used in vitro but also for in vivo gene delivery in the CNS.

As a first step toward the establishment of an *in vitro* gene editing system for *mHTT,* Applicants performed experiments with reporter genes in HEK 293T cells. For this, a sequence containing the mCherry fluorescent protein followed by the CRISPR target sequence containing a STOP codon and the GFP gene was integrated in the genome of HEK 293T cells using LV (Examples 1-2). mCherry-positive cells were then infected with LV expressing Cas9 (SEQ ID NO: 8) and an sgRNA recognizing the target sequence close to the stop codon were designed (sgTARGET, SEQ ID NO: 2; Figure 2A). As controls, Applicants infected cells with Cas9 or sgTARGET alone. The potency of DNA DSB appearance and NHEJ was quantified with the surveyor nuclease assay and specific restriction-length fragment polymorphisms (RFLP). Results at the DNA levels show up to 45% of DSB, 2 weeks post-infection (Figure 3B). Using Western blots, we observed the fusion mCherry-GFP protein after NHEJ formation (Figure 3D). Additionally, 3 days post-transfection of HEK-mCherry-GFP cells with a plasmid containing the sequence for homologous recombination without a stop codon, Applicants observed around 27% of HR (Figures 3C-D; SEQ ID NO: 9). The efficiency of DSB was further increased when multiple sgRNA (sgGFP1, sgGFP2 and sgGFP3, respectively SEQ ID NO: 3, 10, 11) targeting the GFP reporter gene were used (Figure 3E). Finally, Applicants infected mouse primary cortical neuronal (Figure 4A-C) and astrocytic cultures (Figure 4B; Example 3) with LV expressing Cas9, eGFP and Tomato-sgGFPl targeting the eGFP sequence (sgGFP1). Applicants observed strong eGFP NHEJ formation at the target site couples with a loss of eGFP fluorescence in both cell populations 3 weeks post-infection (Figures 4A-C). This shows that Applicants' CRISPR system efficiently disrupt eGFP following DSB formation in primary cells.

Applicants evaluated the efficiency of the CRISPR system in the mouse brain to disrupt eGFP sequence (Example 5). Applicants have injected AAV (Figure 4D) or LV (Figure 4E-F) encoding Cas9 and sgGFP1 sequences in the striatum of mice expressing the GFP reporter gene (AAV-GFP or LV-GFP). No toxicity or adverse effects were observed up to 4 weeks post-infection in neurons, as revealed by the preservation of NeuN and DARPP-32 immunostaining (data not shown). In addition, a strong loss of GFP signal was found 3 and 4 weeks post-infection in the infected area (Figure 4E) and gene disruption reached up to 56.6% (Figure 4F). Importantly, eGFP-negative neurons are still expressing the GABAergic marker DARPP32, confirming that eGFP loss is due to gene disruption and not major neuronal dysfunction. Similar loss of eGFP fluorescence was observed after injection of LV-gfaABC1D(B)3-Cas9 (astrocytic tropism) and LV-Tomato-sgGFP1 in BAC GLT1-eGFP transgenic mice expressing eGFP specifically in astrocytes (Figure 4G).

Applicants engineered a new Cas9 sequence fused to a V5-tag allowing the visualization of Cas9 protein by immunohistochemistry (Example 1, SEQ ID NO: 41). The V5-tag has been added at the 3' end of the Cas9 gene just after the nuclear localisation signal (NLS). Expression of the Cas9-V5 tag (cloned in AAV or LV backbones) in HEK-293T cells reveal that the most of the staining is located in the cytoplasm, with some molecules are present in the nucleus (Figure 5A). This indicates that the fusion of V5-tag close to the NLS has slightly modified the subcellular localization of Cas9, while maintaining its activity. This new property of Cas9-V5 might be particularly interesting to reach high DSB while limiting potential toxic effects in the CNS.

Applicants used gene editing with the CRISPR system as therapeutic strategy for HD. One important prerequisite for the development of *in vivo* gene editing/repair strategy in the CNS is an efficient delivery system. Applicants use reporter genes to develop and validate VSV-G pseudotyped LV (but other envelopes might be considered) and AAV vectors (AAV2/5, AAV2/6 and AAV-DJ, but other serotypes or chimeric AAV variants might be considered, including AAV1, 9, 10) (21). The AAV-DJ has been shown to increase spontaneous HR efficiency (22) and combined with the CRISPR system is increasing *HTT* editing efficiency. These gene transfer systems are suitable for *in vitro* and *in vivo* delivery (intraparenchymal or intraventricular or peripheral administrations) of the CRISPR system (data not shown). To optimize *HTT* gene editing, various vectors and expression cassettes were produced and tested (choice of the promoters driving the expression of the transgenes, ratio between Cas9 and sgRNA, integration of multiple sgRNA, integration of the CRISPR system in a single vector or in two separate vectors, choice of LV pseudotyping and AAV serotypes to ensure large diffusion and retrograde/anterograde transport in large areas of the CNS).

To block mutant *HTT* (*mHTT*) expression, Applicants did not use repressors (ZFN or TALEN) (WO 2013/130824) but disrupt the *HTT* gene (non allele- or allele-specific targeting) using DNA-targeted nucleases (CRISPR). In particular, Applicants use the recently described CRISPR system to induce *mHTT* double-strand break (DSB) around the transcription start site (TSS) or translation initiation codon (ATG) of the *HTT* gene for permanent and irreversible *mHTT* inactivation (Examples 3-4, Figures 6-7, SEQ ID NO: 4-7, SEQ ID NO: 13-39). Both DNA strand (sense or anti-sense) are targeted by the sgRNA. Species-specific or sgRNA targeting conserved region of the *HTT* gene in human but also rodent and non-human primates were selected (SEQ ID NO: 4-7, SEQ ID NO: 13-39). The later one will greatly facilitate the pre-clinical validation of *HTT gene* editing in rodent and large animal models (primates) (SEQ ID NO: 4-6, 15, 28, 37-39; Figure 2B). To maximize NHEJ efficiency, vectors with multiple sgRNA have been produced (SEQ NO 12; Figure 3E).

The sgHTT targeting regions around of ATG or the TSS were transfected in 293T cells (SEQ NO 4, 36). Gene editing efficiencies reaching 17-45% were observed on endogenous *HTT* locus (Figure 6A-B). Transfection of human embryonic kidney cells (HEK 293T cells) with a plasmid expressing a fragment of the human *m*HTT (first 171 amino acids with 82 CAG repeats) fused to the green fluorescent protein (GFP) reporter gene was used to further evaluate the impact of NHEJ on the protein aggregation. In 2/3 of the cases, indels modify the reading frame of the HTT-GFP and lead to the appearance of an early STOP codon (decrease GFP expression). Surveyor, western blots and cell imaging were used to confirm that NHEJ formation with sgHTT1 leads to a decrease of *HTT-GFP* protein and aggregation in HEK-293T cells (Example 3-4, Figure 6C-D).

The feasibility and efficacy of the approach was further validated in post-mitotic primary neuronal and astrocytic cultures infected with an LV expressing the first 171 amino acids of human HTT with 82 CAG and LV expressing Cas9 and the sgHTT1 (full homology for human *HTT* and one mismatch at the 5' end of sgRNA with endogenous mouse *HTT,* Figure 7). A strong disruption of both human and mouse HTT was observed 3 weeks post-infection of mouse cortical striatal neurons demonstrating that the CRISPR system efficiently disrupt both human and mouse *HTT* genes. Immunostaining of mHTT aggregates demonstrate that the treatment with the CRISPR-HTT1 vectors strongly decreased both large and small mHTT aggregate of (Figure 7B). Similar data were observed when the CRISPR-HTT1 system was used in the mouse striatum four weeks post-injection (Figure 7C).

For allele-specific inactivation, Applicants selected single-nucleotide polymorphisms (SNP) located the *HTT* gene with high frequency of heterozygosity in the human population (> 5%, preferably > 15%, more preferably >20% and even more preferably >30%) (http://www.ncbi.nlm.nih.gov/projects/SNP/snp_refcgi?geneId=3064) with a preference for SNP located near the TSS/ATG and HD mutation and designed sgRNA targeting the disease isoform of heterozygous single-nucleotide polymorphisms (SNP; Figure 1, 2B; SEQ ID 7, 13-35).

As a second strategy for the allele-specific gene editing of mutant *HTT* gene, Applicants use two sgRNAs, one targeting a SNP located close to the TSS/ATG and HD mutation of the *HTT* gene and a second sgRNA targeting the disease isoform of heterozygous single-nucleotide polymorphisms (SNP) located after the CAG expansion and preferentially in an intron (Figure 2B). This leads to the complete deletion of exon 1 containing the TSS/ATG codon and expanded CAG mutation of the *HTT.*

For non-allele-specific or allele-specific DNA repair strategy, an sgRNA targeting any sequence located after the CAG expansion, preferentially in intron 1 of the *HTT* gene has been used to induce DSB and a donor sequence containing the WT human *HTT* exon1 and flanking sequences was used to induce homologous recombination (SEQ ID NO: 42-43). To avoid potential expression of a short HTT fragment in the recombination vector (HR donor template), the HR sequence is preferably excluding the TSS/ATG sites. For the allele-specific repair of *mHTT,* Applicants used sgRNA targeting the disease isoform of heterozygous single-nucleotide polymorphisms (SNP) located after the CAG expansion and preferentially in an intron (Figure 2B-C).

Finally, as a first strategy to transiently express Cas9 protein, Applicants design an sgRNA targeting the Cas9 gene (Example 1, SEQ ID NO 40). Eliminating Cas9, should further improve the biosafety of the proposed approach. Weak promoters should be favored to favor genome editing at the beginning of the treatment and then progressively inactivating Cas9 protein. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended claims.

The foregoing description will be more fully understood with reference to the following Examples. Such examples are however exemplary of methods of practicing the present invention.

### Examples

### Example 1: Development of a viral-based delivery system for the CRISPR system

### Material and methods

### Plasmid construction

The pcDNA3.3-TOPO-CMV-hCas9 plasmid contains the human codon-optimized Cas9 (hCas9, SEQ ID NO: 8) sequence fused to a nuclear localization signal expressed under the CMV promoter (Addgene, Cambridge MA, USA). The hCas9 gene was excised from the plasmid using PmeI and NcoI (Roche Diagnostics GmbH, Mannheim, Germany) restriction sites and inserted into a pENTR-dual plasmid (Invitrogen, Life Technologies, Regensburg, Germany) digested with NcoI and EcoRV (Roche Diagnostics GmbH, Mannheim, Germany) (pENTR-dual-hCas9). Finally, a site-specific recombination was performed with the gateway system (Invitrogen, Regensburg, Germany; (5)) and the destination vector SIN-cPPT-PGK-RFA-WPRE, SIN-cPPT-CMV-RFA-WPRE, SIN-cPPT-gfaABC1D(b)3-RFA-WPRE, pAAV2ss-ITR-CBA-RFA-WPRE-bGHpolyA-ITR and pAAV2ss-ITR-PGK-RFA-WPRE-bGHpolyA-ITR (23) to produce the SIN-cPPT-PGK-hCas9-WPRE, SIN-cPPT-CMV-hCas9-WPRE, SIN-cPPT-gfaABC1D(b)3-hCas9-WPRE, pAAV2ss-ITR-CBA-hCas9-WPRE-bGHpolyA-ITR and pAAV2ss-ITR-PGK-hCas9-WPRE-bGHpolyA-ITR.

The pMA-T-hCas9frag-V5 containing the last 692bp of hCas9 including the V5-tag (SEQ ID NO 8, 41, GeneArt, Invitrogen, Life Technologies, Regensburg, Germany) was digested with XhoI and XbaI and inserted in the pENTR-dual-hCas9 to replace the 3' last nucleotides of hCas9 between the XhoI and XbaI sites (pENTR-dual-hCas9-V5, Roche Diagnostics GmbH, Mannheim, Germany). Then, a site-specific recombination was performed with the gateway system (Invitrogen, Regensburg, Germany; (5)) and the destination vector SIN-cPPT-PGK-RFA-WPRE, SIN-cPPT-CMV-RFA-WPRE, pAAV2ss-ITR-CBA-RFA-WPRE-bGHpolyA-ITR and pAAV2ss-ITR-PGK-RFA-WPRE-bGHpolyA-ITR (23) to produce the SIN-cPPT-PGK-hCas9-V5-WPRE, SIN-cPPT-CMV-hCas9-V5-WPRE, pAAV2ss-ITR-CBA-hCas9-V5-WPRE-bGHpolyA-ITR and pAAV2ss-ITR-PGK-hCas9-V5-WPRE-bGHpolyA-ITR.

The sgRNA are expressed under the control of the HI, U6 or 7SK polymerase III promoters. The H1-sgTARGET (targeting the mCherry-target-GFP sequence, SEQ ID NO 2; Figures 1 and 2A) was synthesized and cloned in pDONR221 vector (GeneArt, Invitrogen, Life Technologies, Regensburg, Germany). The sgRNA targeting the GFP (sgGFP1, SEQ ID NO: 3) was synthesized in a pMK-T vector (GeneArt, Invitrogen, Life Technologies, Regensburg, Germany) and transferred in pDONR221 (pDONR221-H1-sgGFP). Finally, sgRNA targeting the huntingtin (sgHTT1; SEQ ID NO 4) were synthesized as phosphorylated oligonucleotides (Microsynth AG, Balgachm, Switzerland) and annealed in a thermocycler (BioLabo, Chatel-Saint-Denis, Switzerland). The resulting double-stranded DNA was digested by using BglII and NdeI restriction sites (Roche Diagnostics GmbH, Mannheim, Germany) and inserted into the pDONR221-H1-sgRNA digested by BglII and NdeI (pDONR221-H1-sgHTT).

The plasmid containing the three sgGFP under independent polymerase III promoters was ordered from GeneArt (pENTR221-H1-sgGFP1-U6-sgGFP2-7SK-sgGFP3, GeneArt, Invitrogen, Life Technologies, Regensburg, Germany, SEQ ID NO 3,10-12). Restriction enzyme cutting were performed to produce pENTR221-H1-sgGFP1, pENTR221-H1-sgGFP1-U6-sgGFP2, pENTR221-H1-sgGFP1-7SK-sgGFP3, pENTR221-U6-sgGFP2, pENTR221-U6-sgGFP2-7SK-sgGFP3 and pENTR221-7SK-sgGFP3 (pENTR221-polIII). sgHTT2-sgHTTn were synthesized as phosphorylated oligonucleotides (MicrosynthAG, Balgachm, Switzerland) and annealed in a thermocycler (BioLabo, Chatel-Saint-Denis, Switzerland) or ordered directly as small double-stranded DNA blocks (gBlocks, Integrated DNA Technology, (IDT), Leuven, Belgium). Cloning of various sgHTT in pENTR221-polIII was done using the appropriate restriction enzyme depending on the promoter of choice (SEQ ID NO 4-7, 13-39).

A gateway reaction was used to insert each sgRNA in the transfer vectors for LV production (SIN-H1-gateway cassette-WPRE, SIN-cPPT-PGK-tdTomato-WPRE-LTR-TREtight-H1-gateway cassette, and SIN-cPPT-gateway cassette-PGK-mCherry-WHV) (5, 23, 24). For AAV, a unique Asp718 restriction site between the 5'ITR and CBA promoter of a pAAV2ss-ITR-CBA-mCherry-WPRE-bGHpolyA-ITR plasmid was digested and blunted to insert the sgRNA of choice from the pENTR221-polIII to generate the final pAAV2ss-ITR-polIII-sgRNA-CBA-mCherry-WPRE-bGHpolyA-ITR vector.

Plasmid coding for the mCherry-target-GFP sequence was designed, synthesized and cloned in pDONR221 vector (GeneArt, Invitrogen, Life Technologies, Regensburg, Germany) (pDONR221-mCherry-target-GFP) (Figure 2A). A LR clonase (Invitrogen, Life Technologies, Regensburg, Germany) was used to insert the target sequence into a transfer vector for LV production (SIN-cPPT-PGK-mCherry-target-GFP-WPRE). The DNA template for HR with the mCherry-target-GFP reporter system (Figure 2A) was synthesized (SEQ ID NO: 9, GeneArt, Invitrogen, Life Technologies, Regensburg, Germany) and cloned into the pDONR221 plasmid (pDONR221-template). Finally it was transferred into the pAAV2ss-ITR-DEST-RFA-bGHpoly-ITR plasmid by LR clonase reaction (kindly provided by Dr. A. Bemelmans, CEA/DSV/I2BM/MIRcen, Fontenay-aux-Roses, France) to produce the AAV2-ITR-template-bGHpolyA-ITR.

To evaluate NHEJ with sgRNA targeting the human *HTT,* a plasmid encoding the mutant HTT (first 171 amino acids of the human HTT with 82 CAG repeats; was fused to the eGFP (Clontech, Saint-Germain-en-Laye, France) containing 5 copies of myc tag epitope (EQKLISEEDL). This fusion construct was cloned in a third generation LV transfer vector (25) under a CMV promoter (pRRL-cPPT-CMV-Htt171-82Q-5xMyc-GFP-WPRE). The same construct without eGFP was used to generate the SIN-cPPT-PGK-Htt171-82Q-WPRE for *mHTT* editing in neurons. To identify infected cells *in vivo,* a LV transfer vector encoding the red monomer fluorescent protein mCherry was used (SIN-cPPT-PGK-mCherry-WPRE).

### Cell culture

HEK-293T cells (LGC, Wesel, Germany; human embryonic kidney cell line immortalized by the adenoviral E1A/E1B protein and expressing the SV40 large T antigen) were cultured in DMEM-Glutamax supplemented with 10% FBS and 1% Penicillin/Streptomycin (Gibco, Life Technologies, Zug, Switzerland). Cells were passed every three days using Trypsin for dissociation (Gibco, Life Technologies, Zug, Switzerland). For transfection experiments, 300'000 cells were plated in corresponding wells (6-wells; Vitaris, Baar, Switzerland) the day before transfection. Transfections were done using calcium-phosphate method. Up to 4µg total of plasmids for 9.5cm2 plate (6 well) were mixed in equal amount of H₂O and CaCl₂ and then added drop by drop in HEPES (Sigma-Aldrich, Buchs, Switzerland, ratio plasmid-H₂O-CaCl₂: HEPES = 1:1). Mixture was incubated at RT for 5 minutes and then added to the corresponding cultures drop by drop. Medium was completely changed 6 hours post-transfection.

### LV production and titration

LV were produced in HEK-293T cells with the four-plasmid system, as described previously (24). The HIV-1 vectors were pseudotyped with the VSV-G envelope and concentrated by ultracentrifugation and resuspended in phosphate-buffered saline (PBS, Gibco, Life Technologies, Zug, Switzerland) with 1% bovine serum albumin (BSA, Sigma-Aldrich, Buchs, Switzerland). The viral particle content of each batch was determined by p24 antigen enzyme-linked immunosorbent assay (p24 ELISA, RETROtek; Kampenhout, Belgium). The stocks were finally stored at -80°C until use.

### AA Vproduction

The AAV recombinant genome contains the transgene under the control of mammalian promoters, the WPRE, and the bovine hormone (bGH) gene polyadenylation signal, flanked by AAV2 ITRs. This expression cassette was encapsidated in an AAV-5, 6, DJ capsid as described previously (22, 26, 27). For this, the plasmids coding for the transgene, the capsid and Ad helper were transfected by calcium phosphate precipitation in HEK-293T cells. Cells and supernatant were harvested 3 days post-transfection and centrifuged at 300 g for 10 minutes at 4°C. Supernatant was supplemented with 8% PEG (Sigma-Aldrich, Buchs, Switzerland, 2.5M NaCl, Merck, Nottingham, UK) and was kept at 4°C for at least 2 hours. Cell pellets were pooled and incubated in lysis buffer (NaCl 0.15M, Merck, Nottingham, UK, Tris-HCl 50mM, pH 8.5, Sigma-Aldrich, Buchs, Switzerland) for 3 consecutive freeze/thaw cycles (30 minutes in dry ice/ethanol followed by 30 minutes at 37°C). PEG-containing supernatant was centrifuged at 4'000 g for 20 minutes at 4°C after the 2 hours incubation and supernatant was discarded. Cell lysate was added to the pellets and the mixture was incubated at 37°C for 1 hour to ensure full homogenization of pellets. Lysate was then treated with benzonase (0.15 units, Sigma-Aldrich, Buchs, Switzerland) in filtered MgCl₂ 1M at 37°C for 30 minutes (Sigma-Aldrich, Buchs, Switzerland). Treated lysate was clarified by centrifugation at 4'000 g for 20 minutes at 4°C. AAV were separated using iodixanol (AxonLab, Le Mont sur Lausanne, Switzerland) gradient ultracentrifugation at 59'000 rpm (70Ti rotor, Beckman-Coulter, Nyon, Switzerland) for 90 minutes at 20°C as previously described. Phase containing AAV was harvested and loaded on an Amicon Ultra-15 PL 100 (Millipore, Zug, Switzerland) with 0.001% Pluronic F68 D-PBS (Gibco, Life Technologies, Zug, Switzerland) for iodixanol cleaning and viral particles concentration. Tubes were first centrifuged at 4'000 g at 4°C until the totality of solution has passed through the column. Two additional wash with 0.001% Pluronic F68 D-PBS were done and AAV were finally resuspended in 250-500µL 0.001% Pluronic F68 D-PBS. AAV were kept at -80°C until the use. Titration was performed by qPCR as previously described (28).

### Results

To validate and assess the efficacy of the CRISPR system, two sgRNAs targeting the mCherry-target-GFP (sgTARGET) and GFP (sgGFP1, 2, 3) sequences were designed (Figure 1 and 2A; SEQ ID NO: 3, 10-12). In addition, sgRNA targeting the human, rodent and primate *HTT* were produced (sgHTT1) (Figure 2B; SEQ ID NO: 4). The majority of them are targeting the 5' region, promoter, exon and intron 1 of the *HTT* gene to ensure loss of mutant HTT expression or repair of the mutation. The DNA templates for HR (Figure 2C; SEQ ID NO: 9) were delivered with AAV (and in particular AAV-DJ) to ensure high number of episomes in the nucleus. Functionality of plasmids containing reporter genes was evaluated by transient transfection in HEK-293T cells (data not shown).

### Example 2: CRISPR optimization for DSB and HR

### Material and methods

### Cell culture

HEK-293T cell culture was performed as described in the example 1. An LV-SIN-cPPT-mCherry-target-GFP-WPRE containing a target sequence and a GFP fluorescent protein (Figure 2A) was used to infect HEK-293T cells and produce populations suitable to evaluate DSB formation (HEK-TARGET). HEK-TARGET cells were infected with the various amount of viral particles diluted in culture medium. Up to 600ng p24 were used to infect 3x10⁵ HEK-293T cells in 9.5cm2 wells (6 well). Cells were passed every 7 days after infection and medium was changed every 3 days.

Evaluation of the functionality of hCas9-V5 was done by transfection as described in example 1. Plasmids encoding hCas9-V5 in AAV (pAAV2ss-ITR-CBA-hCas9-V5-WPRE-bGHpolyA-ITR and pAAV2ss-ITR-PGK-hCas9-V5-WPRE-bGHpolyA-ITR) and LV backbones (SIN-cPPT-PGK-hCas9-V5-WPRE and SIN-cPPT-CMV-hCas9-V5-WPRE) were used. As a positive control for immunohistochemistry, SIN-cPPT-PGK-Tau-V5-WHV was used as previously described (29). Comparison of hCas9 and hCas9-V5 editing efficiency was done by transfecting SIN-cPPT-PGK-GFP-WHV and SIN-cPPT-PGK-Tomato-WHV-LTR-TREtight-H1-sgGFP1 and SIN-cPPT-CMV-hCas9-V5-WPRE (Cas9-V5 group) or SIN-cPPT-CMV-hCas9-WPRE (Cas9 group) in HEK-293T cells. Additive effect of multiple independent sgRNA was evaluated with SIN-cPPT-PGK-GFP-WHV, SIN-cPPT-CMV-hCas9-WPRE and SIN-cPPT-H1-sgGFP1-U6-sgGFP2-7SK-sgGFP3-PGK-mCherry-WHV (CRISPR-poly-sgGFP) or SIN-cPPT-PGK-Tomato-WHV-LTR-TREtight-H1-sgGFP1 (CRISPR-GFP).

### FACS

HEK-TARGET cells were harvested and processed for FACS analysis 3-5 weeks post-infection. The cells were washed and resuspended in small volume of sterile PBS to ensure high cell concentration (1-10x10⁶/ml). For each sample, 50'000 events were analyzed. mCherry-positive cells were sorted with a Beckman-Coulter system and were grown on 96-well plates. Based on mCherry fluorescence, four distinct cell populations were isolated: cells with low copy number of target sequence (populations 1 and 10) and cells with high copy number of target sequence (populations 50 and 100).

### qPCR

HEK-TARGET cells were passed and resuspended in PBS. Genomic DNA (gDNA) was extracted from 1.10⁶ cells with Trizol Reagent and resuspended at a concentration of 50 ng/µL in DNAse-free water. Provirus copy number was determined by SYBRgreen qPCR (KapaBiosystems, Le Mont sur Lausanne, Switzerland) and the primers HIV-1F-TGTGTGCCCGTCTGTTGTTGT and HIV-2R- GAGTCCTGCGTCGAGAGAGC. qPCR with primers targeting the endogenous human *HTT* were used as genomic standard (HTT-21F-ATGGACGGCCGCTCAGGTTCT, HTT-68R- GCTCAGCACCGGGGCAATGAA). Serial dilutions of gDNA from each population were performed and compared to the standard curve of HTT. Provirus number per cell was quantified based on the Lenti-X provirus quantitation kit (Clontech, Saint-Germain-en-Laye, France).

### Primary neuronal cultures

Timed-pregnant FVB mice were killed by CO₂ inhalation and E17 embryos were collected in Petri dish containing HBSS. Cortex were dissected in corresponding medium containing H₂O, Na₂SO₄ 1M, K₂SO₄ 0.5M, MgCl₂ 1M, CaCl₂ 1M, HEPES 1M pH 7.3-7.4, Glucose 2.5M, Phenol red 0.5%, NaOH IN pH 7.4 (Sigma-Aldrich, Buchs, Switzerland) and Ky/Mg (ImM kynurenic acid / 10mM MgCl₂, Sigma-Aldrich, Buchs, Switzerland) and kept on ice in a 15mL Falcon. Dissection medium was removed and structures were enzymatically dissociated in dissection medium containing 1 mg/mL L-cysteine and 0.125mg/mL papain (Sigma-Aldrich, Buchs, Switzerland). Mixture was incubated at 37°C for 10 minutes, solution was removed and the operation was repeated a second time. After the final incubation, all the papain/cysteine solution was removed and lysates were washed 3 times in dissection medium. Washed lysates were then incubated in dissection medium containing 10mg/mL of trypsin inhibitor containing solution for 7 minutes 30 seconds at 37°C. During wash steps, overnight incubated wells in poly-D-lysine (0.019µg/µL, Becton Dickinson, Allschwill, Switzerland) and laminin (0.027µg/µL, Life Technologies, Zug, Switzerland) with or without glass coverslips were washed three times with H₂O and then incubated in Neurobasal supplemented with 2% B27, 1% penicillin/Streptamycin and 1% Glutamax (Gibco, Life Technologies, Zug, Switzerland) until cells plating. Lysates were then washed 3 times in dissection medium and 3 times in Opti-MEM-Glucose 2.5M (Gibco, Life Technologies, Zug, Switzerland) solution after final dissociation in Neurobasal supplemented with 2% B27, 1% penicillin/Streptamycin and 1% Glutamax (Gibco, Life Technologies, Zug, Switzerland) using Paster pipettes. Fully dissociated cells were pooled in the same tube and counted. The cells were platted at a density of 1,5 x 10⁵ cells per cm2 in multi-wells dishes in Neurobasal supplemented with 2% B27, 1% penicillin/Streptamycin and 1% Glutamax (Gibco, Life Technologies, Zug, Switzerland).

Different AAV serotypes were compared to determine the best delivery system for the DNA template *in vitro.* One day post-seeding of HEK-293T cells, 1x10⁷ Vg of AAV2/5, AAV2/6 and 5.5x10⁶ Vg AAV2/DJ-ITR-CBA-GFP-WPRE-ITR were incubated on cells. The cultures were fixed 2 weeks post-infection in 4% paraformaldehyde (Electron Microscopy Sciences, Hatfield) at room temperature (RT) for 10 minutes, washed 3 times in PBS and analyzed for direct GFP fluorescence.

Cortical neurons were infected with 20ng/wells of LV-cPPT-PGK-GFP-WHV (LV-PGK-GFP) at DIV1. Cells were then infected with LV-cPPT-PGK-Tomato-WHV-LTR-TREtight-H1-sgGFP1 (LV-Tomato-sgGFP1, 15ng) and LV-cPPT-CMV-hCas9-WHV (LV-hCas9, 15ng, Cas9 group) or LV-cPPT-CMV-hCas9-V5-WHV (LV-hCas9-V5, 15ng, Cas9-V5 group). Cells were kept in culture until DIV25.

### Stereotaxic surgery

Concentrated viral stocks were thawed on ice and resuspended by repeated pipetting. AAV were diluted in D-PBS 1x (Gibco, Life Technologies, Zug, Switzerland) to inject 9.5x 10⁷ Vg in 2µL.

Mice were anesthetized by intraperitoneal injection of a mixture of 100 mg/kg ketamine (Ketasol, Graeub, Bern, Switzerland) and 10 mg/kg xylazine (Rompun, Bayer Health Care, Uznach, Switzerland). Suspensions of AAV were injected into the mouse striatum with a 34-gauge blunt-tip needle linked to a Hamilton syringe by a polyethylene catheter. Stereotaxic coordinates were: anteroposterior +1 mm from bregma; mediolateral +/-1.5 mm and dorsoventral -3.5 mm from the skull, with the tooth bar set at -3.3 mm. Mice received a total volume of 3 µl of the vector preparation, administered at a rate of 0.2 µl/min. At the end of injections, needles were left in place for 5 min before being slowly removed. The skin was sutured with 6-0 Prolene suture (B-Braun Mediacal SA, Sempach, Switzerland) and mice were allowed to recover on a heating mat.

### DNA, RNA and proteins extractions

DNA, RNA and proteins were extracted from the same sample with the Trizol reagent according to the manufacturer's protocol (Invitrogen, Life Technologies, Zug, Switzerland). Briefly, cells were washed in PBS (Gibco, Life Technologies, Zug, Switzerland) and lysed in Trizol. DNA and RNA were resuspended in RNAse-DNAse sterile water (Gibco, Life Technologies, Zug, Switzerland) whereas proteins were resuspended in 1% SDS, 10% Urea solution (Sigma-Aldrich, Buchs, Switzerland). RNA were kept at -80°C, proteins were kept at -20°C and DNA diluted to 100ng/µL were conserved at 4°C.

Genomic DNA from primary cultures was extracted using the QuickExtract DNA Extraction Solution 1.0 (Epicentre, Offenbach, Germany) following the manufacturer's instructions. Culture medium were removed and cells were incubated in Trypsin at 37°C for 10 minutes. Cells were resuspended, diluted in 500µL of IX PBS/well and centrifuged at 300g for 5 minutes. Supernatant was removed, cells were resuspended in 50µL of lysis buffer, each tube was vortexed for 15 secondes and incubated at 65°C for 15 minutes with agitation. Each tube was then vortexed 15 secondes and lysis buffer was inactivated at 98°C for 2 minutes. Genomic DNA concentration was quantified with Nanodrop (Thermo Scientific, Reinach, Switzerland) using lysis buffer as blank solution before conservation at -20°C.

### Surveyor analysis

DSB formation was evaluated using the surveyor mutation detection kit, which detects mutations and polymorphisms in DNA (Transgenomic Inc, Glasgow, UK) (30). Briefly, forward (TARGET-1F: ACGGCGAGTTCATCTACAAGGTGAAGCTGC, GFP-1F: TGACAAATGGAAGTAGCACG) and reverse oligonucleotides (TARGET-2R: TTGTACTCCAGCTTGTGCCCCAGGATGTTG, GFP-2R: GAGATGAGTTTTTGTTCGAAGGG) complementary to a region located around the target sequence in the genomic DNA (100 ng) are used with the Taq polymerase (Roche Diagnostics GmbH, Mannheim, Germany) to perform a PCR reaction. The concentration of the amplified fragment is evaluated by agarose gel electrophoresis using the GeneRuller 1kb DNA ladder (Thermo Scientific, Reinach, Switzerland). Three hundred nanograms of PCR product is incubated at 95°C in a thermocycler and subjected to the surveyor nuclease according to the manufacturer's protocol. Migration of the digested products is done on 10% TBE polyacrylamide precast gels (BioRad, Reinach, Switzerland). DNA is stained using SYBR gold (Molecular Probes, Life Technologies, Zug, Switzerland). The percentage of indels is determined by quantifying band intensities with Image J software (http://rsb.info.nih.gov/ij/, NIH, Bethesda) as described by Ran and collaborators (31).

### Restriction fragment length polymorphism (RFLP)

RFLP analysis exploits DNA variations and in particular restriction enzymes. The DNA sample is digested and the resulting restriction fragments are separated according to their lengths by gel electrophoresis. In the specific example described here, Applicants used 300-500ng of PCR product and digest it with the restriction enzyme NcoI for 2h at 37°C (New England Biolabs, Allschwill, Switzerland). The resulting products were analyzed on a 10% TBE polyacrylamide precast gel (BioRad, Reinach, Switzerland) and stained in SYBR gold (Molecular Probes, Life Technologies, Zug, Switzerland). The Image J software was used to quantify the bands intensities and determine the percentage of RFLP (31).

### Western blot analysis

Two days after transfection, the 293T cells were harvested and lysed Trizol. Protein concentration was determined by the Bradford method (Thermo Scientific, Reinach, Switzerland). Twenty micrograms of proteins were run on 12% SDS-polyacrylamide gels, and transferred to nitrocellulose membranes in 0.025M Tris, 0.192M glycine, and 20% ethanol (6). The membranes were washed 3 x 10 minutes in PBS and blocked 1 hour in 33% LI-COR blocking buffer in 1% PBS-Tween20 (blocking, LI-COR, Bad Homburg, Germany) at RT under agitation. Primary antibodies were incubated in blocking solution overnight at 4°C under agitation. The following antibodies were used: anti-mCherry antibody (mCherry, 1/1000, SicGen, Lisboa, Portugal), goat anti-HTT antibody (HTT, 1/1000, SicGen, Lisboa, Portugal) and rabbit anti-Actin antibody (Actin, 1/1000, Abcam, Cambridge, UK). Membranes were rinsed 3 x 10 minutes in PBS-Tween20 and incubated with the following secondary antibodies in blocking for 1 hour at RT under agitation: donkey anti-goat IgG IRdye 800 (1/7000, LI-COR, Bad Homburg, Germany) and donkey anti-rabbit IgG IRdye 680 (1/7000, LI-COR, Bad Homburg, Germany). Membranes were rinsed 3 x 10 minutes in 1% PBS-Tween20 and 3 x 10 minutes in PBS before scanning on Odyssey system (LI-COR, Bad Homburg, Germany). Band intensities were determined using ImageJ software as described previously.

### Immunohistochemistry

Three days post-transfection, HEK-293T cells were washed in IX PBS and fixed in 4% PFA for 15 minutes at room temperature. Immunohistochemistry was performed as described in example 4. Mouse anti-V5 primary antibody (Invitrogen, Life Technologies, Zug, Switzerland) and goat anti-mouse 594 secondary antibody (Invitrogen, Life Technologies, Zug, Switzerland) were used.

### Image acquisitions

Pictures were acquired on a Zeiss Axiovision microscope using 20-40x objectives (Carl Zeiss Microscopy GmBH, Göttingen, Germany). Constant exposure time was used to compare fluorescence of control and treated groups.

### Results

To produce cells with an integrated target sequence, HEK-293T Cells were infected with increasing doses of LV-cPPT-PGK-mCherry-target-GFP-WPRE (MOI 1, 10, 50 and 100) and selected by FACS. Based on mCherry fluorescence, four independent populations with increasing number of integrated provirus were selected (HEK-TARGET; Figure 3A) and used for the optimization of the CRISPR system delivery. Equal amounts of LV-cPPT-CMV-hCas9-WPRE and LV-H1-sgTARGET-WPRE were incubated on each population to determine the efficiency of the CRISPR system. Cells were harvested at 3, 7, 14 and 21 days post-infection and DNA and proteins were extracted. Regions flanking the target sequence were PCR-amplified and subjected to the Surveyor mutation detection assay. In all cases, the percentage of DSB formation increased with time and is inversely proportional with the number of integrated target sequences (Figure 3B). Importantly, cell populations carrying low (12 and 32) and high (52 and 91) provirus copies can be distinguished with Surveyor results 7 days post-infection (45% and 42% compared with 37% and 35%, respectively) but the difference is reduced at 14 days post-infection (48% and 46% compared with 43%, respectively), suggesting that a plateau has been reached. For HR delivery, we used co-transfection of the CRISPR system and the AAV2-ITR-DNA-template-bGHpolyA-ITR plasmids in cell populations 1 and 10. Cells were lysed 3 and 7 days post-transfection and subjected to RFLP with the NcoI restriction site integrated after HR (Figure 2A). Data show an increased HR efficiency with time (respectively 21.7% and 18.1% for populations 1 and 10, 3 days post-transfection and 37.5% and 23.5% for populations 1 and 10 at 7 days post-transfection), which is corroborated by the expression of the fusion mCherry-GFP protein (Figure 3C-D). The Applicants also evaluated the benefit of multiple transcriptionally independent sgRNA targeting the same gene. Co-transfection of plasmids coding for hCas9, Tomato-sgGFPl and GFP in HEK-293T cells result in ∼50% of gene disruption (CRISPR-sgGFP group, Figure 3E). However, the replacement of the Tomato-sgGFPl plasmid by a plasmid encoding H1-sgGFP1-U6-sgGFP2-7SK-sgGFP3 (CRISPR-poly-sgGFP group) leads to a great increase of GFP gene editing up to 80% three days post-transfection (Figure 3E). This demonstrates the added value of multiple sgRNA on DSB formation efficiency.

Applicants have evaluated the delivery of the DNA template using viral vectors in mouse primary neuronal cultures. Applicants used GFP fluorescence to assess the efficiency of gene delivery in those cells. Applicants compared AAV2/5, AAV2/6 and AAV2/DJ-ITR-CBA-GFP-WPRE-bGH polyA-ITR. Direct GFP fluorescence reveals that AAV2/5 serotype is less efficient than AAV2/6 and AAV2/DJ in vitro (data not shown). Only mild differences were observed between AAV2/6 and AAV2/DJ in both HEK-293T cells and primary neurons, revealing that these two serotypes are suitable for *in vitro* HR experiments. For *in vivo* HR experiments, AAV2/5-ITR-CBA-GFP-WPRE-bGHpolyA-ITR was injected in C57Bl/6 mice striatum based on previous studies (27). Direct fluorescence reveals strong GFP signal and large diffusion of the virus, confirming that this serotype is a potent gene transfer system for *in vivo* HR (data not shown).

Finally, Applicants fused the hCas9 sequence to a V5-tag to enable hCas9 labeling by immunohistochemistry (Figure 5). Immunostaining three days post-transfection of various AAV and LV backbones expressing hCas9-V5 in HEK-293T cells reveal that all plasmids encoding hCas9 are expressed. In addition, the majority of the staining was observed in the cytoplasm with few staining in the nucleus, meaning that the addition of the V5-tag reduced the incorporation of hCas9 into the nucleus (Figure 5A). As a consequence, editing efficiency of hCas9 and hCas9-V5 was evaluated in HEK-293T cells by transfection and in cortical neurons with LV infection. For both experiments, a GFP sequence was used as target and Surveyor was used to evaluate the rate of NHEJ. Results demonstrate that hCas9-V5 is as effective as hCas9 constructs (Figure 5B-C).

### Example 3: CRISPR system efficiency in primary neurons and astrocytes

### Material and methods

### Primary neuronal cultures

Mouse cortical neurons were cultured as described in Example 2.

### Primary astrocytic cultures

Before dissection, overnight-incubated wells in poly-D-lysine (15µg/mL, Becton Dickinson, Allschwill, Switzerland) with or without glass coverslips were washed three times with IX PBS (Gibco, Life Technologies, Zug, Switzerland) and then incubated in IX PBS until cells plating. PI mouse cortex were killed by decapitation and dissected in DMEM High Glucose medium (Sigma-Aldrich, Buchs, Switzerland) dissolved in autoclaved nanopure water and supplemented with antibiotic/antimycotic (diluted 10x, Sigma-Aldrich, Buchs, Switzerland) and 3.7g/L of sodium bicarbonate at pH 7.2 (Merck, Zug, Switzerland, DMEM-astro). Dissected cortexes were passed in 19G, 21G and 25G needle with syringe 3 times each to dissociate the structures. Cells were counted and platted in DMEM-astro at a density of 50'000 cells per cm².

### Primary culture infections

Cortical neurons were infected with 20ng/wells of LV-cPPT-PGK-GFP-WHV (LV-PGK-GFP) at DIV1. Cells were then infected with LV-cPPT-CMV-hCas9-WHV (LV-hCas9, 15ng) and LV-cPPT-PGK-Tomato-WHV-LTR-TREtight-H1 -sgGFP 1 (LV-Tomato-sgGFP1, 15ng, CRISPR-GFP group) or LV-hCas9 (15ng) and LV-cPPT-PGK-mCherry-WHV (LV-mCherry, 15ng, hCas9 group) or LV-Tomato-sgGFP1 (15ng, sgGFP group). Cells were kept in culture until DIV25.

Cortical astrocytes were infected with 20ng/wells of LV-cPPT-CMV-GFP-WHV (LV-CMV-GFP) at DIV7. Cells were then infected with LV-hCas9, (15ng) and LV-Tomato-sgGFP1 (15ng, CRISPR-GFP group) or LV-Tomato-sgGFP1 (15ng, sgGFP group). Cells were kept in culture until DIV56.

### DNA extraction

Genomic DNA from HEK-293T cells was extracted with the Trizol Reagent as described in example 2. Genomic DNA from primary cultures was extracted using the QuickExtract DNA Extraction Solution 1.0 (Epicentre, Offenbach, Germany) following the manufacturer's instructions. Culture medium were removed and cells were incubated in Trypsin at 37°C for 10 minutes. Cells were resuspended, diluted in 500µL of IX PBS/well and centrifuged at 300g for 5 minutes. Supernatant was removed, cells were resuspended in 50µL of lysis buffer, each tube was vortexed for 15 secondes and incubated at 65°C for 15 minutes with agitation. Each tube was then vortexed 15 secondes and lysis buffer was inactivated at 98°C for 2 minutes. Genomic DNA concentration was quantified with Nanodrop (Thermo Scientific, Reinach, Switzerland) using lysis buffer as blank solution before conservation at -20°C.

### Surveyor analysis

DSB formation was evaluated using the surveyor mutation detection kit, which detects mutations and polymorphisms in DNA (Transgenomic Inc, Glasgow, UK) (30). Briefly, forward (GFP-1F: TGACAAATGGAAGTAGCACG) and reverse oligonucleotides (GFP-2R: GAGATGAGTTTTTGTTCGAAGGG) complementary to a region located around the target sequence in the genomic DNA (100 ng) are used with the Kapa HiFi Hotstart polymerase (KapaBiosystems, Le Mont sur Lausanne, Switzerland) to perform a PCR reaction. For each PCR, a negative sample containing water was used. PCR products were purified with the Qiaquick PCR purification kit (Qiagen) and eluted in RNAse-DNAse free water. PCR product concentration was measured with Nanodrop (Thermo Scientific, Reinach, Switzerland) using RNAse-DNAse free water as blank. For each sample, the detected concentration in negative PCR reaction (water) was subtracted to correct for remaining oligonucleotides purification. The presence of a single PCR product was verified for each sample before further Surveyor processing. Three hundred nanograms of PCR product is incubated at 95°C in a thermocycler and subjected to the surveyor nuclease according to the manufacturer's protocol. Migration of the digested products is done on 10% TBE polyacrylamide precast gels (BioRad, Reinach, Switzerland). DNA is stained using SYBR gold (Molecular Probes, Life Technologies, Zug, Switzerland). The percentage of indels is determined by quantifying band intensities with Image J software (http://rsb.info.nih.gov/ij/, NIH, Bethesda) as described by Ran and collaborators (31).

### Image acquisitions

Neuronal cultures were fixed in 4% paraformaldehyde (PFA) for 15 minutes at room temperature. Cells were washed 3 times with IX PBS and coverslips were mounted on microscope slides. Pictures were acquired on a Zeiss Axiovision microscope using 20-40x objectives (Carl Zeiss Microscopy GmBH, Göttingen, Germany). Constant exposure time was used to compare fluorescence of control and treated groups.

### Results

The efficiency of CRISPR system was evaluated both in cultured cortical neurons and astrocytes. Three weeks post-infection with the CRISPR vectors, genomic DNA was extracted and subjected to Surveyor assay to detect the presence of indels in the target sequence. Quantifications reveal around 45% and 30% of gene disruption in the GFP gene in neurons and astrocytes, respectively (Figure 4A-B). Microscope acquisitions show that the majority of Tomato-positive neurons are also GFP-positive in the sgGFP group. However, Tomato-positive neurons in the CRISPR-GFP samples are mainly GFP-negative (Figure 4C). Equivalent observations were made with astrocytic cultures (data not shown). Altogether, these data demonstrate that efficient gene disruption leading to the protein loss of function is occurring both in cultures neurons and astrocytes with LV-delivered CRISPR system.

### Example 4: Delivery and efficacy of the CRISPR system in vivo

### Material and methods

### Animals

Adult females C57B1/6 (Janvier, Le Genest Saint Isle, France) and males and females BAC GLT1-eGFP (kindly provided by Pr. J. Rothstein, Baltimore, MD, USA) of 10-weeks old transgenic mice expressing GFP specifically in astrocytes were used for *in vivo* experiments. The animals were housed in a temperature-controlled room (22°C +- 1°C) and maintained on a 12h light/night cycle. Food and water were available *ad libitum.* All experimental procedures were performed in strict accordance with the recommendations of the European Community directive (86/609/EEC) and Swiss legislation about the care and use of laboratory animals. Sample size was chosen to take into account statistical variability due to surgical procedure based on previous studies (5).

### Stereotaxic surgery

### Stereotaxic surgery was performed according to procedure described in the example 2

Concentrated viral stocks were thawed on ice and resuspended by repeated pipetting. 100ng of LV-PGK-GFP, 100ng of LV-hCas9 and 100ng of LV-Tomato-sgGFP1 (CRISPR-GFP group) were injected in the mouse striatum. As negative controls, 100ng of LV-PGK-GFP and 100ng of LV-Tomato-sgGFP1 (sgGFP group) were used. For AAV editing, the same experimental design was used except that LV-GFP was replaced by an AAV2/5-CBA-GFP-WHV-bGHpolyA (1.10⁷ vg/site).

For GFP editing in astrocytes, BAC GLT1-eGFP mice were injected with 100ng of LV-cPPT-gfaABC1D(b)3-hCas9-WHV (LV-gfaABC1D(b)3-hCas9) and 100ng of LV-Tomato-sgGFP1 (CRISPR-GFP group). Mice injected only with 100ng of LV-Tomato-sgGFP1 were used as negative controls (sgGFP group).

### Brain processing

Three weeks post-lentiviral injection, the animals were killed by an overdose of sodium pentobarbital (NaCl, B-Braun, Sampach, Germany; Esconarkon, Streuli, Uznach, Germany) and transcardially perfused with a 4% PFA (Electron Microscopy Sciences, Hatfield). The brains were removed and post-fixed in 4% PFA for 12 h and then cryoprotected in 20% sucrose / PBS for 3 h and in 30% sucrose / PBS for 24 h. A sledge microtome with a freezing stage at - 30 °C (Leica SM2010R, Biosystems Switzerland, Nunningen, Switzerland) was used to cut coronal sections between 20µm thickness. Slices throughout the entire striatum were collected and stored in tubes as free-floating sections in anti-freeze solution (18% sucrose, 25% sodium azide, ethylene glycol and sodium phosphate 50mM, pH = 7.4). Slices were then stored at - 20°C.

Mice used for Surveyor analysis were killed by an overdose of sodium pentobarbital (NaCl, B-Braun, Sampach, Germany; Esconarkon, Streuli, Uznach, Germany) and directly decapitated. Tomato-positive infected area was dissected under an inverted fluorescent microscope for direct genomic DNA extraction.

### DNA extraction

Genomic DNA was extracted using the QuickExtract DNA Extraction Solution 1.0 (Epicentre, Offenbach, Germany) following the manufacturer's instructions. Dissected striatum was disrupted in 50µL of lysis buffer with an automated disruptor, each tube was vortexed for 15 seconds and incubated at 65°C for 15 minutes with agitation. Each tube was then vortexed 15 seconds and lysis buffer was inactivated at 98°C for 2 minutes. Genomic DNA concentration was quantified with Nanodrop (Thermo Scientific, Reinach, Switzerland) using lysis buffer as blank solution before conservation at -20°C.

### Surveyor analysis

Surveyor assay against the GFP target sequence were performed as described in the Example 3 with the same primers.

### Immunohistochemistry

The following primary antibodies were used: rabbit polyclonal anti-DARPP-32 antibody (DARPP-32, 1/1000, Cell Signaling, Allschwill, Switzerland) and rabbit anti-NeuN antibody (Millipore, Schaffhausen, Switzerland). Free floating slices were rinsed 3 x 10 minutes in PBS (Laboratorium Dr Bichsel AG, Interlaken, Switzerland), followed by the saturation of nonspecific sites in PBS containing 10% BSA or 5% NGS (Sigma-Aldrich, Buchs, Switzerland) and 0.1% Triton X100 (Fluka, Sigma-Aldrich, Buchs, Switzerland) for 1h at room temperature (RT) under agitation. Primary antibodies were incubated overnight at 4°C under agitation. Slices were rinsed 3 x 10 minutes in PBS and incubated with the secondary antibodies for 1h at room temperature under agitation. The following secondary antibody was used: goat anti-rabbit IgG AlexaFluor 488 (1/1000, Invitrogen, Life Technologies, Zug, Switzerland). Slices were then rinsed 3 x 10 minutes in PBS, followed by mounting with Vectashield mounting medium for fluorescence with DAPI (Vector Lab Inc, Burlingame). For in vitro experiments, fixed cells were rinsed 3 x 1 minute in PBS after fixation and incubated in blocking solution (5% PBS-BSA, 0.2% Triton X100) for 20 minutes at RT. Primary antibody were incubated on cells for 1 hours at RT. Cells were washed 3 x 1 minutes in PBS and incubated in secondary antibody for 30 minutes at RT. The following secondary antibodies were used: donkey anti-goat IgG AlexaFluor 488 (1/1000, Invitrogen, Life Technologies, Zug, Switzerland) and goat anti-mouse IgG AlexaFluor 594 (1/1000, Invitrogen, Life Technologies, Zug, Switzerland). Coverslips were then washed 3 x 1 minute in PBS, incubated in DAPI for 5 minutes at RT, washed 3 x 1 minute in PBS and then mounted on a microscope slice in FluorSafe (Calbiochem, Allschwill, Switzerland) medium.

### Image acquisitions

Mosaics were acquired on a Zeiss Axiovision microscope using 20-40x objectives (Carl Zeiss Microscopy GmBH, Göttingen, Germany). Constant exposure time was used to compare fluorescence of control and treated groups. Acquisitions for direct GFP mean fluorescence intensity quantifications were performed with a Zeiss LSM 510 confocal at 63x magnification (Carl Zeiss Microscopy GmBH, Göttingen, Germany). Cell-by-cell analysis was done with the Image J software (http://rsb.info.nih.gov/ij/, NIH, Bethesda). The cytoplasm of each Tomato-positive neuron was manually contoured and GFP intensity was measured.

### Results

Lentiviral vectors coding for hCas9 and the Tomato-sgGFP were injected separately in C57Bl/6 mice to evaluate toxicity of the system. LV-PGK-mCherry was co-injected to visualize the infected area and was used as an indirect marker of neuronal toxicity. NeuN and DARPP-32 immunoreactivity, respectively two markers of neuronal survival and functionality, were not altered in the mCherry positive area 3 weeks post-injection of sgGFP or hCas9, suggesting the absence of sgRNA or hCas9 toxicity in the mouse brain. GFP editing of genomic integrated target (LV-PGK-GFP experiment) or extrachromosomal episomes (AAV-GFP experiment) resulted in a lot of Tomato-positive cells that are GFP-negative after infection with the CRISPR system (Figure 4D-E). Cell-by-cell GFP intensity was quantified for the LV-PGK-GFP experiment. Results reveal that Tomato-positive cells of the CRISPR-GFP group are around 97% less fluorescent than Tomato-positive cells of the sgGFP group (Figure 4E). At the DNA level, Surveyor assay shows a rate of NHEJ ranging from 49.8 to 56.6% in the Tomato-positive area (Figure 4F). Then, injection of an astrocytic LV expressing hCas9 (LV-gfaABC1D(b)3-hCas9) with LV-Tomato-sgGFP1 in BAC GLT1-eGFP mice expressing GFP only in astrocytes resulted in a strong loss of GFP signal in the infected area in the CRISPR-GFP group compared to the sgGFP (Figure 4G). Altogether, these data demonstrate the low toxicity of the CRISPR system in the mouse brain and its strong potency for both genomic and episomal target sequence editing in neurons and glial cells.

### Example 5: Human HTT disruption with the CRISPR system

### Selection of sgRNA targeting the HTT gene

The sgHTT were designed around the HTT mutation (Ensembl accession number: ENSG00000197386, position of the first CAG repeat in exon 1: 3'074'877-3'074'879), and comprising the region upstream of the *HTT* gene (positions 3066800) up to the 5' end of intron 1 (position 3086939). For the non-allele-specific gene editing approaches, three specific regions of interest were used for the selection of the sgRNA. Region upstream of the *HTT* gene including the promoter (Ensembl accession number: ENSG00000197386; positions 3066800 to 3074509), exon 1 of the *HTT* gene including the 5'UTR the transcription and translation start sites (TSS and ATG; Ensembl accession number: ENSG00000197386: positions 3074510 to 3075088), intron 1 of the *HTT* (Ensembl accession number: ENSG00000197386: positions 3075089 to 3086939).

### Cell culture

HEK-293T cells culture, transfection and infection were performed as described in example 1 and 2.

### DNA and proteins extraction

DNA and proteins extractions using the Trizol reagent were done as described in the example 2.

### Surveyor analysis

Concentration of the extracted DNA was measured using nanodrop (Thermo Scientific, Reinach, Switzerland). Hundred nanograms of genomic DNA from HEK-293T was PCR-amplified using Kapa HiFi polymerase (KapaBiosystems, Le Mont sur Lausanne, Switzerland) with oligonucleotides located around exon 1 of the human *HTT* gene (HTT-IF: TTGCTGTGTGAGGCAGAACCTGCGG, HTT-2R: TGCAGCGGCTCCTCAGCCAC, HTT-3F: CACTTCACACACAGCTTCGC, HTT-4R: TGCTGCTGGAAGGACTTGAG). The PCR products were purified on column with the High Pure PCR product purification Kit (Roche Diagnostics GmbH, Mannheim, Germany) and eluted in 10-20 µL elution buffer. The Surveyor analysis was performed as described in example 3.

### Western blots

Western blot analysis was performed according to procedure described in the example 2.

### Mutant HTT aggregates imaging

HEK-293T Cell cultures (24 wells plates; LGC, Wesel, Germany) were grown and transfected on coverslips. Cells were fixed in 4% PFA for 10 minutes at RT, washed 2 times in PBS and mounted on microscope slides. Pictures were acquired on a Zeiss Axiovision microscope using a 10x objective and FITC channel (Carl Zeiss Microscopy GmBH, Göttingen, Germany). Constant exposure time was used to compare fluorescence of control and treated groups.

### Results

Applicants first targeted the endogenous human *HTT* gene in HEK-293T cells using the CRISPR system. Applicants co-transfected plasmids coding for hCas9 and the sgHTT1 (CRISPR-HTT1, Figure 6A) or sgHTT3 (CRISPR-HTT3, Figure 6B) and assessed gene editing 3 (CRISPR-HTT1 and CRISPR-HTT3) and 7 days post-transfection (CRISPR-HTT1). Surveyor analysis shows between 17- 45% of DSB formation at the expected target site 3-7 days post-transfection (Figure 6A-BA). Applicants next co-transfected plasmids coding for hCas9, sgHTT1 and the fused HTT171-82Q-GFP sequence in HEK-293T cells to determine the impact of *mHTT* disruption on pathological aggregates. Western blots analysis reveal up to 90% of mHTT loss in treated groups when compared with control cells (Figure 6C). Epifluorescence microscopy reveals the presence of GFP-positive nuclear and cytoplasmic aggregates in non-treated cells, which disappear in cells transfected with the CRISPR system (Figure 6D). This demonstrates that *mHTT* disruption leads to the loss of pathological aggregates.

### Example 6: Mutant HTT editing with the CRISPR system in neurons

### Primary neuronal cultures

Mouse cortical neurons were cultured as described in the Example 2.

### Primary culture infections

Cortical neurons were infected with LV-cPPT-CMV-hCas9-WHV (LV-hCas9, 15ng) and LV-cPPT-H1-sgHTT1 (LV-sgHTT1, 15ng, CRISPR-HTT group) at DIV1. Neurons infected with LV-hCas9 (15ng) and LV-Tomato-sgGFP1 (15ng, CRISPR-GFP) or LV-sgHTT1 alone (15ng, sgHTT1) were used as negative controls. All wells were then infected with 15ng of LV-cPPT-PGK-Htt171-82Q-WHV (LV-mHTT) at DIV4. Cells were kept in culture until DIV25.

### DNA extraction

Genomic DNA extractions were performed as described in example 3

### Surveyor analysis

Surveyor assay was performed as described in example 3. Primers for PCR amplification of human *HTT* were: hHTT-1F: TGACAAATGGAAGTAGCACG, hHTT-2R: GAGATGAGTTTTTGTTCGAAGGG. Primers for PCR amplification of mouse *HTT* were: mmHTT-1F: CCTCCTCACTTCTTTTCTATCG, mmHTT-2R: AGCATTATGTCATCCACTACC.

### Animals

Adult females C57B1/6 (Janvier, Le Genest Saint Isle, France) of 10-weeks old were used for *in vivo* experiments. The animals were housed in a temperature-controlled room (22°C +- 1°C) and maintained on a 12h light/night cycle. Food and water were available *ad libitum.* All experimental procedures were performed in strict accordance with the recommendations of the European Community directive (86/609/EEC) and Swiss legislation about the care and use of laboratory animals. Sample size was chosen to take into account statistical variability due to surgical procedure based on previous studies (5).

### Stereotaxic surgery

Stereotaxic surgery was performed according to procedure described in the example 3.

Concentrated viral stocks were thawed on ice and resuspended by repeated pipetting. 100ng of LV-mHTT, 100ng of LV-hCas9 and 100ng of LV-cPPT-H1-sgHTT1-PGK-mCherry-WHV (LV-sgHTT1-mCherry, CRISPR-HTT group) were injected in the mouse striatum. As negative controls, 100ng of LV-mHTT, 100ng of LV-hCas9 and 100ng of LV-Tomato-sgGFP1 (CRISPR-GFP group) were used.

### Brain processing and culture neurons fixation

Three weeks post-lentiviral injection, the animals were killed by an overdose of sodium pentobarbital (NaCl, B-Braun, Sampach, Germany; Esconarkon, Streuli, Uznach, Germany) and transcardially perfused with a 4% PFA (Electron Microscopy Sciences, Hatfield). The brains were removed and post-fixed in 4% PFA for 12 h and then cryoprotected in 20% sucrose / PBS for 3 h and in 30% sucrose / PBS for 24 h. A sledge microtome with a freezing stage at - 30 °C (Leica SM2010R, Biosystems Switzerland, Nunningen, Switzerland) was used to cut coronal sections between 20µm thickness. Slices throughout the entire striatum were collected and stored in tubes as free-floating sections in anti-freeze solution (18% sucrose, 25% sodium azide, ethylene glycol and sodium phosphate 50mM, pH = 7.4). Slices were then stored at - 20°C.

Neuronal cultures were fixed in 4% paraformaldehyde (PFA, Electron Microscopy Sciences, Hatfield) for 15 minutes at roOm temperature. Cells were washed 3 times with IX PBS (Gibco, Life Technologies, Zug, Switzerland) and coverslips were mounted on microscope slides.

### Immunohistochemistry

The following primary antibodies weas used: goat polyclonal anti-HTT (1/1000, SicGen, Lisboa, Portugal). Free floating slices were rinsed 3 x 10 minutes in PBS (Laboratorium Dr Bichsel AG, Interlaken, Switzerland), followed by the saturation of non-specific sites in PBS containing 5% BSA (Sigma-Aldrich, Buchs, Switzerland) and 0.1% Triton X100 (Fluka, Sigma-Aldrich, Buchs, Switzerland) for 1h at room temperature (RT) under agitation. Primary antibodies were incubated overnight at 4°C under agitation. Slices were rinsed 3 x 10 minutes in PBS and incubated with the secondary antibodies for 2h at room temperature under agitation. The following secondary antibody was used: donkey anti-goat IgG AlexaFluor 488 (1/500, Invitrogen, Life Technologies, Zug, Switzerland). Slices were then rinsed 3 x 10 minutes in PBS, followed by mounting with Vectashield mounting medium for fluorescence with DAPI (Vector Lab Inc, Burlingame).

### Image acquisitions

Mosaics and *in vitro* aggregates imaging were acquired on a Zeiss Axiovision microscope using 20-40x objectives (Carl Zeiss Microscopy GmBH, Göttingen, Germany). Constant exposure time was used to compare fluorescence of control and treated groups.

### Results

Mutant *HTT* editing with the sgHTT1 was first evaluated in mouse cortical neurons. Three weeks post-infection with the LV-mHTT vector, genomic DNA was extracted for Surveyor assay. Quantifications reveal around 35% of human mutant *HTT* disruption in neurons (Figure 7A). Because our sgHTT1 is homologous for mouse endogenous *HTT* except the first nucleotide (5' of the target sequence), we also amplified the target sequence with mouse-specific primers for Surveyor assay. Results demonstrate that cleavages occur at the same efficiency as for human *HTT,* meaning that our sgHTT1 is efficiently conserved across human and mouse (Figure 7A, right part). Immunostaining of HTT in CRISPR-GFP group reveals the presence of numerous large and small mHTT aggregates, mainly localized in nucleus/cytoplasm or neuritis, respectively (Figure 7B). The vast majority of these aggregates disappear in the CRISPR-HTT group at DIV25, especially small aggregates. Additionally, co-injection of vectors expressing CRISPR-HTT and LV-mHTT in the mouse striatum leads to the same loss of mHTT aggregation in CRISPR-HTT samples compared to CRISPR-GFP ones three weeks post-infection (Figure 7C). Altogether, these data demonstrate that our sgHTT1 is able to efficiently cleave *HTT* gene in primary neurons and to reverse mHTT aggregation both *in vitro* and *in vivo.*

### REFERENCES

1. Vonsattel JP & DiFiglia M (1998) Huntington disease. J Neuropath Exp Neurol 57:369-384.
2. Zuccato C, Valenza M, & Cattaneo E (2010) Molecular mechanisms and potential therapeutical targets in Huntington's disease. Physiol Rev 90(3):905-981.
3. Ross CA & Tabrizi SJ (2011) Huntington's disease: from molecular pathogenesis to clinical treatment. Lancet Neurol 10(1):83-98.
4. Fiszer A & Krzyzosiak WJ (2014) Oligonucleotide-based strategies to combat polyglutamine diseases. Nucleic Acids Res 42(11):6787-6810.
5. Drouet V, et al. (2009) Sustained effects of nonallele-specific Huntingtin silencing. Ann Neurol 65(3):276-285.
6. Drouet V, et al. (2014) Allele-specific silencing of mutant huntingtin in rodent brain and human stem cells. PLoS One 9(6):e99341.
7. Garriga-Canut M, et al. (2012) Synthetic zinc finger repressors reduce mutant huntingtin expression in the brain of R6/2 mice. Proc Natl Acad Sci USA 109(45):E3136-3145.
8. Cong L, et al. (2013) Multiplex genome engineering using CRISPR/Cas systems. Science 339(6121):819-823.
9. Jinek M, et al. (2013) RNA-programmed genome editing in human cells. eLife 2:e00471.
10. Sternberg SH, Redding S, Jinek M, Greene EC, & Doudna JA (2014) DNA interrogation by the CRISPR RNA-guided endonuclease Cas9. Nature 507(7490):62-67.
11. Merienne N, Le Douce J, Faivre E, Deglon N, & Bonvento G (2013) Efficient gene delivery and selective transduction of astrocytes in the mammalian brain using viral vectors. Front Cell Neurosci 7:106.
12. Simonato M, et al. (2013) Progress in gene therapy for neurological disorders. Nat Rev Neurol 9(5):277-291.
13. Cartier N, et al. (2009) Hematopoietic stem cell gene therapy with a lentiviral vector in X-linked adrenoleukodystrophy. Science 326(5954):818-823.
14. Palfi S, et al. (2014) Long-term safety and tolerability of ProSavin, a lentiviral vector-based gene therapy for Parkinson's disease: a dose escalation, open-label, phase 1/2 trial. Lancet 383(9923):1138-1146.
15. Weinberg MS, Samulski RJ, & McCown TJ (2013) Adeno-associated virus (AAV) gene therapy for neurological disease. Neuropharmacology 69:82-88.
16. Naldini L, et al. (1996) In vivo gene delivery and stable transduction of nondividing cells by a lentiviral vector. Science 272(5259):263-267.
17. Mali P, et al. (2013) RNA-guided human genome engineering via Cas9. Science 339(6121 ):823-826.
18. Jinek M, et al. (2012) A programmable dual-RNA-guided DNA endonuclease in adaptive bacterial immunity. Science 337(6096):816-821.
19. Hirano M, et al. (2013) Highly efficient retrograde gene transfer into motor neurons by a lentiviral vector pseudotyped with fusion glycoprotein. PLoS One 8(9):e75896.
20. Castle MJ, Gershenson ZT, Giles AR, Holzbaur EL, & Wolfe JH (2014) Adeno-Associated Virus Serotypes 1, 8, and 9 Share Conserved Mechanisms for Anterograde and Retrograde Axonal Transport. Hum Gene Ther 25(8):705-720.
21. Grimm D, et al. (2008) In vitro and in vivo gene therapy vector evolution via multispecies interbreeding and retargeting of adeno-associated viruses. J Virol 82(12):5887-5911.
22. Melo SP, et al. (2014) Somatic Correction of Junctional Epidermolysis Bullosa by a Highly Recombinogenic AAV Variant. Mol Ther 22(4):725-733.
23. Deglon N, et al. (2000) Self-inactivating lentiviral vectors with enhanced transgene expression as potential gene transfer system in Parkinson's disease. Hum Gene Ther 11(1): 179-190.
24. Hottinger AF, Azzouz M, Deglon N, Aebischer P, & Zurn AD (2000) Complete and long-term rescue of lesioned adult motoneurons by lentiviral-mediated expression of glial cell line-derived neurotrophic factor in the facial nucleus. J Neurosci 20(15):5587-5593.
25. Dull T, et al. (1998) A third-generation lentivirus vector with a conditional packaging system. J Virol 72(11):8463-8471.
26. Low K, Aebischer P, & Schneider BL (2013) Direct and retrograde transduction of nigral neurons with AAV6, 8, and 9 and intraneuronal persistence of viral particles. Hum Gene Ther 24(6):613-629.
27. Drinkut A, Tereshchenko Y, Schulz JB, Bahr M, & Kugler S (2012) Efficient gene therapy for Parkinson's disease using astrocytes as hosts for localized neurotrophic factor delivery. Mol Ther 20(3):534-543.
28. Aurnhammer C, et al. (2012) Universal real-time PCR for the detection and quantification of adeno-associated virus serotype 2-derived inverted terminal repeat sequences. Hum Gene Ther Methods 23(1):18-28.
29. Dujardin S, et al. (2014) Ectosomes: a new mechanism for non-exosomal secretion of tau protein. PLoS One 9(6):e100760.
30. Qiu P, et al. (2004) Mutation detection using Surveyor nuclease. Biotechniques 36(4):702-707.
31. Ran FA, et al. (2013) Genome engineering using the CRISPR-Cas9 system. Nat Protoc 8(11):2281-2308.

## Claims

1. A kit suitable for the treatment of Huntington's disease (HD) for allele or non-allele-specific huntingtin (*HTT*) gene editing or repair comprising a gene delivery vector consisting of:
one or more nucleic acid sequences of at least one viral vector selected from the group consisting of adeno-associated vector serotypes (AAV) and lentiviral vectors (LV);
at least one nucleic acid sequence that encodes a Cas9 being human codon-optimized or fused to an epitope tag selected among the group of FLAG, His, myc, Tap, HA, V5;
at least one nucleic acid sequence that encodes at least one artificial single guide RNA (sgRNA) having a total size from 63-115 nucleotides comprising a tracrRNA sequence of 48-85 nucleotides long and a crRNA sequence encoded by a nucleic acid sequence selected from the group consisting of SEQ ID NOS: 4-7 and 13-39 and recognizing the sequence of the *HTT* gene within the region defined by position 3066800 to position 3086939 excluding the expanded CAG repeat mutation (ENSG00000197386, position of the first CAG repeat in exon 1: 3'074'877-3'074'879), wherein said crRNA sequence binds directly upstream of the required 5'-NGG/NAG-3' protospacer adjacent motif (PAM) and whereas said crRNA sequence base-pairs with the target *HTT* sequence and Cas9 mediates a double-stranded break (DSB) 3-4 bp upstream of said PAM.

2. The kit suitable for the treatment of Huntington's disease of claim 1, **characterized in that** said gene delivery vector contains various expression cassettes comprising promoters and/or miRNA-regulated system so as to modulate transgene expression.

3. The kit suitable for the treatment of Huntington's disease according to any of claims 1-2, **characterized in that** said AAV is a mutant vector.

4. The kit suitable for the treatment of Huntington's disease according claim 3, **characterized in that** said AAV is the AAV-DJ.

5. The kit suitable for the treatment of Huntington's disease according to any of claims 1-4, **characterized in that** said Cas9 is mutated to improve the efficiency and safety.

6. The kit suitable for the treatment of Huntington's disease according claim 5, **characterized in that** said mutated Cas9 is a Cas9 nickase or a Cas9-V5.

7. The kit suitable for the treatment of Huntington's disease according to any of claims 1-6, **characterized in that** said gene delivery vector consists in a single vector or several vectors.

8. The kit suitable for the treatment of Huntington's disease according to any of claims 1-7, **characterized in that** said kit comprises multiple transcriptionally independent sgRNA as defined in claim 1 targeting the *HTT* gene.

9. The kit suitable for the treatment of Huntington's disease according to any of claims 1-8, further comprising an exogenous DNA template having at least 95% of homology with the homology arms located 10-20bp away from the double strand break site and with a minimum length of 100bp and up to 2.5 kb on both side of the double-stranded break (DSB).

10. The kit suitable for the treatment of Huntington's disease according to claim 9, **characterized in that** said exogenous DNA template for HR contains or not the HTT transcriptional start sites (TSS).

11. The kit suitable for the treatment of Huntington's disease according to any of claims 9-10, **characterized in that** said exogenous DNA template for HR is selected among the group comprising SEQ ID NO: 42-43.

12. The kit according to any of claims 1-8, for use in an *in vivo* method for non-allele-specific *HTT* inactivation of the human *HTT* wild-type and mutant alleles for the treatment of Huntington's disease in patients.

13. The kit according to any of claims 1-11, for use in a method of treatment of Huntington's disease in patients by *in vivo* non-allele-specific *HTT* gene repair based on HR with a DNA template containing a wild-type *HTT* sequence.

14. The kit according to any of claims 1-8, for use in a method of treatment of Huntington's disease in patients by *in vivo* allele-specific *HTT* inactivation of the human mutant HTT gene wherein said at least one sgRNA is capable of recognizing SNP sequences located upstream a PAM along the *HTT* gene with a high frequency of heterozygosity in the human population > 5% (according to dbSNP or 1000 Genome Project database).

15. The kit according to any of claims 1-11, for use in a method of treatment of Huntington's diesease in patients by *in vivo* mutant *HTT* gene repair based on HR with a DNA template containing a wild-type *HTT* sequence, wherein said at least one sgRNA is capable of recognizing SNP sequences located upstream a PAM along the *HTT* gene with a high frequency of heterozygosity in the human population > 5% (according to dbSNP or 1000 Genome Project database).

16. The kit suitable for the treatment of Huntington's disease according to any of claims 1-11, further comprising an additional transcriptionally independent artificial sgRNA capable of self-inactivating Cas9 expression after human *HTT* editing, wherein said additional transcriptionally independent artificial sgRNA comprises a crRNA sequence which recognizes key regions driving Cas9 expression selected among the ATG and/or the promoter driving the expression of Cas9.

17. The kit according to any of claims 1-11, for use in a method of treating Huntington's disease in a patient in need thereof, wherein said gene delivery vector used to deliver said Cas9 and said at least one artificial single guide RNA (sgRNA) to neuronal and glial cells is suitable for a local administration in the human striatum or is engineered to allow diffusion and/or transport from the striatum to the entire of the basal ganglia network of said patient.

## Patentansprüche

1. Kit, das zur Behandlung der Huntington-Krankheit (HD) geeignet ist, für die allel- oder nicht-allelspezifische Gen-Editierung oder Reparatur des Huntingtin-Gens (*HTT*)*,* umfassend einen Genübertragungsvektor bestehend aus:
einer oder mehreren Nukleinsäuresequenzen von mindestens einem viralen Vektor ausgewählt aus der Gruppe bestehend aus adeno-assoziierten Vektorserotypen (AAV) und lentiviralen Vektoren (LV);
zumindest einer Nukleinsäuresequenz, die für eine Cas9 codiert, die für das menschliche Codon optimiert oder mit einem Epitopen-Tag fusioniert ist, das aus der Gruppe von FLAG, His, myc, Tap, HA, V5 ausgewählt ist;
zumindest einer Nukleinsäuresequenz, die für zumindest eine künstliche Single-Guide-RNA (sgRNA) codiert, mit einer Gesamtgröße von 63 - 115 Nukleotiden, umfassend eine tracrRNA-Sequenz von 48 - 85 Nukleotiden Länge und eine crRNA-Sequenz, die durch eine Nukleinsäuresequenz codiert wird, die aus der Gruppe bestehend aus den SEQ ID NR: 4-7 und 13-39 ausgewählt ist, und die Sequenz des *HTT-*Gens innerhalb der Region erkennt, die durch Position 3066800 bis Position 3086939 definiert ist, unter Ausschluss der erweiterten CAG-Wiederholungs-Mutation (ENSG00000197386, Position der ersten CAG-Wiederholung in Exon 1: 3'074'877 - 3'074'879), wobei die crRNA-Sequenz direkt vor dem erforderlichen zum Protospacer benachbarten Motiv (PAM) 5'-NGG/NAG-3' bindet, und wobei die crRNA-Sequenz Basenpaare mit der Ziel-*HTT*-Sequenz bildet und Cas9 einen Doppelstrangbruch (DSB) 3-4 bp vor dem PAM vermittelt.

2. Kit, das zur Behandlung der Huntington-Krankheit geeignet ist, nach Anspruch 1, **dadurch gekennzeichnet, dass** der Genübertragungsvektor verschiedene Expressionskassetten enthält, die Promotoren und/oder ein miRNA-reguliertes System umfassen, um die Transgen-Expression zu modulieren.

3. Kit, das zur Behandlung der Huntington-Krankheit geeignet ist, nach einem der Ansprüche 1-2, **dadurch gekennzeichnet, dass** der AAV ein mutierter Vektor ist.

4. Kit, das zur Behandlung der Huntington-Krankheit geeignet ist, nach Anspruch 3, **dadurch gekennzeichnet, dass** der AAV AAV-DJ ist.

5. Kit, das zur Behandlung der Huntington-Krankheit geeignet ist, nach einem der Ansprüche 1-4, **dadurch gekennzeichnet, dass** die Cas9 mutiert ist, um die Effizienz und Sicherheit zu verbessern.

6. Kit, das zur Behandlung der Huntington-Krankheit geeignet ist, nach Anspruch 5, **dadurch gekennzeichnet, dass** die mutierte Cas9 eine Cas9-Nickase oder eine Cas9-V5 ist.

7. Kit, das zur Behandlung der Huntington-Krankheit geeignet ist, nach einem der Ansprüche 1-6, **dadurch gekennzeichnet, dass** der Genübertragungsvektor aus einem einzelnen Vektor oder mehreren Vektoren besteht.

8. Kit, das zur Behandlung der Huntington-Krankheit geeignet ist, nach einem der Ansprüche 1-7, **dadurch gekennzeichnet, dass** das Kit mehrere transkriptionell unabhängige sgRNAs wie in Anspruch 1 definiert umfasst, die das Gen targetieren.

9. Kit, das zur Behandlung der Huntington-Krankheit geeignet ist, nach einem der Ansprüche 1-8, ferner umfassend eine exogene DNA-Matrize mit mindestens 95 % Homologie mit den homologen Armen, die sich 10-20 bp von der Doppelstrangbruchstelle entfernt befinden, und mit einer minimalen Länge von 100 bp und bis zu 2,5 kb auf beiden Seiten des Doppelstrangbruchs (DSB).

10. Kit, das zur Behandlung der Huntington-Krankheit geeignet ist, nach Anspruch 9, **dadurch gekennzeichnet, dass** die exogene DNA-Matrize für HR die Transkriptionsstartpunkte (TSS) von *HTT* enthält oder nicht.

11. Kit, das zur Behandlung der Huntington-Krankheit geeignet ist, nach einem der Ansprüche 9-10, **dadurch gekennzeichnet, dass** die exogene DNA-Matrize für HR ausgewählt ist aus der Gruppe umfassend die SEQ ID NR: 42-43.

12. Kit nach einem der Ansprüche 1-8, zur Verwendung in einem in-vivo-Verfahren zur nicht-allelspezifischen *HTT-*Inaktivierung der menschlichen Wildtyp- und mutierten Allele von *HTT* , für die Behandlung der Huntington-Krankheit bei Patienten.

13. Kit nach einem der Ansprüche 1-11, zur Verwendung in einem Verfahren zur Behandlung der Huntington-Krankheit in Patienten durch nicht-allelspezifische HTT-Genreparatur in vivo auf Basis von HR mit einer DNA-Matrize, die eine Wildtyp-*HTT*-Sequenz enthält.

14. Kit nach einem der Ansprüche 1-8, zur Verwendung in einem Verfahren zur Behandlung der Huntington-Krankheit in Patienten durch allelspezifische HTT-Inaktivierung des menschlichen Mutanten-*HTT*-Gens in vivo, wobei die zumindest eine sgRNA in der Lage ist, SNP-Sequenzen, die sich dem PAM vorgelagert entlang des *HTT*-Gens befinden, zu erkennen, mit einem hohen Vorkommen der Heterozygosität in der menschlichen Population von > 5 % (nach dbSNP oder der 1000 Genome Project Database).

15. Kit nach einem der Ansprüche 1-11, zur Verwendung in einem Verfahren zur Behandlung der Huntington-Krankheit in Patienten durch Reparatur des Mutanten-*HTT*-Gens in vivo auf Basis von HR mit einer DNA-Matrize, die eine Wildtyp-HTT-Sequenz enthält, wobei die zumindest eine sgRNA in der Lage ist, SNP-Sequenzen, die sich dem PAM vorgelagert entlang des *HTT*-Gens befinden, zu erkennen, mit einem hohen Vorkommen der Heterozygosität in der menschlichen Population von > 5 % (nach dbSNP oder der 1000 Genome Project Database).

16. Kit, das zur Behandlung der Huntington-Krankheit geeignet ist, nach einem der Ansprüche 1-11, ferner umfassend eine zusätzliche, transkriptionell unabhängige künstliche sgRNA, die zur Autoinaktivierung der Cas9-Expression nach der Editierung des menschlichen *HTT* in der Lage ist, wobei die zusätzliche, transkriptionell unabhängige künstliche sgRNA eine crRNA-Sequenz umfasst, die Schlüsselregionen erkennt, welche die Expression von Cas9 steuern, ausgewählt aus ATG und/oder dem Promoter, der die Expression von Cas9 steuert.

17. Kit nach einem der Ansprüche 1-11, zur Verwendung in einem Verfahren zur Behandlung eines Patienten mit der Huntington-Krankheit, der die Behandlung benötigt, wobei der Genübertragungsvektor, der verwendet wird, um die Cas9 und die zumindest eine Single-Guide-RNA (sgRNA) an neuronale oder Glia-Zellen zu übertragen, für eine lokale Verabreichung in das menschliche Striatum geeignet ist, oder gentechnisch konstruiert ist, um eine Diffusion und/oder einen Transport aus dem Striatum in das gesamte Basalgangliennetz des Patienten zu erlauben.

## Revendications

1. Un Kit approprié pour le traitement de la maladie de Huntington (HD) pour l'édition ou la réparation du gène de la huntingtine (*HTT*) à allèle spécifique ou non spécifique, comprenant un vecteur de libération de gène constitué par :
une ou plusieurs séquence(s) d'acide nucléique d'au moins un vecteur viral choisi dans le groupe constitué par des sérotypes de vecteurs adéno-associés (AAV) et des vecteurs lentiviraux (LV) ;
au moins une séquence d'acide nucléique qui code pour une Cas9 qui est une Cas9 humaine à codon optimisé ou fusionnée à un marqueur épitope choisi dans le groupe de FLAG, His, myc, Tap, HA, V5 ;
au moins une séquence d'acide nucléique qui code pour au moins un ARN guide unique (ARNsg) artificiel ayant une taille totale de 63 à 115 nucléotides comprenant une séquence d'ARNtracr ayant une longueur de 48 à 85 nucléotides et une séquence d'ARNcr codée par une séquence d'acide nucléique choisie dans le groupe constitué par les SEQ ID NO : 4-7 et 13-39 et reconnaissant la séquence du gène *HTT* dans la région définie par la position 3066800 à la position 3086939 à l'exclusion de la mutation de répétitions CAG étendues (ENSG00000197386, position de la première répétition CAG dans l'exon 1 : 3'074'877-3'074'879), où ladite séquence d'ARNcr se lie directement en amont du motif adjacent au proto-espaceur 5'-NGG/NAG-3' (PAM) requis et où ladite séquence d'ARNcr effectue un appariement des bases avec la séquence *HTT* cible et Cas9 induit une cassure double brin (CDB) de 3-4 pb en amont dudit PAM.

2. Le Kit approprié pour le traitement de la maladie de Huntington de la revendication 1, **caractérisé en ce que** ledit vecteur d'administration de gène contient diverses cassettes d'expression comprenant des promoteurs et/ou un système régulé par miARN de manière à moduler l'expression de transgène.

3. Le Kit approprié pour le traitement de la maladie de Huntington selon l'une des revendications 1 et 2, **caractérisé en ce que** ledit AAV est un vecteur mutant.

4. Le Kit approprié pour le traitement de la maladie de Huntington selon la revendication 3, **caractérisé en ce que** ledit AAV est l'AAV-DJ.

5. Le Kit approprié pour le traitement de la maladie de Huntington selon l'une des revendications 1 à 4, **caractérisé en ce que** ladite Cas9 est mutée pour améliorer l'efficacité et la sécurité.

6. Le Kit approprié pour le traitement de la maladie de Huntington selon la revendication 5, **caractérisé en ce que** ladite Cas9 mutée est une Cas9 nickase ou une Cas9-V5.

7. Le Kit approprié pour le traitement de la maladie de Huntington selon l'une des revendications 1 à 6, **caractérisé en ce que** ledit vecteur d'administration de gène est constitué d'un seul vecteur ou de plusieurs vecteurs.

8. Le Kit approprié pour le traitement de la maladie de Huntington selon l'une des revendications 1 à 7, **caractérisé en ce que** ledit kit comprend de multiples ARNsg transcriptionnellement indépendants tels que définis dans la revendication 1 ciblant le gène *HTT.*

9. Le Kit approprié pour le traitement de la maladie de Huntington selon l'une des revendications 1 à 8, comprenant en outre une matrice d'ADN exogène ayant une homologie d'au moins 95% avec les bras d'homologie situés à 10-20 pb du site de cassure double brin et avec une longueur minimale de 100 pb et jusqu'à 2,5 kb sur les deux côtés de la cassure double brin (CDB).

10. Le Kit approprié pour le traitement de la maladie de Huntington selon la revendication 9, **caractérisé en ce que** ladite matrice d'ADN exogène pour une recombinaison homologue (HR) contient ou non les sites d'initiation de la transcription (TSS) de HTT.

11. Le Kit approprié pour le traitement de la maladie de Huntington selon l'une des revendications 9 à 10, **caractérisé en ce que** ladite matrice d'ADN exogène pour HR est choisie dans le groupe comprenant les SEQ ID NO : 42-43.

12. Le Kit selon l'une des revendications 1 à 8, destiné à être utilisé dans un procédé *in vivo* pour une inactivation de *HTT* allèle non spécifique des allèles mutants et de type sauvage de *HTT* humaine pour le traitement de la maladie de Huntington chez les patients.

13. Le Kit selon l'une des revendications 1 à 11, destiné à être utilisé dans un procédé de traitement de la maladie de Huntington chez les patients par une réparation *in vivo* de gène *HTT* allèle non spécifique sur la base de HR avec une matrice d'ADN contenant une séquence *HTT* de type sauvage.

14. Le Kit selon l'une des revendications 1 à 8, destiné à être utilisé dans un procédé de traitement de la maladie de Huntington chez les patients par une inactivation *in vivo* de *HTT* allèle spécifique du gène *HTT* mutant humain, où ledit au moins un ARNsg est capable de reconnaître des séquences SNP situées en amont d'un PAM le long du gène *HTT* avec une haute fréquence d'hétérozygotie dans la population humaine > 5% (selon dbSNP ou la base de données 1000 Genome Project).

15. Le Kit selon l'une des revendications 1 à 11, destiné à être utilisé dans un procédé de traitement de la maladie de Huntington chez les patients par une réparation *in vivo* de gène *HTT* mutant sur la base de HR avec une matrice d'ADN contenant une séquence *HTT* de type sauvage, où ledit au moins un ARNsg est capable de reconnaître des séquences SNP situées en amont d'un PAM le long du gène *HTT* avec une haute fréquence d'hétérozygotie dans la population humaine > 5% (selon dbSNP ou la base de données 1000 Genome Project).

16. Le Kit approprié pour le traitement de la maladie de Huntington selon l'une des revendications 1 à 11, comprenant en outre un ARNsg artificiel supplémentaire transcriptionnellement indépendant capable d'auto-inactiver l'expression de Cas9 après l'édition de *HTT* humaine, où ledit ARNsg artificiel supplémentaire transcriptionnellement indépendant comprend une séquence d'ARNcr qui reconnaît les régions clés entraînant l'expression de Cas9 choisies parmi l'ATG et/ou le promoteur entraînant l'expression de Cas9.

17. Le Kit selon l'une des revendications 1 à 11, destiné à être utilisé dans un procédé de traitement de la maladie de Huntington chez un patient en ayant besoin, où ledit vecteur d'administration de gène utilisé pour libérer ladite Cas9 et ledit au moins un ARN guide unique artificiel (ARNsg) aux cellules neuronales et gliales est adapté pour une administration locale dans le striatum humain ou est conçu pour permettre la diffusion et/ou le transport du striatum vers la totalité du réseau des noyaux gris centraux dudit patient.
